(19) Europäisches Patentamt
European Patent Office
Office européen des brevets

(11) EP 3 884 948 A1

(12) EUROPEAN PATENT APPLICATION

(43) Date of publication:
**29.09.2021 Bulletin 2021/39**

(51) Int Cl.:
***A61K 31/497*** *(2006.01)* ***A61P 35/00*** *(2006.01)*

(21) Application number: **20165999.2**

(22) Date of filing: **26.03.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Ecole Polytechnique Fédérale de Lausanne (EPFL)**
**1015 Lausanne (CH)**

(72) Inventors:
- **ORICCHIO, Elisa**
  **1003 Lausanne (CH)**
- **BATTISTELLO, Elena**
  **1004 Lausanne (CH)**
- **SANTIN, Marco**
  **36030 Caldogno (IT)**
- **REYMOND, Luc**
  **1806 St-Legier (CH)**

(74) Representative: **reuteler & cie SA**
**Chemin de la Vuarpillière 29**
**1260 Nyon (CH)**

# (54) CATHEPSIN INHIBITORS AND USES THEREOF

(57) The present invention relates to new agents and compositions thereof useful for anti-cancer and immunological disorder therapy and uses thereof. The invention further relates to a method of identifying agents useful in the potentiation of immunotherapy, in particular Cathepsin S inhibitors.

EP 3 884 948 A1

SEQUENCE LISTING

<110> ECOLE POLYTECHNIQUE FEDERALE DE LAUSANNE (EPFL)

<120> CATHEPSIN SUBSTRATES & CATHEPSIN INHIBITORS AND USES THEREOF

<130> P2469EP00

<160> 24

<170> PatentIn version 3.5

<210> 1
<211> 9
<212> PRT
<213> Artificial sequence

<220>
<223> Synthetic peptide

<400> 1

Lys Leu Arg Met Lys Leu Pro Lys Pro
1 5

<210> 2
<211> 9
<212> PRT
<213> Artificial sequence

<220>
<223> Synthetic peptide

<400> 2

Lys Leu Arg Met Lys Leu Pro Lys Asp
1 5

<210> 3
<211> 9
<212> PRT
<213> Artificial sequence

<220>
<223> Synthetic peptide

<400> 3

Lys Leu Arg Met Lys Leu Pro Lys Lys
1 5

<210> 4
<211> 8
<212> PRT
<213> Artificial sequence

<220>
<223> Synthetic peptide

<400> 4

Lys Pro Pro Lys Pro Val Ser Lys
1               5

<210> 5
<211> 8
<212> PRT
<213> Artificial sequence

<220>
<223> Synthetic peptide

<400> 5

Lys Pro Pro Lys Pro Val Ser Asp
1               5

<210> 6
<211> 32
<212> DNA
<213> Artificial sequence

<220>
<223> Plasmid

<400> 6
gctgaaaccg gtcagttgca taaagatcct ac 32

<210> 7
<211> 35
<212> DNA
<213> Artificial sequence

<220>
<223> Plasmid

<400> 7
gtgcttggta ccgatttctg ggtaagaggg aaagc 35

<210> 8
<211> 20
<212> DNA
<213> Artificial sequence

<220>
<223> Plasmid

<400> 8
gggaatgcat acctaccaag 20

<210> 9
<211> 20
<212> DNA
<213> Artificial sequence

<220>
<223> Plasmid

<400> 9
cttggtaggt atgcattccc 20

<210> 10
<211> 20
<212> DNA
<213> Artificial sequence

<220>
<223> Plasmid

<400> 10
cgagggttat ccagaagctg 20

<210> 11
<211> 20
<212> DNA
<213> Artificial sequence

<220>
<223> Plasmid

<400> 11
cagcttctgg ataaccctcg 20

<210> 12
<211> 20
<212> DNA
<213> Artificial sequence

<220>
<223> Plasmid

<400> 12
agtatactca cgccacccac 20

<210> 13
<211> 20
<212> DNA
<213> Artificial sequence

<220>
<223> Plasmid

<400> 13
gtgggtggcg tgagtatact 20

<210> 14
<211> 24
<212> DNA
<213> Artificial sequence

<220>
<223> Plasmid

<400> 14
caccgttgca taaagatcct accc 24

<210> 15
<211> 24
<212> DNA
<213> Artificial sequence

<220>
<223> Plasmid

<400> 15
aaacgggtag gatctttatg caac 24

<210> 16
<211> 20
<212> PRT
<213> Artificial sequence

<220>
<223> Synthetic peptide

<400> 16

```
Gln Leu Glu Asn Leu Arg Met Lys Leu Pro Lys Pro Pro Lys Pro Val
1               5                   10                  15

Ser Lys Met Arg
            20
```

<210> 17
<211> 20
<212> PRT
<213> Artificial sequence

<220>
<223> Synthetic peptide

<400> 17

```
Glu Glu Ile Glu Arg Met Val Gln Glu Ala Glu Lys Tyr Lys Ala Glu
1               5                   10                  15

Asp Glu Val Gln
            20
```

<210> 18
<211> 20
<212> PRT
<213> Artificial sequence

<220>
<223> Synthetic peptide

<400> 18

```
Ile Lys Val Tyr Leu Pro Arg Met Lys Met Glu Glu Lys Tyr Asn Leu
1               5                   10                  15
```

Thr Ser Val Leu
20

<210> 19
<211> 20
<212> PRT
<213> Artificial sequence

<220>
<223> Synthetic peptide

<400> 19

Asn Val Lys Asn Leu Tyr Glu Lys Val Lys Ser Gln Leu Lys Asn Asn
1 5 10 15

Ala Lys Glu Ile
20

<210> 20
<211> 20
<212> DNA
<213> Artificial sequence

<220>
<223> Plasmid

<400> 20
ggatcaccac tggcatctct 20

<210> 21
<211> 20
<212> DNA
<213> Artificial sequence

<220>
<223> Plasmid

<400> 21
attcccattg aatgctccag 20

<210> 22
<211> 20
<212> DNA
<213> Artificial sequence

<220>
<223> Plasmid

<400> 22
caaactttgc tttccctggt 20

<210> 23
<211> 20
<212> DNA
<213> Artificial sequence

<220>
<223> Plasmid

<400> 23
caagggcata tccaacaaca 20

<210> 24
<211> 9
<212> PRT
<213> Artificial sequence

<220>
<223> Synthetic peptide

<220>
<221> X
<222> (9)..(9)
<223> any amino acid

<400> 24

Lys Leu Arg Met Lys Leu Pro Lys Xaa
1 5

## Description

### Field of the Invention

The present invention relates generally to the field of immunotherapy, in particular cancer immunotherapy. The present invention further relates to a molecular assay for identifying Cathepsin S inhibitors and uses thereof.

### Background of the Invention

The immune system is composed of cells (B cells, T cells, macrophages, NK cells, granulocytes) able to recognize and destroy pathogens (e.g. virus and bacteria) that enter in our body through a process named antigen recognition. To allow antigen recognition, specific cells (like macrophage or the B cells) catch the pathogens and after "digestion", they expose small fractions of the pathogen proteins (the antigens) to T cells by MHC-class-II and MHC-class-I complexes. In particular, the cell interaction and antigen recognition between B and T cells happens inside a specific human organ called lymph node (inside the germinal center). The interaction between B and T cells induces B cell amplification and differentiation according to the type of the antigen. The B cell proliferation is a discreet process (highly controlled) since the B cells are subjected to genetic mutation for the creation of specific antibodies against the specific antigens.

In the past years, functional annotations of cancer genomic lesions have steered cancer treatment towards more targeted and personalized therapies. This approach in combination with the introduction of immune therapies has significantly improve cancer treatment. However, curative approaches are still rare and resistance to treatments and disease relapse are frequent and linked with tumor evolution.

Cancer selects genomic alterations that promote tumour growth and impair immune recognition.

Lymphoma is a heterogeneous malignancy of the lymphatic system characterized by proliferation of lymphoid cells or their precursors. It is possible to identify two main types of lymphoma: Non-Hodgkin lymphoma and Hodgkin lymphoma; only the 14 % of the lymphoma belongs to the Hodgkin lymphoma while the rest of the tumour are part of the Non-Hodgkin lymphoma (NHL) family.

In non-Hodgkin lymphoma (NHL), malignant B-cells accumulate genomic alterations that favor interactions with pro-tumorigenic immune-cells to sustain cell proliferation, while escaping immune surveillance (Pasqualucci et al., 2014, Cell Rep., 6, 130-140*;* Boice et al., 2016, Cell 167, 405-418.e13*;* Kridel et al., 2016, PLOS Med., 13, e1002197*;* Okosun et al., 2014, Nat. Genet., 46, 176-181*).* For example, in follicular lymphoma (FL) and diffuse large B-cell lymphoma (DLBCL), the most common forms of indolent and aggressive NHL, mutations or deletions of *TNFRSF14* or *B2M* are common *(Okosun et al*., *2014, supra;* Pasqualucci et al., 2011, Nat. Genet., 43, 830-837). Lack of *TNFRSF14* facilitates recruitment and communication of follicular lymphoma cells with CD4+ T-follicular helper (Tfh) cells, thus promoting B-cell expansion *(Boice et al., 2016, supra;* Cheung et al., 2010, Cancer Res., 70, 9166-9174*;* Kotsiou et al., 2014, Blood 124, 2426-2426 *or* 2016, Blood 128, 72-81*),* while loss of *B2M* expression impairs tumor recognition by cytotoxic CD8+ T-cells *(Pasqualucci et al., 2014, supra; Okosun et al*., *2014, supra;* Challa-Malladi et al., 2011, Cancer Cell, 20, 728-740). Hence, the cross-talk between malignant B-cells and the germinal center microenvironment is coopted to support tumorigenesis.

Non-Hodgkin lymphomas develop in the germinal centers where interactions between B-cells, CD4+ Tfh, and follicular dendritic cells are essential for B-cell expansion and selection *(*Crotty, 2014; Immunity 41, 529-542*;* Scott and Gascoyne, 2014, Nat. Rev. Cancer, 14, 517-534*;* Ise et al., 2018, Immunity, 48, 702-715.e4*).* The co-stimulatory interplay between B and CD4+ T cells is part of the humoral immune response and it is initiated by interactions between antigen-loaded MHC-class-II molecules and antigen-specific T-cell receptors. The formation of antigen-MHC-class-II complexes occurs in specialized endocytic compartments and it depends on the proteolytic activity of cathepsin S (gene name: *CTSS) (*Riese et al., 1996, J. Clin. Invest. 101, 2351-2363*;* Shi et al., 1999, Immunity, 10, 197-206*).* The formation of MHC-class-II complexes starts in the endoplasmic reticulum where the MHC-class-II molecules bind the chaperon protein CD74 (also named *Ii*) to prevent the premature loading of peptides *(*Roche and Furuta, 2015, Nat. Rev. Immunol., 15, 203-216*;* Schröder, 2016, Mol. Cell Res., 1863, 1269-1281). Then, CD74-MHC-class-II complexes reach the endocytic compartment, where CD74 is cleaved by cathepsin S leading to the formation of a smaller peptide CLIP (class-II-associated invariant chain peptide). CLIP is then displaced to allow variable antigenic peptides to bind MHC-class II molecules and be presented on the cell surface *(Riese et al*., *1996, supra*; Villadangos et al., 1997, J. Exp. Med., 186, 549-560).

Cathepsin S is part of a family of cysteine proteases and, although they often have redundant and compensatory functions, loss of cathepsin S is sufficient to induce accumulation of unprocessed CD74 peptides (p10) and limiting the presentation of specific extracellular antigens to CD4+ T-cells *(Shi et al., 1999, supra;* Riese et al., 1998, J. Clin. Invest., 101, 2351-2363). The activity of cathepsin S is important to mediate B-cell expansion in the germinal center after immunization *(Shi et al*., *1999, supra*). Cysteine proteases small molecules inhibitors have been developed and could

represent interesting therapeutic avenues. Unfortunately, their pan-reactivity across the cysteine protease family hinders their clinical development.

The oncogenic role of cathepsin S in solid tumors has been mainly associated with its extracellular activity on matrix degradation that favors metastasis formation (Gocheva et al., 2006, Genes Dev., 20, 543-556*;* Olson and Joyce, 2015, Nat. Rev. Cancer, 15, 712-729*;* Sevenich et al., 2014, Nat. Cell Biol., 16, 876-888*).* However, despite the importance of cathepsin S in the physiological process of antigen presentation, its intracellular activity in hematological malignancies has not been explored, yet.

The escape of cancer cells from immune surveillance by limiting antigen presentation through multiple mechanisms and modification of the antigen repertoire on cancer cell surface can transform a "cold" tumor in a "hot" inflamed tumor. Therefore, inducing antigen diversification by targeting regulators of antigen processing represents an attractive therapeutic strategy to increase tumor immunogenicity.

## Summary of the Invention

The present invention is based on the unexpected findings that patients with aggressive and indolent lymphomas (5%) over-expressed cathepsin S and follicular lymphoma patients harbor a recurrent hot-spot mutation targeting tyrosine 132 (Y132D) that enhances cathepsin S activity. Further, it was found that cathepsin S *(CTSS)* regulates antigen processing and CD4+ and CD8+ T-cell mediated immune responses. Importantly, loss of cathepsin S reduced tumor growth by limiting the communication with CD4+ T follicular helper cells while inducing antigen diversification and activation of CD8+ T-cells. Further, it was found that mutated Cathepsin S is overactivated and this mutation induces the uncontrolled proliferation of the B due to the continuous expression of the antigen on the MHC II. Therefore, since cathepsin S mutation and over-expression accelerate tumorigenesis in animal models of lymphoma, cathepsin S is a promising therapeutic target to turn a pro-tumorigenic into a tumor suppressive microenvironment by altering the antigen repertoire of tumor cells.

Further, the present invention is based on the finding of a specific substrate for Cathepsin S which allows advantageously identify specific Cathepsin S inhibitors. Finally, the invention provides support that inhibition of cathepsin S has non-redundant therapeutic potential to enhance anti-tumor immune responses in indolent and aggressive lymphomas.

According to a first aspect, the invention provides cathepsin inhibitors, in particular cathepsin S inhibitors for use in the prevention and/or treatment of a cancer, wherein said cathepsin inhibitor is to be administered in combination with immunotherapy or in the prevention and/or treatment of an immunological disorder, such as auto-immune diseases.

According to another aspect of the invention, is provided a pharmaceutical composition comprising at least one cathepsin inhibitor according to the invention and a pharmaceutically acceptable carrier, diluent or excipient thereof and at least one agent useful in anti-cancer therapy or immunotherapy.

According to another aspect, the invention provides a use of a cathepsin inhibitor according to the invention for the preparation of a pharmaceutical composition for the prevention and/or treatment of a cancer, wherein said cathepsin inhibitor is to be administered in combination with immunotherapy or for the prevention and/or treatment of auto-immune diseases. According to another aspect, the invention provides a method of preventing or treating a cancer, said method comprising administering in a subject in need thereof a therapeutically effective amount of at least one cathepsin inhibitor of the invention.

According to another, the invention provides a method of inducing or enhancing tumor immunogenicity, said method comprising administering in a subject in need thereof a therapeutically effective amount of at least one cathepsin inhibitor of the invention According to another, the invention provides a method of identifying Cathepsin enzyme inhibitors, in particular agents useful in the potentiation of immunotherapy.

According to another aspect, the invention provides peptides useful as a substrate for a Cathepsin enzyme and uses thereof.

According to another aspect, the invention provides a kit comprising at least one peptide of the invention.

## Description of the figures

**Figure 1** represents the setting and the results of a FRET assay using different peptides as substrate and the ability of CTSS, CTSS Y132D to cleave a particular substrate as tested in Examples 1 and 6. **a:** Schematic representation of the FRET assay b: quantification of MCA fluorescent emission obtained with CTSS, CTSS Y132D by using peptide of **SEQ ID NO: 1** as substrate. **c**: Quantification of MCA fluorescent emission by using CTSS and peptides of **SEQ ID NO: 1 and 4** as substrate

**Figure 2** represents the setting and the results of a FRET assay using different peptides as substrate and the ability of CTSS, CTSB and CTSH to cleave a particular substrate as tested in Examples 1 and 6. **a:** Quantification of MCA

fluorescent emission by using CTSS and peptides of **SEQ ID NO: 1, 2 and 3** as substrate **b:** Quantification of MCA fluorescent emission by using CTSB and peptide of **SEQ ID NO: 5** as substrate c: Quantification of MCA fluorescent emission by using CTSH and peptide of **SEQ ID NO: 2** as substrate .

**Figure 3** represents the results of a FRET assay according to the invention by using three representative molecules that are able to inhibit cathepsin S activity as described in Example 8. **a:** Quantification of MCA fluorescent emission by using CTSS and peptides of **SEQ ID NO: 1** as substrate and compound **(1); b:** Quantification of MCA fluorescent emission by using CTSS and peptides of **SEQ ID NO: 1** as substrate and compound **(5). c.** Quantification of MCA fluorescent emission by using CTSS and peptides of **SEQ ID NO: 1** as substrate and compound **(11).**

**Figure 4** represents the results of a FRET assay according to the invention by using three representative molecules that are able to inhibit cathepsin S activity as described in Example 8, but not Cathepsin B; **a:** Quantification of MCA fluorescent emission by using CTSB and peptides of **SEQ ID NO: 5** as substrate and compound **(1); b:** Quantification of MCA fluorescent emission by using CTSB and peptides of **SEQ ID NO: 5** as substrate and compound **(5). c.** Quantification of MCA fluorescent emission by using CTSB and peptides of **SEQ ID NO: 5** as substrate and compound **(11).**

**Figure 5** represents the results of a FRET assay according to the invention by using three representative molecules that are able to inhibit cathepsin S activity as described in Example 8, but not Cathepsin H; **a:** Quantification of MCA fluorescent emission by using CTSH and peptides of **SEQ ID NO: 2** as substrate and tcompound **(1); b:** Quantification of MCA fluorescent emission by using CTSH and peptides of **SEQ ID NO: 2** as substrate and compound **(5) . c.** Quantification of MCA fluorescent emission by using CTSH and peptides of **SEQ ID NO: 2** as substrate and compound **(11).**

**Figure 6:** represents *in vivo* studies using animal models as described in Example 4 and Example 5. **a:** percentage of tumor free per day measured by counting the number of lymphoblast in the peripheral blood in mice; **b:** tumor evolution after engraftment in mice engrafted with lymphoma cells CTSS KO. **c:** tumor evolution after engraftment in mice engrafted with lymphoma cells CTSS KO and treated twice a week with anti-PD1 antibody as described in Example 5.

**Detailed description**

The following paragraphs provide definitions of the various chemical moieties that make up the compounds according to the invention and are intended to apply uniformly through-out the specification and claims, unless an otherwise expressly set out definition provides a broader definition.

The term "alkyl" when used alone or in combination with other terms, comprises a straight chain or branched $C_1$-$C_{20}$ alkyl which refers to monovalent alkyl groups having 1 to 20 carbon atoms. This term is exemplified by groups such as methyl, ethyl, n-propyl, i-propyl, n-butyl, s-butyl, i-butyl, t-butyl, n-pentyl, 1-ethylpropyl, 2-methylbutyl, 3-methylbutyl, 2,2-dimethylpropyl, n-hexyl, 2-methylpentyl, 3-methylpentyl, 4-methylpentyl, n-heptyl, 2-methylhexyl, 3-methylhexyl, 4-methylhexyl, 5-methylhexyl, n-heptyl, n-octyl, n-nonyl, n-decyl, tetrahydrogeranyl, n-dodecyl, n-tridecyl, n-tetradecyl, n-pentadecyl, n-hexadecyl, n-octadecyl, n-nonadecyl, and n-eicosanyl and the like. Preferably, these include $C_1$-$C_9$ alkyl, more preferably $C_1$-$C_6$ alkyl, especially preferably $C_1$-$C_4$ alkyl, which, by analogy, refer respectively to monovalent alkyl groups having 1 to 9 carbon atoms, monovalent alkyl groups having 1 to 6 carbon atoms and monovalent alkyl groups having 1 to 4 carbon atoms. Particularly, those include $C_1$-$C_6$ alkyl.

The term "aryl" refers to an unsaturated aromatic carbocyclic group of from 6 to 14 carbon atoms having a single ring (*e.g.,* phenyl) or multiple condensed rings (*e.g.,* indenyl, naphthyl). Aryl include phenyl, naphthyl, anthryl, phenanthrenyl and the like.

The term "heteroaryl" refers to a monocyclic heteroaromatic, or a bicyclic or a tricyclic fused-ring heteroaromatic group. Particular examples of heteroaromatic groups include optionally substituted pyridyl, pyrrolyl, pyrimidinyl, furyl, thienyl, imidazolyl, oxazolyl, isoxazolyl, thiazolyl, isothiazolyl, pyrazolyl, 1,2,3-triazolyl, 1,2,4-triazolyl, 1,2,3-oxadiazolyl, 1,2,4-oxadia-zolyl, 1,2,5-oxadiazolyl, 1,3,4-oxadiazolyl,1,3,4-triazinyl, 1,2,3-triazinyl, benzofuryl, [2,3-dihydro]benzofuryl, isobenzofuryl, benzothienyl, benzotriazolyl, isobenzothienyl, indolyl, isoindolyl, 3H-indolyl, benzimidazolyl, imidazo[1,2-a]pyridyl, benzothiazolyl, benzoxa-zolyl, quinolizinyl, quinazolinyl, pthalazinyl, quinoxalinyl, cinnolinyl, napthyridinyl, pyridyls such as pyrido[3,4-b]pyridyl, pyrido[3,2-b]pyridyl, pyrido[4,3-b]pyridyl, quinolyl, isoquinolyl, tetrazolyl, 5,6,7,8-tetrahydroquinolyl, 5,6,7,8-tetrahydroisoquinolyl, purinyl, pteridinyl, carbazolyl, xanthenyl or benzoquinolyl.

The term "heteroaryl $C_1$-$C_6$ alkyl" refers to $C_1$-$C_6$ alkyl groups having a heteroaryl substituent, including furyl methyl and the like.

The term "$C_3$-$C_{10}$-cycloalkyl" refers to a saturated carbocyclic group of from 3 to 10 carbon atoms having a single ring (*e.g.,* cyclohexyl) or multiple condensed rings (*e.g.,* norbornyl). $C_3$-$C_8$-cycloalkyl includes cyclopentyl, cyclohexyl, norbornyl and the like. By analogy, the term "$C_3$-$C_8$-cycloalkyl" refers to a saturated carbocyclic group of from 3 to 8 carbon atoms having a single ring.

The term "heterocycloalkyl" refers to a $C_3$-$C_8$-cycloalkyl group according to the definition above, in which up to

3 carbon atoms are replaced by heteroatoms chosen from the group consisting of O, S, NR, R being defined as hydrogen or methyl. Heterocycloalkyl include pyrrolidinyl, piperidinyl, piperazinyl, morpholinyl, tetrahydrofuranyl and the like.

The term "acyloxy" refers to the group -OC(O)R where R includes H, "$C_1$-$C_6$ alkyl", "$C_2$-$C_6$ alkenyl," "$C_2$-$C_6$ alkynyl," "$C_3$-$C_8$-cycloalkyl," "heterocycloalkyl," "aryl," "heteroaryl," "aryl $C_1$-$C_6$ alkyl", "heteroaryl $C_1$-$C_6$ alkyl," "aryl $C_2$-$C_6$ alkenyl," "heteroaryl $C_2$-$C_6$ alkenyl," "aryl $C_2$-$C_6$ alkynyl," "heteroaryl $C_2$-$C_6$ alkynyl," "$C_3$-$C_8$-cycloalkyl $C_1$-$C_6$ alkyl," or "heterocycloalkyl $C_1$-$C_6$ alkyl", including acetyloxy and the like.

The term "acylamino" refers to the group -NRC(O)R' where R and R' are independently H, "$C_1$-$C_6$ alkyl," "$C_2$-$C_6$ alkenyl," "$C_2$-$C_6$ alkynyl," "$C_3$-$C_8$-cycloalkyl," "heterocycloalkyl," "aryl," "heteroaryl," "aryl $C_1$-$C_6$ alkyl", "heteroaryl $C_1$-$C_6$ alkyl," "aryl $C_2$-$C_6$ alkenyl," "heteroaryl $C_2$-$C_6$ alkenyl," "aryl $C_2$-$C_6$ alkynyl," "heteroaryl $C_2$-$C_6$ alkynyl," "cycloalkyl $C_1$-$C_6$ alkyl," or "heterocycloalkyl $C_1$-$C_6$ alkyl", including acetylamino and the like.

The term "aminocarbonyl" refers to the group -C(O)NRR' where R and R' are independently H, $C_1$-$C_6$ alkyl, aryl, heteroaryl, "aryl $C_1$-$C_6$ alkyl" or "heteroaryl $C_1$-$C_6$ alkyl," including N-phenyl carbonyl and the like.

The term "aminosulfonyl" refers to a group -$SO_2$-NRR' where R and R' are independently H, "$C_1$-$C_6$ alkyl," "$C_2$-$C_6$ alkenyl," "$C_2$-$C_6$ alkynyl," "$C_3$-$C_8$-cycloalkyl," "heterocycloalkyl," "aryl," "heteroaryl," "aryl $C_1$-$C_6$ alkyl", "heteroaryl $C_1$-$C_6$ alkyl," "aryl alkenyl," "heteroaryl $C_2$-$C_6$ alkenyl," "aryl $C_2$-$C_6$ alkynyl," "heteroaryl $C_2$-$C_6$ alkynyl," "$C_3$-$C_8$-cycloalkyl $C_1$-$C_6$ alkyl," or "heterocycloalkyl $C_1$-$C_6$ alkyl", and where R and R', together with the nitrogen atom to which they are attached, can optionally form a 3-8-membered heterocycloalkyl ring. Aminosulfonyl groups include cyclohexylaminosulfonyl, piperidinylsulfonyl and the like. The term "sulfonylamino" refers to a group -$NRSO_2$-R' where R and R' are independently "$C_1$-$C_6$ alkyl," "$C_2$-$C_6$ alkenyl," "$C_2$-$C_6$ alkynyl," "$C_3$-$C_8$-cycloalkyl," "heterocycloalkyl," "aryl," "heteroaryl," "aryl $C_1$-$C_6$ alkyl", "heteroaryl $C_1$-$C_6$ alkyl," "aryl $C_2$-$C_6$ alkenyl," "heteroaryl $C_2$-$C_6$ alkenyl," "aryl $C_2$-$C_6$ alkynyl," "heteroaryl $C_2$-$C_6$ alkynyl," "$C_3$-$C_8$-cycloalkyl $C_1$-$C_6$ alkyl," or "heterocycloalkyl $C_1$-$C_6$ alkyl".

The term "sulfonylamino $C_1$-$C_6$ alkyl" refers to alkyl groups having a sulfonylamino substituent, including 2-(ethylsulfonylamino) ethyl and the like.

The term "aminocarbonyl" refers to the group -C(O)NRR' where R and R' are independently H, $C_1$-$C_6$ alkyl, aryl, heteroaryl, "aryl $C_1$-$C_6$ alkyl" or "heteroaryl $C_1$-$C_6$ alkyl," including N-phenyl carbonyl and the like.

The term "aminocarbonyl $C_1$-$C_6$ alkyl" refers to alkyl groups having an aminocarbonyl substituent, including 2-(dimethylaminocarbonyl)ethyl, N-ethyl acetamidyl, N,N-Diethyl-acetamidyl and the like.

Unless otherwise constrained by the definition of the individual substituent, the term "substituted" refers to groups substituted with from 1 to 5 substituents selected from the group consisting of "$C_1$-$C_6$ alkyl," "$C_2$-$C_6$ alkenyl," "$C_2$-$C_6$ alkynyl," "$C_3$-$C_8$-cycloalkyl," "heterocycloalkyl," "$C_1$-$C_6$ alkyl aryl," "$C_1$-$C_6$ alkyl heteroaryl," "$C_1$-$C_6$ alkyl cycloalkyl," "$C_1$-$C_6$ alkyl heterocycloalkyl," "cycloalkyl $C_1$-$C_6$ alkyl," "heterocycloalkyl $C_1$-$C_6$ alkyl," "amino," "aminosulfonyl," "sulfonylamino," "ammonium," "alkoxy," "acyl amino," "amino carbonyl," "aryl," "aryl $C_1$-$C_6$ alkyl," "heteroaryl," "heteroaryl $C_1$-$C_6$ alkyl," "sulfinyl," "sulfonyl," "sulphonamide", "alkoxy," "alkoxy carbonyl," "acyloxy," "carbamate," "sulfanyl," "halogen," "carboxy," trihalomethyl, cyano, hydroxy, mercapto, nitro, and the like.

The term "Cathepsin inhibitor" refers to agents able to inhibit Cathepsin, in particular Cathepsin S. Inhibition of Cathepsin S can be assessed for example by measuring the enzymatic transformation of Cathepsin S substrates (peptides of 9-10 amino acids) into products (peptides of 4-6 amino acids) for example by a FRET assay or mass spectrometry or in cell-based assays described in Example 6. Inhibition of Cathepsin S can be also assessed at the functional level for example by testing the inhibitory effect by measuring changes of antigen repertoire, T cell activation and accumulation of CD74(p10) protein such as described in Example 5 Among those agents, some Cathepsin S inhibitors were actively developed for the treatment of different diseases including auto-immune disease (e.g. systemic lupus erythematosus, Sjogren's syndrome) such as described in Theron et al. 2017 (https://www.ncbi.nlm.nih.gov/pmc/articles/PMC5512459/)

The expression "immunotherapy" includes strategies used to activate effector immune cells to increase the efficacy of the patient's own immune response against neoplastic cells (lymphoma, melanoma, lung carcinoma, glioblastoma, renal carcinoma, gastrointestinal stromal carcinoma and leukemia). Immunotherapy includes cancer vaccine, oncolytic viruses, cell therapies and antibody conjugated drugs such as described in Example 6.

Vaccine can be used in an immunotherapy according to the invention. For example, those therapeutic vaccines are against antigen presented on the cancer cell surface as described in Example 7.

Oncolytic viruses can be used in an immunotherapy according to the invention. For example, oncolytic viruses that can serve as carrier for cathepsin inhibitors or specific antigens and peptides such as described in Garofalo et al., 2015, Cancer - Oncolytic Viruses II (23) Suppl. I, S 247.

Cell therapy in an immunotherapy according to the invention includes treatments based on genetically modified lymphocytes (T cells or NK cells), for instance, modification of macrophages or B-cells to express specific antigens that will promotes activation of the immune system such as described for Car-T cells receptors *(*Guedan et al., 2019, Molecular Therapy, Methods & Clinical Development, 12, P145-156).

Antibody drug conjugated according to the invention includes specific antibodies that can serve as carrier for the delivery of cathepsin inhibitors and activation of immune response as described in Chau et al., 2019, Therapeutics, 394, 10200, P793-804.

The expression "immunological disorder" in the context of the present invention includes auto-immune disorders that involve activity of antigen presenting cells as B-cell, macrophages, NK-cell and T-cells. Those disorders include auto-immune disease (e.g. systemic lupus erythematosus, Sjogren's syndrome) such as described in Theron et al., 2017, Front Immunol., 8: 806.

As used herein, "treatment" and "treating" and the like generally mean obtaining a desired pharmacological and physiological effect. The effect may be prophylactic in terms of preventing or partially preventing a disease, symptom or condition thereof and/or may be therapeutic in terms of a partial or complete cure of a disease, condition, symptom or adverse effect attributed to the disease. The term "treatment" as used herein covers any treatment of a disease in a mammal, particularly a human, and includes: (a) preventing the disease from occurring in a subject which may be predisposed to the disease but has not yet been diagnosed as having it such as a preventive early asymptomatic intervention; (b) inhibiting the disease, i.e., arresting its development; or relieving the disease, i.e., causing regression of the disease and/or its symptoms or conditions such as improvement or remediation of damage. In particular, the methods, uses, formulations and compositions according to the invention are useful for the prevention and/or treatment of a cancer or an immunological disorder.

The expression "solid tumour cancer" includes, lung cancer (small cell and non-small cell), breast cancer, ovarian cancer, cervical cancer, uterus cancer, head and neck cancer, glioblastoma, hepatocellular carcinoma, colon cancer, rectal cancer, colorectal carcinoma, kidney cancer, prostate cancer, gastric cancer, bronchus cancer, pancreatic cancer, urinary bladder cancer, hepatic cancer and brain cancer or skin cancer, melanoma. The expression of hematological malignancies includes lymphoma, leukemia, multiple myeloma, myelodysplastic syndrome and neoplasia, chronic lymphocytic leukemia.

The term "subject" as used herein refers to mammals. For examples, mammals contemplated by the present invention include human, primates, domesticated animals such as cattle, sheep, pigs, horses, laboratory rodents, other pets and the like.

The term "effective amount" as used herein refers to an amount of at least one compound of the invention or a pharmaceutical formulation thereof according to the invention that elicits the biological or medicinal response in a tissue, system, animal or human that is being sought. In one embodiment, the effective amount is a "therapeutically effective amount" for the alleviation of the symptoms of the disease or condition being treated. In another embodiment, the effective amount is a "prophylactically effective amount" for prophylaxis of the symptoms of the disease or condition being prevented. The term also includes herein the amount of compound of the invention sufficient to reduce the progression of the disease, notably to reduce or inhibit the progression of a cancer disorder and thereby elicit the response being sought (i.e. an "effective amount"). Typically, an effective amount can be used to reduce initial phase of tumor development. Further, an effective amount can be used to inhibit the growth of cancer cells, i.e. any slowing of the rate of cancer cell proliferation and/or migration, arrest of cancer cell proliferation and/or migration, or killing of cancer cells, such that the rate of cancer cell growth is reduced in comparison with the observed or predicted rate of growth of an untreated control cancer cell. The term "inhibits growth" can also refer to a reduction in size or disappearance of a cancer cell or tumor, as well as to a reduction in its metastatic potential. Preferably, such an inhibition at the cellular level may reduce the size, defer the growth, reduce the aggressiveness, or prevent or inhibit metastasis of a cancer in a patient. Those skilled in the art can readily determine, by any of a variety of suitable indicia, whether cancer cell growth is inhibited.

The term "efficacy" of a treatment according to the invention can be measured based on changes in the course of disease in response to a use or a method according to the invention. According to a particular embodiment, the efficacy can be measured through the measuring of the elicited immune response against cancer cells such as by analyzing tumor-specific T cells or by assessing cancer cell death and/or inhibition of tumor growth, progression and dissemination, reduction of tumour volume, and/or an increase of progression free survival time and/or increased health and well-being of the subject (e.g. repressing a cancer). The efficacy of a treatment of a cancer according to the invention can be measured by an inhibition of cancer cell growth evidenced for example by an arrest of cancer cells in a particular phase of the cell cycle, e.g., arrest at the G2/M phase of the cell cycle. Inhibition of cancer cell growth can also be evidenced using well known imaging methods such as magnetic resonance imaging, computerized axial tomography, PET, SPECT, photo-acoustic imaging, X-rays and fluorescence imaging/detection. Cancer cell growth can also be determined indirectly, for example by determining the levels of circulating carcino-embryonic antigen, circulating tumor cells, expression of cancer- specific antigens that are correlated with cancer cell growth.

In particular, efficacy of a combined treatment according to the invention can be assessed by expansion of tumor infiltrating CD8+ T cells, reduction of tumour size, reduction of germinal center expansion, disappearance of tumour, survival of tumor bearing mice or of any biomarker relevant for a cancer type.

The term "pharmaceutically acceptable salts" refers to salts of the below-specified compounds of the invention. Examples of such salts include, but are not restricted, to base addition salts formed by reaction of compounds of the invention with organic or inorganic bases such as hydroxide, carbonate, bicarbonate or the like, of a metal cation such as those selected in the group consisting of alkali metals (sodium, potassium or lithium), alkaline earth metals (e.g. calcium or magnesium), or with an organic primary, secondary or tertiary alkyl amine. Other examples of such salts

include, but are not restricted, to acid addition salts formed by reaction of compounds of the invention with organic or inorganic acids such as hydrochloric acid, hydrobromic acid, sulphuric acid, para-toluene sulfonic acid, 2-naphtalene sulfonic acid, camphosulfonic acid, benzene sulfonic acid, oxalic acid or the like.

"Pharmaceutically active derivative" refers to any compound that upon administration to the recipient, is capable of providing directly or indirectly, the activity disclosed herein. The term "indirectly" also encompasses prodrugs which may be converted to the active form of the drug via endogenous enzymes or metabolism. The prodrug is a derivative of the compound according to the invention and presenting anti-tumor activity that has a chemically or metabolically decomposable group, and a compound that may be converted into a pharmaceutically active compound *in vivo* under physiological conditions.

The term "pharmaceutical formulation" refers to preparations which are in such a form as to permit biological activity of the active ingredient(s) to be unequivocally effective and which contain no additional component which would be toxic to subjects to which the said formulation would be administered.

## Compounds according to the invention

According to a particular aspect, are provided cathepsin inhibitors, in particular cathepsin S inhibitors for use in the prevention and/or treatment of a cancer, in particular in anti-cancer immunotherapy, wherein said cathepsin inhibitor is to be administered in combination with immunotherapy or in the prevention and/or treatment of an immunological disorder, such as auto-immune diseases.

Cathepsin inhibitors can be small molecules inhibitors, peptides, mAbs, chimeric proteins or fusion proteins, aptamers (including peptide aptamers, DNA and RNA aptamers), soluble receptors and such agents may be acting by silencing, or down-regulating Cathepsin at the genetic level by nucleic acid interaction, nucleic acid mutations or deletion aiming to abrogate the enzymatic activity of Cathepsin using CRISPR-Cas9 technologies or by systemic administration of RNA interference delivered by liposomes or nanoparticles in human.

According to a particular embodiment, Cathepsin S inhibitors according to the invention are according to Formula (I):

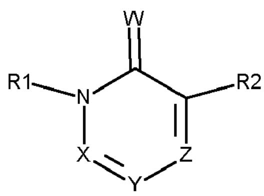

**(I)**

Wherein $R^1$ is optionally substituted $C_1$-$C_6$ alkyl, such as optionally substituted amino $C_1$-$C_6$ alkyl, optionally substituted aminocarbonyl $C_1$-$C_6$ alkyl, optionally substituted acylamino $C_1$-$C_6$ alkyl and optionally substituted amino-sulfonyl $C_1$-$C_6$ alkyl; $R^2$ is selected from H, optionally substituted $C_1$-$C_6$ alkyl optionally substituted aryl and optionally substituted heteroaryl; W is selected from O and S; X is selected from N and $CR^{11}$; Y is selected from N and $CR^{12}$ and Z is selected from N and $CR^{13}$, wherein $R^{11}$ is selected from H and optionally substituted $C_1$-$C_6$ alkyl, $R^{12}$ is selected from H, optionally substituted $C_1$-$C_6$ alkyl, optionally substituted aryl such as optionally substituted phenyl (e.g. methoxy phenyl like 4-methoxy phenyl, chlorophenyl like 2-chlorophenyl, 4-fluorophenyl and optionally substituted heteroaryl such as optionally substituted furanyl (e.g. optionally substituted 2-furanyl) or optionally substituted thiophene; or $R^2$ and $R^{13}$ can form an optionally substituted fused ring such as an optionally substituted heteroaryl like an optionally substituted thiazole (e.g. methyl thiazole fused to the diazinone ring) and an optionally substituted oxazole (e.g. methyl oxazole fused to the diazinone ring) as well as pharmaceutically acceptable salts, hydrates, solvates, tautomers, polymorphs, racemic mixtures, optically active forms, prodrugs and pharmaceutically active derivative thereof.

According to a further particular embodiment, $R^1$ is substituted $C_1$-$C_6$ alkyl such as $C_1$-$C_6$ alkyl (e.g. methyl, ethyl, propyl) substituted by optionally substituted acylamino or optionally substituted aminosulfonyl.

According to a further particular embodiment, $R^1$ is substituted $C_1$-$C_6$ alkyl such as $C_1$-$C_6$ alkyl (e.g. methyl, ethyl, propyl) substituted by optionally substituted aminosulfonyl such as aminocarbonyl substituted by optionally substituted phenyl (e.g. halo phenyl such as fluoro phenyl) or aminocarbonyl substituted by optionally substituted heteroaryl such as by ethyl pyridazinyl

According to a further particular embodiment, $R^1$ is substituted $C_1$-$C_6$ alkyl such as $C_1$-$C_6$ alkyl (e.g. methyl, ethyl, propyl) substituted by optionally substituted acylamino such as aminocarbonyl substituted by optionally substituted phenyl (e.g. methoxy phenyl, halo phenyl such as fluoro or chloro phenyl) or by optionally substituted heteroaryl $C_1$-$C_6$ alkyl (e.g. optionally substituted isoxazole methyl such as dimethyl isoxazole methyl)

According to a further particular embodiment, $R^1$ is substituted $C_1$-$C_6$ alkyl such as $C_1$-$C_6$ alkyl (e.g. methyl, ethyl, propyl) substituted by optionally substituted aminocarbonyl such as aminocarbonyl substituted by optionally substituted heteroaryl $C_1$-$C_6$ alkyl (e.g. optionally substituted pyridazine such as furanyl methyl).

According to a further particular embodiment, $R^1$ is substituted $C_1$-$C_6$ alkyl such as $C_1$-$C_6$ alkyl (e.g. methyl, ethyl, propyl) substituted by optionally substituted aminocarbonyl such as aminocarbonyl substituted by C3-C10 cycloalkyl (e.g. N-cyclooctyl).

According to a further particular embodiment, $R^1$ is selected from optionally substituted $C_1$-$C_6$ alkyl such as $C_1$-$C_6$ alkyl (e.g. methyl, ethyl, propyl) optionally substituted by one of the following groups: -NHC(O)-$R^{14}$, -O(CO)-$R^{14}$, -C(O)N$R^{14}R^{19}$ and -NHS$(O)_2$-$R^{14}$, wherein $R^{14}$ is selected from optionally substituted $C_4$-$C_{10}$ cycloalkyl, optionally substituted phenyl such as optionally substituted halogeno phenyl (e.g. 1-fluorophenly, 3 fluorophenyl, 1-chlorophenyl) or hydroxyphenyl (e.g. 3-hydroxyphenyl), optionally substituted heteroaryl such as optionally substituted thiophenyl (e.g. methyl thiophenyl) or optionally substituted heteroaryl $C_1$-$C_6$ alkyl such as optionally substituted isoxazole $C_1$-$C_6$ alkyl (e.g. di-methyl isoxazolyl methyl) or optionally substituted furanyl $C_1$-$C_6$ alkyl (e.g. furanyl methyl), $R^{19}$ is selected from H and $C_1$-$C_6$ alkyl or $R^{14}$ and $R^{19}$ can form an optionally substituted $C_3$-$C_{10}$ cycloalkyl (e.g. optionally substituted cyclononane) or an optionally substituted $C_3$-$C_8$ heterocycloalkyl.

According to a further particular embodiment, $R^2$ is H.

According to a further particular embodiment, $R^2$ and $R^{13}$ form an optionally substituted fused ring such as an optionally substituted thiazole (e.g. methyl thiazole fused to the diazinone ring).

According to a further particular embodiment, X is selected from N and CH.

According to a further particular embodiment, X is N.

According to a further particular embodiment, X is CH.

According to a further particular embodiment, Y is -C$R^{12}$.

According to a further particular embodiment, C$R^{12}$ is an optionally substituted heteroaryl such as optionally substituted furyl.

According to a further particular embodiment, C$R^{12}$ is an optionally substituted heteroaryl such as optionally substituted thienyl.

According to a further particular embodiment, $R^{13}$ is H.

According to a further particular embodiment, Z is selected from N and CH.

According to a further particular embodiment, Z is CH.

According to a further particular embodiment, compounds of Formula (I) are of the following Formula (Ia):

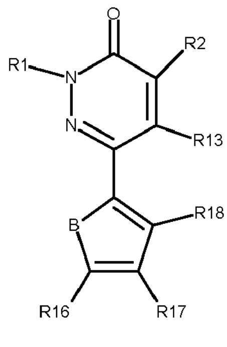

**(Ia)**

Wherein $R^2$ is selected from H and and optionally substituted $C_1$-$C_6$ alkyl, $R^{16}$ is selected from H and optionally substituted $C_1$-$C_6$ alkyl; $R^{17}$ is selected from H and optionally substituted $C_1$-$C_6$ alkyl, $R^{18}$ is selected from H and optionally substituted $C_1$-$C_6$ alkyl, B is selected from O and S and $R^1$ and R3 are as defined herein.

According to a further particular embodiment, $R^1$ is selected from methyl, ethyl or propyl substituted by -NHC(O)-$R^{14}$, -C(O)N$R^{14}R^{19}$ and -S$(O)_2$-$R^{13}$.

According to a further particular embodiment, $R^{16}$, $R^{17}$ and $R^{18}$ are H.

According to a further particular embodiment, $R^2$ and $R^{13}$ are H.

According to a further particular embodiment, compounds of Formulae (I) or (Ia) are selected from the following group:

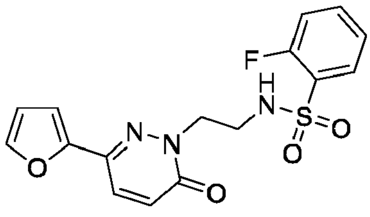

**(1)**

(2-fluoro-N-[2-[3-(2-furanyl)-6-oxo-1(6H)-pyridazinyl] ethyl]-benzenesulfonamide, CAS registry n° 946263-96-3);

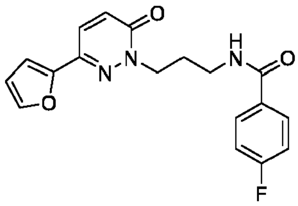

**(2)**

(4-fluoro-N-[3-[3-(2-furanyl)-6-oxo-1(6H)-pyridazinyl]propyl]-benzamide, CAS registry n° 1021225-32-0);

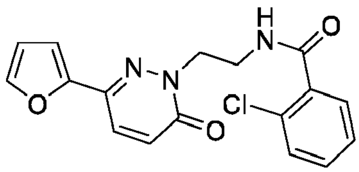

**(3)**

(2-chloro-N-[2-[3-(2-furanyl)-6-oxo-1(6H)-pyridazinyl]ethyl] -benzamide, CAS registry n° 946365-20-4);

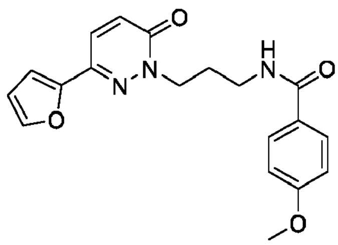

**(4)**

(N-[3-[3-(2-furanyl)-6-oxo-1(6H)-pyridazinyl]propyl]-4-methoxy-benzamide, CAS registry n° 1021225-23-9);

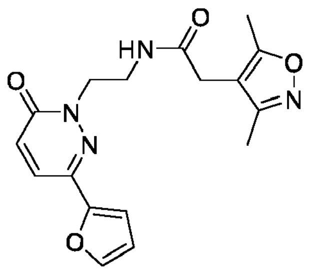

**(5)**

(N-[2-[3-(2-furanyl)-6-oxo-1(6H)-pyridazinyl]ethyl]-3,5-dimethyl-4-isoxazoleacetamide, CAS registry n° 1219845-08-5);

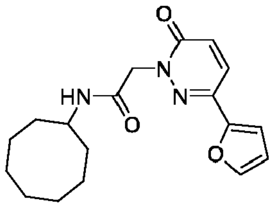

(6)

(N-cyclooctyl-3-(2-furanyl)-6-oxo-1(6H)-pyridazineacetamide, CAS registry n° 1282103-56-3);

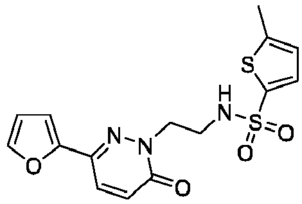

(7)

(N-[2-[3-(2-furanyl)-6-oxo-1(6H)-pyridazinyl]ethyl]-5-methyl-2-thiophenesulfonamide, CAS registry n° 946212-65-3); and

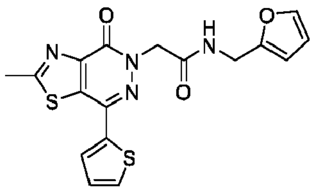

(8)

(N-(2-furanylmethyl)-2-methyl-4-oxo-7-(2-thienyl)-thiazolo[4,5-d]pyridazine-5(4H)-acetamide, CAS registry n° 942004-60-6), as well as pharmaceutically acceptable salts, hydrates, solvates, tautomers, polymorphs, racemic mixtures, optically active forms prodrugs and pharmaceutically active derivative thereof.

According to another particular embodiment, Cathepsin S inhibitors according to the invention are according to Formula (II):

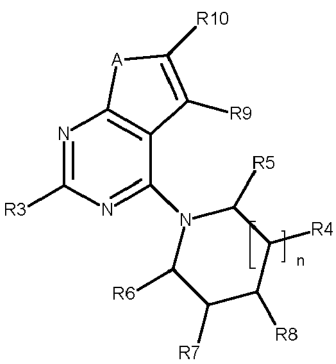

**(II)**

Wherein A is selected from O, S, N and $CR^{20}$; $R^3$ is selected from H, optionally substituted $C_1$-$C_6$ alkyl such as methyl; $R^4$ is selected from H and optionally substituted $C_1$-$C_6$ alkyl; $R^5$ and $R^6$ are independently selected from H, optionally substituted $C_1$-$C_6$ alkyl, $R^7$ is selected from $-C(O)NR^{21}R^{22}$, $-C(O)OR^{21}$ and $-S(O_2)R^{21}$; $R^8$ is selected from H and optionally substituted $C_1$-$C_6$ alkyl; $R^9$ and $R^{10}$ are independently selected from optionally substituted $C_1$-$C_6$ alkyl such as methyl, $R^{21}$ is selected from H and optionally substituted $C_1$-$C_6$ alkyl; $R^{22}$ is selected from optionally substituted optionally substituted heteroaryl $C_1$-$C_6$ alkyl such as optionally substituted furanyl $C_1$-$C_6$ alkyl, for example optionally substituted furanyl methyl or ethyl; n is an integer selected from 0, 1 and 2, as well as pharmaceutically acceptable salts, hydrates, solvates, tautomers, polymorphs, racemic mixtures, optically active forms prodrugs and pharmaceutically active derivative thereof.

According to a further particular embodiment, $R^3$ is selected from H and optionally substituted $C_1$-$C_6$ alkyl such as methyl.

According to a further particular embodiment, A is O.

According to another further particular embodiment, $R^4$ is H.

According to another further particular embodiment, $R^5$ is H.

According to another further particular embodiment, $R^6$ is H.

According to a further particular embodiment, $R^7$ is -C(O)NHR$^{22}$.

According to another further particular embodiment, $R^6$ is H.

According to another further particular embodiment, $R^8$ is H.

According to another further particular embodiment, n is 1.

According to a further particular embodiment, compounds of Formula (II) are selected from the following group:

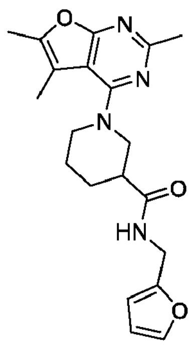

**(9)**

(N-(2-furanylmethyl)-1-(2,5,6-trimethylfuro[2,3-d]pyrimidin-4-yl)-3-piperidinecarboxamide, CAS registry n°1114614-87-7);

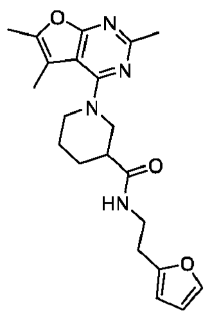

**(10)**

(N-[2-(2-furanyl)ethyl]-1-(2,5,6-trimethylfuro[2,3-d]pyrimidin-4-yl)-3-piperidinecarboxamide, CAS registry n°1111056-12-2); and

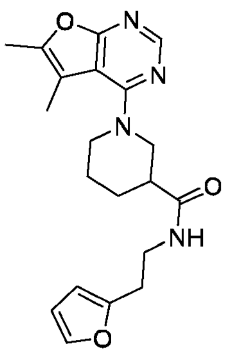

**(11)**

(1-(5,6-dimethylfuro[2,3-d]pyrimidin-4-yl)-N-[2-(2-furanyl)ethyl]-3-piperidinecarboxamide, CAS registry n°1114632-87-9), as well as pharmaceutically acceptable salts, hydrates, solvates, tautomers, polymorphs, racemic

mixtures, optically active forms prodrugs and pharmaceutically active derivative thereof.

Further inhibitors can be identified by a method of the invention as described below or alternatively, further inhibitors can be identified by *in silico* models, for example such as described in Example 2.

According to another aspect of the invention, it is provided a peptide having a sequence comprising a sequence of **SEQ ID NO: 24** (KLRMKLPK(Xaa)), wherein Xaa is any amino acid, wherein sais amino acid can be a natural or a non-natural amino acid. According to a particular embodiment, a non-natural amino acid can be selected from those described in Geurink et al., 2013, J. Med. Chem., 56, 1262 or Rand et al., 2012, Med. Chem. Commun, 3, 1282.

According to another aspect of the invention, it is provided a peptide comprising a sequence of **SEQ ID NO: 24,** wherein said peptide comprises or consists in a sequence selected from the following group: **SEQ ID NO: 1** (KLRMKLPKP); **SEQ ID NO: 2** (KLRMKLPKD) and **SEQ ID NO: 3** (KLRMKLPKK).

According to another aspect of the invention, peptides comprising or consisting in a sequence of **SEQ ID NO: 1-3** are cathepsin S and are cathepsin H substrates.

According to another aspect of the invention, peptides of the invention as cathepsin substrates are from about 9 to about 20 amino acid long.

According to another aspect of the invention, it is provided a peptide comprising or consisting in a sequence of **SEQ ID NO: 4** (KPPKPVSD) **SEQ ID NO: 5** (KPPKPVSD) or a variant thereof.

According to another aspect of the invention, a peptide comprising a sequence of **SEQ ID NO: 4** or of **SEQ ID NO: 5** is a Cathepsin C and Cathepsin B substrate.

According to another aspect of the invention, peptides of the invention are labelled with a fluorescent group on the first amino acid (5') or a fluorescence quencher group attached to the last amino acid of the peptide (3').

According to another aspect of the invention, is provided a use of a peptide having a sequence comprising or consisting in a sequence selected from **SEQ ID NO: 1, SEQ ID NO: 2, SEQ ID NO: 3, SEQ ID NO: 4** and **SEQ ID NO: 5** or a variant as a Cathepsin enzyme substrate.

## Synthesis of compounds of the invention

The compounds of the invention can be prepared from readily available starting materials using the following general methods and procedures. It will be appreciated that where typical or preferred experimental conditions (i.e. culture or reaction temperatures, time, moles of reagents, solvents etc.) are given, other experimental conditions can also be used unless otherwise stated. Optimum reaction conditions may vary with the particular reactants or solvents used, but such conditions can be determined by the person skilled in the art, using routine optimization procedures.

Examples of synthesis of compounds of the invention can be found for pyridazones in Ameriks et al., 2010, Bioorganic & Medicinal Chemistry Letters 20, 4060-4064 *or* Coates et al., Synthesis, 1993, 03: 334-342. For pyrimidines of the invention (Formula (II)), an example of a synthetic route is described as follows:

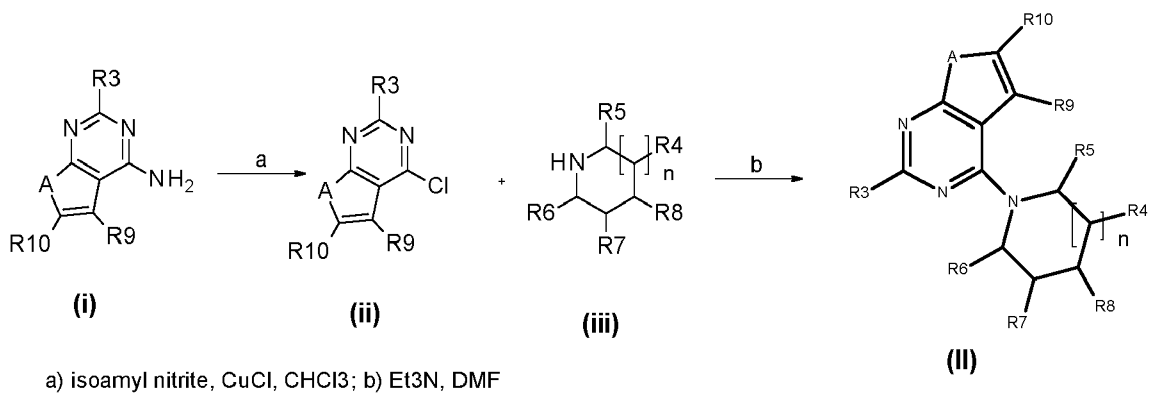

(i) (ii) (iii) (II)

## Compositions according to the invention

The invention provides pharmaceutical or therapeutic agents as compositions and methods for treating a subject, preferably a mammalian subject, and most preferably a human patient who is suffering from a medical disorder, and in particular a cancer, in particular a solid tumor cancer.

In one embodiment, the invention provides a pharmaceutical composition containing at least one compound of the invention and a pharmaceutically acceptable carrier, diluent or excipient thereof.

Agent of the invention or formulations thereof may be administered as a pharmaceutical formulation, which can contain one or more agents according to the invention in any form described herein. The compositions according to the

invention, together with a conventionally employed adjuvant, carrier, diluent or excipient may be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, or in the form of sterile injectable solutions for parenteral (including subcutaneous) use by injection or continuous infusion. Injectable compositions are typically based upon injectable sterile saline or phosphate-buffered saline or other injectable carriers known in the art. Such pharmaceutical compositions and unit dosage forms thereof may comprise ingredients in conventional proportions, with or without additional active compounds or principles, and such unit dosage forms may contain any suitable effective amount of the active ingredient commensurate with the intended daily dosage range to be employed.

Compositions of this invention may be liquid formulations including, but not limited to aqueous or oily suspensions, solutions, emulsions, syrups, and elixirs. The compositions may also be formulated as a dry product for reconstitution with water or other suitable vehicle before use. Such liquid preparations may contain additives including, but not limited to, suspending agents, emulsifying agents, non-aqueous vehicles and preservatives. Suspending agents include, but are not limited to, sorbitol syrup, methylcellulose, glucose/sugar syrup, gelatin, hydroxyethyl cellulose, carboxymethyl cellulose, aluminum stearate gel, and hydrogenated edible fats. Emulsifying agents include, but are not limited to, lecithin, sorbitan monooleate, and acacia. Preservatives include, but are not limited to, methyl or propyl p-hydroxybenzoate and sorbic acid. Dispersing or wetting agents include but are not limited to poly(ethylene glycol), glycerol, bovine serum albumin, Tween®, Span®.

Compositions of this invention may also be formulated as a depot preparation, which may be administered by implantation or by intramuscular injection.

Solid compositions of this invention may be in the form of tablets or lozenges formulated in a conventional manner. For example, tablets and capsules for oral administration may contain conventional excipients including, but not limited to, binding agents, fillers, lubricants, disintegrants and wetting agents. Binding agents include, but are not limited to, syrup, accacia, gelatin, sorbitol, tragacanth, mucilage of starch and polyvinylpyrrolidone. Fillers include, but are not limited to, lactose, sugar, microcrystalline cellulose, maize starch, calcium phosphate, and sorbitol. Lubricants include, but are not limited to, magnesium stearate, stearic acid, talc, polyethylene glycol, and silica. Disintegrants include, but are not limited to, potato starch and sodium starch glycollate. Wetting agents include, but are not limited to, sodium lauryl sulfate. Tablets may be coated according to methods well known in the art.

The compounds of this invention can also be administered in sustained release forms or from sustained release drug delivery systems.

According to a particular embodiment, compositions according to the invention are for intravenous use.

According to a particular embodiment, compositions according to the invention are for intraperitoneal use.

According to a particular embodiment, compositions according to the invention are for intratumoral use.

In another particular aspect, the compositions according to the invention are adapted for delivery by repeated administration.

According to a particular embodiment, compositions of the invention are veterinary compositions.

Further materials as well as formulation processing techniques and the like are set out in *Part 5 of* Remington's "The Science and Practice of Pharmacy", 22nd Edition, 2012, University of the Sciences in Philadelphia, Lippincott Williams & Wilkins, which is incorporated herein by reference.

## Mode of administration

Cathepsin inhibitors of the invention can be administered in humans alone or in combination with immune modulators (e.g. anti-PD1, anti-PDL1, anti-CTLA4)

Compounds and formulations thereof according to this invention may be administered in any manner including parenterally, intravenously, intra-tumorally, intrathecally, transmucosally, intranasally, rectally, or combinations thereof. Parenteral administration includes, but is not limited to, intravenous, intra-arterial, intra-peritoneal, subcutaneous and intramuscular. The compositions of this invention may also be administered in the form of an implant, which allows slow release of the compositions as well as a slow controlled i.v. infusion.

The dosage administered, as single or multiple doses, to an individual will vary depending upon a variety of factors, including pharmacokinetic properties, patient conditions and characteristics (sex, age, body weight, health, size), extent of symptoms, concurrent treatments, frequency of treatment and the effect desired.

## Combination

According to one aspect, compounds of the invention are to be administered in further combination with at least one therapeutic strategy useful in the prevention and/or treatment of a cancer, in particular an anti-cancer immunotherapy such as ACT therapy or immune checkpoint blockade therapy.

According to another particular aspect, the compounds of the invention are to be administered in combination with ACT therapy.

According to another particular aspect, the compounds of the invention are to be administered in combination with at least one immune checkpoint inhibitor.

According to one aspect is provided a pharmaceutical composition comprising at least one Cathepsin inhibitor and a pharmaceutically acceptable carrier, diluent or excipient thereof and at least one agent useful in ACT therapy.

According to one aspect is provided a pharmaceutical composition comprising at least one Cathepsin inhibitor and a pharmaceutically acceptable carrier, diluent or excipient thereof and at least one immune checkpoint inhibitor.

The invention encompasses the administration of a compound of the invention or a formulation thereof wherein it is administered to a subject prior to, simultaneously or sequentially with other therapeutic regimens or co-agents useful for preventing, treating, and/or stabilizing a cancer such as anti-cancer treatments.

A compound of the invention or a formulation thereof according to the invention that is administered simultaneously with said co-agents can be administered in the same or different composition(s) and by the same or different route(s) of administration.

According to one embodiment, is provided a pharmaceutical formulation comprising a compound of the invention combined with at least one co-agent useful for treating, and/or stabilizing, a neurodegenerative disorder and at least one pharmaceutically acceptable carrier.

**Use of compounds according to the invention**

The invention provides Cathepsin inhibitors for use in the prevention and/or treatment of a cancer, when used in combination with immunotherapy.

The invention provides Cathepsin inhibitors for treatment of an immunological disorder. According to another aspect, a peptide of the invention can be usefully used as Cathepsin substrate. In particular, peptides of the invention are useful for monitoring the activity of the Cathepsin enzyme they are a substrate for in presence or absence of a candidate compound. According to a further particular embodiment, the peptides of the invention are useful in a method of the invention for identifying specific inhibitors of the Cathepsin enzyme they are a substrate for.

**Kits of the invention**

According to one aspect of the invention, is provided a kit for assaying Cathepsin enzyme activity, said kit comprising at least one peptide of the invention.

According to a particular aspect, said kit further comprises at least one mutated cathepsin S Y132D purified protein and at least one wild-type cathepsin.

According to a particular aspect of the invention is provided a kit of the invention further comprising or more of the following:

- An activation buffer for cathepsin S, for example an activation Buffer A comprising NaOAc 50 mM, DTT 5 mM, NaCl 250 Mm, at pH 4.5;
- An activation buffer for cathepsin B, such as an activation buffer B comprising25 mM MES, 5 mM DTT, at pH 5;
- an activation buffer H, for example an activation buffer C comprising 50 mM MES, 10 mM $CaCl_2$, 150 mM NaCl, pH 6.5;
- A stopping buffer for cathepsin H assay, for example a stopping buffercomprising contains 50 mM MES, 10 mM $CaCl_2$, 150 mM NaCl, 20 mM DTT.

According to one aspect of the invention, is provided a kit for assaying Cathepsin S enzyme activity, said kit comprising at least one peptide comprising or consisting in a sequence selected from **SEQ ID NO: 1-4.**

According to one aspect of the invention, is provided a kit for assaying Cathepsin H enzyme activity, said kit comprising at least one peptide comprising or consisting in a sequence selected from **SEQ ID NO: 1-3.**

According to one aspect of the invention, is provided a kit for assaying Cathepsin B enzyme activity, said kit comprising a peptide comprising or consisting in a sequence of **SEQ ID NO: 5.**

According to one aspect of the invention, a kit according to the invention is useful for identifying agents that would inhibit or activate Cathepsin enzyme activity.

According to another aspect of the invention, a kit according to the invention is useful in a method of identifying a Cathepsin enzyme inhibitor.

**Methods according to the invention**

According to another aspect, the invention provides a method of preventing or treating a cancer, in particular

a solid tumor cancer or hematological cancers.

According to another aspect, the invention provides a method of preventing and/or treating a blood cancer, lung cancer (small cell and non-small cell), breast cancer, prostate cancer, cancer, ovarian cancer, cervical cancer, uterus cancer, head and neck cancer, glioblastoma, hepatocellular carcinoma, colon cancer, rectal cancer, colorectal carcinoma, kidney cancer, gastric cancer, bronchus cancer, pancreatic cancer, urinary bladder cancer, hepatic cancer and brain cancer skin cancer.

According to another aspect, the invention provides a method of preventing or treating an immunological disorder.

According to another aspect, the invention provides a method of identifying agents useful in the potentiation of immunotherapy, said method comprising the following steps:

a) providing an activated Cathepsin enzyme selected from Cathepsin S, Cathepsin B and Cathepsin H;
b) providing a Cathepsin substrate for said Cathepsin enzyme;
c) contacting said activated Cathepsin enzyme with said substrate in presence or absence of at least one candidate agent;
d) measuring Cathepsin enzyme activity in presence or absence of at least one candidate agent through the detection of fluorescence emission or by mass spectrometry;
e) identifying agents that are able to inhibit Cathepsin, wherein said Cathepsin substrate is at least one peptide comprising or consisting in a sequence selected from **SEQ ID NO: 1, SEQ ID NO: 2, SEQ ID NO: 3, SEQ ID NO: 4,** and **SEQ ID NO: 5** or variant thereof when said Cathepsin enzyme is Cathepsin S, wherein said Cathepsin substrate is at least one peptide comprising or consisting in a sequence selected from **SEQ ID NO: 1, SEQ ID NO: 2** and **SEQ ID NO: 3,** or variant thereof wherein said Cathepsin enzyme is Cathepsin H and wherein said Cathepsin substrate is a peptide comprising or consisting in a sequence selected from **SEQ ID NO: 4 and SEQ ID NO: 5** or variant thereof, wherein said Cathepsin enzyme is Cathepsin B, said peptide further comprise a fluorescent label if Cathepsin enzyme activity is measured by fluorescence emission.

According to another aspect, the invention provides a method of identifying agents, wherein said Cathepsin enzyme is essentially pure (e.g. 90-95% purity).

According to a particular embodiment, activated Cathepsin S enzyme can be obtained by incubating CTSS in an activation buffer such as an activation buffer comprising NaOAc 50 mM, DTT 5 mM, NaCl 250 Mm, at pH 4.5.

According to a further particular embodiment, is provided a method according to the invention wherein activated Cathepsin S enzyme is provided at a concentration of about 0.5 μg/ml in a buffer. Typically, Cathepsin S enzyme is incubated at 37°C for about 15-90 minutes in the activation buffer.

According to a particular embodiment, measuring Cathepsin enzyme activity can be performed by fluorescence Resonance Energy Transfer (FRET) (e.g. by measuring fluorescence emission at 390 nm if the substrate is conjugated with MCA fluorophore and DNP quencher).

According to a particular embodiment, activated Cathepsin B enzyme can be obtained by incubating CTSB in an activation buffer such as an activation buffer comprising 25 mM MES, 5 mM DTT, at pH 5.

According to a further particular embodiment, is provided a method according to the invention wherein activated Cathepsin B enzyme is provided at a concentration of about 1 μg/ml in a buffer.

Typically, Cathepsin B enzyme is incubated at 37C for about 60-120 minutes in the activation buffer.

According to a particular embodiment Cathepsin H is activated in a buffer containing 50 mM MES, 10 mM $CaCl_2$, 150 mM NaCl, pH 6.5.

According to a further particular embodiment, is provided a method according to the invention wherein activated Cathepsin H enzyme is provided at a concentration of about 200 μg/ml in a buffer.

According to a further particular embodiment, for activating Cathepsin H enzyme, a zinc protease such as thermolysin is added to Cathepsin H enzyme with equal volume of Cathepsin H and incubated for about 1h at room temperature. Then, Phosphoramidon is added to the mixture for about 10 minutes and then a stopping buffer (e.g.50 mM MES, 10 mM $CaCl_2$, 150 mM NaCl, 20 mM DTT). The final concentration of the activated cathepsin H is at about 25 μg/ml and is provided at such concentration under step a.

Typically, a buffer useful in a method of the invention is part of the common knowledge of a skilled person.

## Patients

In an embodiment, patients according to the invention are suffering from any type of cancer. In an embodiment, patients according to the invention are suffering from any type of cancer at any stage, including non-metastatic and metastatic.

In a particular embodiment, patients according to the invention are suffering from blood, lung cancer (small cell

and non-small cell), breast cancer, prostate cancer, cancer, ovarian cancer, cervical cancer, uterus cancer, head and neck cancer, glioblastoma, hepatocellular carcinoma, colon cancer, rectal cancer, colorectal carcinoma, kidney cancer, gastric cancer, bronchus cancer, pancreatic cancer, urinary bladder cancer, hepatic cancer and brain cancer or skin cancer.

In a further particular embodiment, patients according to the invention are suffering from hematological cancer.

In a further particular embodiment, patients according to the invention are suffering from B-cell lymphoma such as follicular lymphoma and diffuse large B-cell lymphoma.

In a further particular embodiment, patients according to the invention are suffering from solid tumor cancer.

In a further embodiment, subjects according to the invention are suffering from or at risk of suffering from a cancer.

Compounds, compositions and methods according to the invention are particularly useful for References cited herein are hereby incorporated by reference in their entirety. The present invention is not to be limited in scope by the specific embodiments and drawings described herein, which are intended as single illustrations of individual aspects of the invention, and functionally equivalent methods and components are within the scope of the invention. The examples illustrating the invention are not intended to limit the scope of the invention in any way.

## EXAMPLES

The following abbreviations refer respectively to the definitions below: **ACN** (acetonitrile); **BSA** (bovine serum albumin); **DAPI** (4',6-diamidino-2-phenylindole); **DNP** (2,4-dinitrophenyl); **DTT** (DL-Dithiothreitol); **EDTA** (Ethylenediaminetetraacetic acid); **FACS** (Fluorescence Activated Cell Sorting); **MCA** (7-Methoxycoumarin-4-yl)acetyl); **MES** (2-(N-morpholino)ethanesulfonic acid); **PBS** (phosphate-buffered saline); **WT** (wildtype).

The following studies are conducted to support the effectiveness of compounds of the invention according to the invention.

### Example 1: Analysis of Cathepsin S Y132D Hotspot mutation in FL and DLBCL samples

Cathepsins are a large family of cysteine proteases frequently over-expressed in multiple cancer types but rarely mutated *(*Reiser et al., 2010, J. Clin. Invest. 120, 3421-3431*;* Olson and Joyce, 2015, Nat. Rev. Cancer, 15, 712-729*).* Since *CTSS* (cathepsin S) activity is important in normal humoral response and B-cell expansion *(Shi et al., 1999, supra),* the whole exome and whole genome sequencing data from multiple studies including both FL and DLBCL samples were analysed *(*Araf et al., 2018, Leukemia 32, 1261-1265*;* Green et al., 2013, Blood 121, 1604-1611*;* Kridel et al., 2016b; PLOSMed. 13, e1002197*;* Morin et al., 2011; Nature 476, 298-30*;* Okosun et al., 2014, Nat. Genet. 46, 176-181*;* Ortega-Molina et al., 2015, Nat. Med. 21, 1199-1208*;* Reddy et al., 2017, Cell 171, 481-494.e15*;* Schmitz et al., 2018, N. Engl. J. Med. 378, 1396-1407*).* Interestingly, *CTSS* was observed to be mainly mutated in FL samples. Notably, 70% (15/20) of the cases with mutated *CTSS* harbor a specific T to G mutation that changes the tyrosine 132 in aspartate (Y132D, c.394T>G).Y132D mutations were observed in 5% of FL analyzed cases (15/299), while CTSS was never mutated in DLBCL, except for one case. Moreover, whole genome sequencing analyses of an additional independent cohort including 75 FL and normal matching samples confirmed that Y132 residue is somatically mutated in 5.3% of the samples (4/75 samples, 3 samples Y132D and 1 sample Y132N). The allele frequency of Y132D mutations is similar to others commonly mutated genes in FL such as KMT2D or CREBBP. Interestingly, phylogenetic analysis of protein sequence in mammals shows that tyrosine 132 is highly conserved and present only in CTSS. Indeed, structure based sequence alignment of eight human mature cathepsins shows that the majority of them have an asparagine (N) in this position, while *CTSF* (cathepsin F) and *CTSB* (cathepsin B) encode for an aspartate (D) and only *CTSS* encodes a tyrosine (Y) *(*Turk et al., 2012, Biochim. Biophys. Acta BBA - Proteins Proteomics, 1824, 68-88), suggesting a functional and specific effect of the Y132D mutation on CTSS activity.

CTSS is synthetized as an inactive enzyme (pro-CTSS). To be converted into an active enzyme, the inhibitory pro-peptide located at the N-terminus of pro-CTSS is cleaved by autocatalytic endopeptidase activity, allowing the release of the activated protein (mature CTSS) *(Turk et al., 2012, supra;* Vasiljeva et al., 2005, FEBS Lett., 579, 1285-1290).

To functionally assess the effect of Y132D mutations on CTSS activity, first, polyhistidine-tagged CTSS wild-type (CTSS-His-WT) and mutated (CTSS-His-Y132D) proteins were expressed and purified and the effect of Y132D mutation was monitored based on protein stability and activity as described herein. To test the consequence of Y132D mutation on CTSS auto-activation, the recombinant wild-type and mutated proteins for 90 minutes was incubated at different pH (6.5, 5.5, and 4.5) and measured the amounts of the pro-CTSS and mature forms as described herein. In all tested condition, the fraction of mature protein was greater for the mutated than for the wild-type CTSS, indicating a more efficient autocatalytic processing and activation of the mutated protein.

Next, the ability of wild-type and mutated CTSS to bind and cleave specific substrates were compared. To this purpose, CD74 was used as a known CTSS substrate and algorithmically predicted the most likely cleavage site based on 3'804 known CTSS cleavage sites (Vizovišek et al., 2015, PROTEOMICS 15, 2479-2490) ("STAR" Methods below)

as describe in Example 2. Out of the 290 possible sequence octamers of CD74, the peptide of **SEQ ID NO: 1** (KLRMKLPKP) scored as most probable CTSS cleavage site, in agreement with a previously published study (Villadangos et al., 1997, J. Exp. Med., 186, 549-560), Moreover, a variant of SEQ ID NO: 1 was identified an improved cathepsin S substrate as a peptide of **SEQ ID NO: 2** (KLRMKLPKD) while the CLIP-juxtaposed peptide of **SEQ ID NO: 4** (KPPKPVSK) scored $25^{th}$ out of 290, it was shown to be a good substrate for Cathepsin B Both **peptides of SEQ ID NO: 1 and 4** were predicted *in silico* with an ability to bind the catalytic pocket of CTSS and the ability of CTSS to cleave them was experimentally measured using a FRET (Fluorescence Resonance Energy Transfer) assay as described below **(Fig. 1).** The ability of CTSS to cleave **SEQ ID NO: 1, SEQ ID NO: 2** and **SEQ ID NO: 3 (Fig. 2a)** and other cathepsins as CTSH to cleave **SEQ ID NO: 2 (Fig. 2c)** and CTSB to cleave **SEQ ID NO: 5 (Fig. 2b)** have been tested. Docking analysis showed that the top scoring peptide of **SEQ ID NO: 1** fits in the CTSS wild-type binding pocket, while the CLIP-juxtaposed peptide of **SEQ ID NO: 4** does not dock properly likely due to a proline in P1' position that hinders the ability of the other amino acids to fit properly in the substrate biding pocket of CTSS. Similarly, the FRET assay showed that CTSS-His-WT cleaved more efficiently the top scoring peptide of **SEQ ID NO: 1** than the CLIP-juxtaposed peptide of **SEQ ID NO: 4.** In a method of identifying a Cathepsin inhibitor according to the invention by FRET as described below, E64 (CAS:66701-25-5) (Selleckchem, cat #S7379), a generic protease inhibitor, was used as control to block CTSS activity. Moreover, to allay not specific effect due to in-house protein purification protocol, the assay was repeated with a commercially available CTSS recombinant protein (R&D Systems cat: 1183-CY-010; https://www.rndsystems.com/products/recombinant-human-cathepsin-s-protein-cf_1183-cy and similar results were observed. Based on this evidence, a peptide of **SEQ ID NO: 1** was used to compare the endopeptidase activity of wild type versus mutated CTSS by FRET. Here, a strikingly increased activity of the mutated versus the wild-type protein was detected. Indeed, CTSS-His-Y132D cleaved the peptide faster and more efficiently than wild-type CTSS-His-WT **(Fig. 1).** Overall, it was demonstrated that *CTSS*$^{Y132D}$ promotes activation of the protein and it enhances its proteolytic activity, supporting an oncogenic role for this mutation in FL.

Further, this supports the usefulness of the identified peptide as a specific substrate for CTSS-His-Y132D, in particular for use in an assay to identify inhibitors according to the invention.

***STAR Methods***

**Identification of CTSS mutations in FL primary samples**

A panel comprised 77 fresh-frozen biopsies with matched normal samples (from peripheral blood) obtained at FL diagnosis were analyzed by whole genome sequencing. After quality control, 2 samples were excluded from further analyses because of poor quality sequencing and only 75 FL samples were considered. Patients were diagnosed according to the 2008 WHO classification, as determined by standardised review by expert hematopathologists. Paired-end whole genome sequencing (WGS) libraries were prepared following the standard preparation and single nucleotide variants (SNVs) and short insertions and deletions (InDels) were identified using SAMtools mpileup followed by MutationSeq for somatic variants and Strelka tool after filtering against known polymorphisms as described in details in (Chun et al., 2016, Cancer Cell, 29, 394-406). Moreover, RNA-sequencing was obtained for the same samples and raw reads were aligned to the reference human genome assembly GRCh37 (hg19) using STAR version 2.5.2.a.

***Protein expression and purification.***

For CTSS purification, the human CTSS sequence depleted of the lysosomal localization signal (amino acids 17 to 331) was amplified from pLVX plasmids expressing WT or Y132D CTSS using the following primers (the pLVX plasmid backbone is commercially available from Takara (https://www.takarabio.com/assets/documents/Vector%20Documents/pLVX-Puro%20Vector%20Information.pdf) and the plasmid was modified to insert CTSS WT and Y132 sequence and the following primer were used to mutagenize CTSS sequence as described in QuickChangeII mutagenesis kit cat # 200521. CTSS_pHLsec_fw: GCTGAAACCGGTCAGTTGCATAAAGATCCTAC **(SEQ ID NO: 6),** CTSS_pHLsec_rev: GTGCTTGGTACCGATTTCTGGGTAAGAGGGAAAGC **(SEQ ID NO: 7).**

The PCR products were digested using AgeI and Kpn1 restriction enzymes (NEB, cat #R3552S and #R3142S), then cloned in pHLsec plasmids carrying a secretion signal at the N-terminal of the MCS and a His-tag sequence at the C-terminal of the MCS. pHLsec-CTSS plasmids were transfected into HEK293 cells with PEI (polyethyleneimine) in presence of VPA (valproic acid). Proteins were purified from the supernatant using a 5 ml His-Trap FF column on an ÄKTA pure system (GE healthcare). Bound proteins were eluted using a 300 mM imidazole solution. Concentrated proteins were further purified by size exclusion chromatography on a HiLoad® 16/600 Superdex® 75 pg column (GE Healthcare) using PBS as mobile phase. Purified proteins were quantified by Nanodrop, diluted in a buffer containing 12.5 mM MES, 75 mM NaCl and 50% glycerol at ph 6.5 and stored at -20°C.

### *FRET (Fluorescence Resonance Energy Transfer)*

CTSS-His WT and Y132D were diluted to 10 μg/mL in Assay buffer (50 mM NaOAc, 5 mM DTT, 250 mM NaCl), at pH 4.5 and incubated at 37°C for 90 minutes to allow auto-activation. The proteins were then diluted to 0.5 ng/μl in Assay buffer and plated in 96-well plates (50 μl/well). The peptides of **SEQ ID NO: 1, SEQ ID NO: 2, SEQ ID NO: 3, SEQ ID NO: 4 and SEQ ID NO: 5** were synthesized and diluted to 20 μM in a buffer (50 mM NaOAc, 5 mM DTT, 250 mM NaCl) then added to the activated CTSS (50 μl/well). 50 μM E64 (Selleckchem, cat #S7379) was added to CTSS prior to the substrate as positive control. A commercially available CTSS purified protein (R&D systems, cat #1183-CY-010) was in addition used as control. MCA emission was measured with SpectraMax iD3 (Molecular Devices) in kinetic mode for 20 minutes (excitation: 330 nm, emission: 390 nm).

### *CTSS auto-activation studies*

To study the CTSS WT and Y132D auto-catalytic activity, the purified proteins were incubated at 10 μg/mL in assay buffer (50 mM NaOAc, 5 mM DTT, 250 mM NaCl) adjusted to pH 4.5, 5.5 or 6.5 using NaOH, at 37°C for 90 minutes. Proteins were then boiled in Laemmli buffer and used for western blot analysis (200 ng/well).

### *Protein stability studies*

The thermal stability of CTSS WT and Y132D was determined using far-UV circular dichroism measurement. Briefly, proteins were diluted in PBS buffer (pH7.4) at a concentration of 15 μM, and the thermal denaturation curves were collected by measuring the change in ellipticity at 218 nm from 20 to 95°C with 2°C increments. Data were acquired on a Chirascan™ V100 Spectrometer (Applied Photophysics) in a 1 mm path-length quartz cuvette.

### *CTSS electrostatic potential calculation*

The electrostatic potential of WT and Y132D CTSS was calculated with the Adaptive Poisson-Boltzmann Solver (APBS) program *(*Baker et al., 2001, roc. Natl. Acad. Sci., 98, 10037-10041*)* through the UCSF Chimera molecular visualization software *(*Pettersen et al., 2004, J. Comput. Chem., 25, 1605-1612), using values of 1.0 and 78.54 for the solute and solvent dielectric constants, respectively. Before APBS calculations, the PDB2PQR *(*Dolinsky et al., 2007, Nucleic Acids Res. 35, W522-W525 *and* 2004, Nucleic Acids Res. 32, W665-667) was used to add missing heavy atoms and hydrogens, and to assign atomic charges and radii with the PARSE force field (Sitkoff et al., 1994, Biophys. J., 67, 2251-2260*).* PROPKA version 3.0 was used to assign the protonation states of titratable residues at pH 7.0 *(*Li et al., 2005, Proteins 61, 704-721*;* Sondergaard et al., 2011, J. Chem. Theory Comput., 7, 2284-2295) Calculation were performed based on the experimental structure of human Cathepsin S, PDB ID 1npz. *(*Pauly et al., 2003, Biochemistry, 42, 3203-3213*)* The Y132D mutation was introduced using the *swapaa* module of UCSF Chimera and the Dunbrack backbone-dependent rotamer library *(*Shapovalov and Dunbrack, 2011, Struct. Lond. Engl., 1993 19, 844-858).

### Example 2 Prediction of possible CTSS cleavage sites in a protein sequence

3'804 known cleavage sites of CTSS were retrieved from the supplementary material of *(Vizovišek et al., 2015, supra*), in the form of peptide octomers spanning residues P4 to P4'. These data were used to calculate the Position Weight Matrix (PWM) of CTSS cleavage site, i.e. the frequencies $PWM_{S,aa}$ with which amino acid *aa* is found in position P of the peptide, with P taking values from P4 to P4' and *aa* from all possible 20 natural amino acids. By definition, for a given position P:

$$\sum_{20\ possible\ aa} PWM_{P,aa} = 1$$

**(1)**

The scoref of a given peptide octamer is defined as a possible CTSS substrate, as the sum of the PWM values for each amino acid actually present in position S4 to S4', divided by the frequency of that residue in UniproKB/Swiss-Prot entries, $US_{aa}$. If *aa*(*P*) is the amino acid present in position P of a given peptide *Pep, f*(*Pep*) is calculated as

$$f(Pep) = \sum_{P \in [P4, P4']} PWM_{P,aa(P)} / US_{aa(P)}$$

**(2)**

Peptides that are likely CTSS substrates are expected to have very frequent amino acids in all P4-P4' positions (compared to the 3'804 known cleavage sites), which corresponds to large *j*(*Pep*) values. To find the most probable CTSS cleavage sites in a protein sequence, all possible octamers are virtually extracted and scored according to *f*. Top-ranked octamers are considered possible CTSS cleavage sites.

***Peptide Docking***

Peptides were docked on the CTSS surface using a docking software Attracting Cavities *(*Zoete et al., 2016, J. Comput. Chem., 37, 437-447*)* using default parameters, $N_{Thr}$=50 and a rotation step of 90° degrees. CTSS Cartesian coordinates were taken for the experimental 3D structure of human cathepsin, PDB ID 1npz (*Pauly et al., 2003, supra*). The search space consisted in a 23.3x28.7x22.6 $Å^3$ box, centered on the center of gravity of the C1P inhibitor present in the X-ray structure. The latter, and all water molecules, were removed prior to docking.

**Example 3: Cathepsin S over-expression in FL and DLBCL**

Since the Y132D mutation is detected in a fraction of FL patients, it has been investigated if oncogenic over-activation of CTSS could also be supported by gene over-expression. First, *CTSS* mRNA expression in FL patients was compared with and without the mutations as described below. In general, mRNA expression of *CTSS* was similar in the two groups, indicating that Y132D mutation affects protein activity but not mRNA expression of the gene. Interestingly, it was noticed that *CTSS* was significantly over-expressed in both FL and DLBCL tumors (n=38 FL and n=73 DLBCL GSE12195, n=191 FL (Dave et al., 2004, N. Engl. J. Med., 351, 2159-2169*)*), compared to normal B-cells (centroblasts and centrocytes) isolated from healthy donors. Moreover, when mRNA expression of cathepsin family members were compared in FL samples, *CTSS* was the most abundantly expressed among cathepsin genes, with the exception of *CTSH.* However, *CTSH* expression was similarly expressed in malignant and normal B-cells. In a panel of DLBCL lymphoma cell lines and in a transformed FL (DoHH2) cell line its was confirmed that CTSS but not CTSB protein was detected. To determine whether high mRNA expression of *CTSS* could be associated with difference in overall survival, patients with high or low CTSS expression (based on the median expression for each study) were compared. However, *CTSS* expression was not associated with significant overall survival difference in FL, suggesting that considering only *CTSS* expression is not a sufficient prognostic marker.

Next, to elucidate the heterogeneity of *CTSS* expression in normal and malignant B-cells, single cell transcriptomic data from purified B-cells obtained from one normal germinal center (GC) sample and one unpurified FL fresh biopsy wereanalyzed. To determine transcriptional differences between the normal and malignant conditions, all single cells were merged in a unique dataset, and analyzed their transcriptome through two-dimensional t-SNE projection. The cells obtained from the normal GC and FL biopsies grouped separately, and multiple cell clusters were found within each sample. To characterize these clusters, first, B and T cells were discriminated based on the expression of the B-cell receptor component *CD79A* and the T-cell marker *CD3.* Then, among B-cells, normal centroblasts and centrocytes were identified using previously designed gene expression signatures (Milpied et al., 2018, Nat. Immunol., 19, 1013*),* while tumor cells were recognized based on *BCL2* over-expression and high expression of *CD27, CD83,* and *CXCR4.* Based on the identification of these clusters, it was observed that *CTSS* and *CTSH* were expressed in tumor cells, centroblasts and centrocytes, while *CTSB* was expressed only in a limited number of malignant cells. Quantification of *CTSS* expression in individual cells showed that *CTSS* was significantly over-expressed in tumor cells compared to centroblasts and centrocytes. In addition to gene expression analyses, CTSS protein expression in tissue-microarrays of 192 FL samples was characterized (Kridel et al., 2015, Clin. Cancer Research 21 (15), 3428-35*).* To reliably identify tumors over-expressing CTSS protein, a low sensitivity staining was carried out to detect CTSS only when abundantly expressed and normal germinal centers were used as baseline (macrophages strongly positive, B cells negative). 40% of FL cases showed intermediate to high level of CTSS protein in 10-80% of the malignant cells. Overall, *CTSS* is over-activated by Y132D hotspot mutations and over-expressed in FL and DLBCL suggesting a specific and selective role of CTSS in lymphoma pathogenesis.

***RNAseq Follicular lymphoma datasets***

The RNASeq data were downloaded from GSE12195 and PRJNA278311 and processed as described in

Donaldson-Collier et al., 219, Nature Genetics. Log2 (TPM+1) values were used to analyze the expression of the different cathepsins in FL. Moreover, mRNA expression data reported in was collected from two separate studies: a collection of 191 FL samples (https://llmpp.nih.gov/FL/), and a collection of centroblast (n=5), centrocytes (n=5), DLBCL (n=73), and FL (n=38) samples (GSE12195). Both datasets were analyzed by Affymetrix microarray (HG-U133 Plus 2.0 array). To aggregate the two datasets, a batch correction analysis was performed using the removeBatchEffect function of the limma R package defining the two studies as separate batches and including a design matrix to specify the sample type.

***Analysis of single cell RNA sequencing data***

Bam files for Follicular Lymphoma (FL) and normal B-cells were downloaded from the Gene Expression Omnibus database under accession code GSE115795 (*Milpied et al.*, *2018, supra*). Raw FASTQ reads were extracted from the bam files using picard tools (v2.5.0) and aligned to GRCh37 using Cell Ranger software (v3.0.2) with default parameters. The resulting matrices were merged using the merge function from Seurat package in R *(*Butler et al., 2018, Nat. Biotechnol. 36, 411-420*;* Satija et al., 2015, Nat. Biotechnol., 33, 495-502*).* Cells with expression of fewer than 200 detected genes and genes with expression detected in fewer than three cells were removed. Seurat package was then used to identify the highly variable genes with the Find Variable Genes method, perform principal components analysis (PCA) dimension reduction on the log-normalized data matrix and the t-distributed stochastic neighbor embedding (t-SNE) analysis. t-SNE maps showing the expression of the log normalized gene expression were plotted using custom python scripts. To identify cells with centrocyte (CC) or centroblast (CB) phenotype, the R package GSVA *(*Hänzelmann et al., 2013, BMC Bioinformatics, 14, 7) was applied using the gene lists provided in the *Milpied et al.* to distinguish light and dark zone B-cells. Cells belonging to the normal B-cells clusters with a higher CC score compared to CB score were defined as CC and likewise cells with higher CB score were defined as CB.

**Example 4: Functional role of mutations and over-expression of Cathepsin S in follicular lymphomagenesis development**

To assess the functional role of CTSS Y132D mutation and over-expression in FL development, vavP-Bcl2 chimeric model of follicular lymphoma expressing either mutated CTSS ($CTSS^{Y132D}$) or over-expressing *CTSS* ($CTSS^{HIGH}$) was developed. This approach has been adopted in previous studies to determine the contribution of genomic lesions in follicular lymphomagenesis *(*Egle et al., 2004, Blood, 103, 2276-2283*;* Oricchio et al., 2011, J. Exp. Med., 211, 1379-1391*; Ortega-Molina et al*., *2015b, supra*). Interestingly, 93% of chimeric animals expressing $CTSS^{Y132D}$ (n=16) and 67% of $CTSS^{HIGH}$ (n=15) rapidly developed tumors (within 49 to 54 days), conversely, only 29% of the animals engrafted with control cells developed tumors ($CTSS^{Y132D}$ vs. vector Log-rank p-value <0.0001, and $CTSS^{HIGH}$ vs. vector Log-rank p-value =0.001). **(Fig. 6a)** Quantitative expression analysis confirmed specific over-expression of the transgenic human *CTSS* in B-cells isolated from these tumors. Histopathological analyses of $CTSS^{HIGH}$ and $CTSS^{Y132D}$ tumors showed characteristic features of follicular lymphoma, with expansion of the germinal centers, accumulation of Bcl6 positive cells, and increased proliferation (Ki-67). Moreover, tumors isolated from $CTSS^{HIGH}$ and $CTSS^{Y132D}$ animals were highly enriched in B220 positive cells and in particular accumulation of germinal center B-cells (B220+, GL7+ and FAS+) was observed. Interestingly, the increase number of germinal center B-cells was associated with a significant increase of CD4+ T-follicular helper (Tfh) cells (CD4+, PD1+, CXCR5+). This increase in CD4+ Tfh was observed in spite of comparable levels of MHC class II expression and total number of infiltrated CD3+, CD4+ among the different groups. To further elucidate the effect of *CTSS* over-expression and mutation, the composition of the germinal centers was analysed in details by multicolor immunostaining and quantify the signal intensity related to CD8+ T-cells, CD4+ T-cells and B-cells in the whole spleen. It was observed that in animals over-expressing *CTSS* or the mutated protein, CD8+ T-cells were excluded from and surrounded the expanded germinal centers, while CD4+ T-cells were highly infiltrated and co-localized with B-cells (blue). This trend was confirmed by Immune histo-chemistry analyses in a panel of human FL samples. Briefly, the patients were stratified based on CTSS, PD1, CD4 and CD8 status. Patients with high expression of CTSS tend to be $CD4^{High}/PD1^{High}/CD8^{Low}$, whereas patients with low expression of CTSS were mostly $CD4^{Low}/PD1^{High}/CD8^{High}$. These results suggested that tumors with high-expression of *CTSS* or expressing mutated *CTSS* increase the recruitment of CD4+ T-cells while limiting CD8+ T-cells infiltration. Overall, over-activation of cathepsin S mediated by Y132D mutation or gene over-expression contributed to accelerate tumor development in chimeric models of FL and modified the organization of the lymphoma microenvironment.

**Example 5: Role of Cathepsin S activity for follicular lymphoma initiation**

In normal B-cells, CTSS regulates MHC class-II maturation and the communication between CD4+ T-cells *(Shi et al., 1999, supra*). Thus, the dependency of oncogenic activity of CTSS on its ability to promote communication between FL cells and CD4+ T follicular helper (Tfh) cells was investigated. CD4+ T-cells were depleted in vavP-Bcl2 chimeric

animals expressing *CTSS*$^{HIGH}$ and *CTSS*$^{Y132D}$ by treating the animals biweekly for one or two months with an anti-CD4 antibody. Notably, depletion of CD4+ T-cells abolished the ability of vavP-Bcl2-*CTSS*$^{HIGH}$ and vavP-Bcl2-*CTSS*$^{Y132D}$ to develop tumors, due to lack of GC B-cells differentiation and specific loss of expansion and proliferation of B-cells in the germinal centers.

To decipher the contribution of Ctss activity to follicular lymphoma initiation, an independent vavP-Bcl2-Ctss-KO dual transgenic animal model was used and the GC composition and spleen enlargement in 20-25 weeks old vavP-Bcl2 and vavP-Bcl2-Ctss-KO transgenic animals were compared. As expected, splenomegaly in vavP-Bcl2 was observed compared to wild-type animals, while the size of the spleen was significantly smaller in vavP-Bcl2-Ctss-KO animals. Moreover, despite similar level of MHC class II expression in vavP-Bcl2 and vavP-Bcl2-Ctss-KO animals, the total number of B220+ and CD4+ T-cells was significantly reduced. In particular, a decreased number of GC B-cells and CD4+ Tfh cells was observed associated with a significant gain of CD8+ T-cells and macrophages infiltrated in the spleen. Loss of GC maturation in vavP-Bcl2-Ctss-KO animals was confirmed by lack of Bcl6 positive cells and loss of proliferating cells in the GC. Importantly, loss of Ctss activity was associated with an increased CD8+ T-cells infiltrated in the germinal center, resulting in a negative ratio between CD4:CD8 cells. Since cathepsin S seemed to regulate the communication with CD4+ Tfh cells, it was checked whether in FL patient mutations of *CTSS* were mutually exclusive with *TNFRSF14* and *RRAGC* mutations, which have been previously associated with interactions between B-cells and CD4+ T-cells *(Boice et al*., *2016, supra; Ortega-Molina et al*., *2019, supra*).

To obtained a representative number of patients harboring CTSS mutations, whole genome sequencing data from our 75 FL patients were combined and targeted sequencing of 248 FL from *Kridel et al*., *2016, supra* and analyzed the pattern of occurrence of *CTSS*$^{Y132D}$*, TNFRSF14* and *RRAGC* mutations using the Mutually Exclusive Modules (MEMo) algorithm (Ciriello et al., 2012, Genome Res., 22, 398-406). Interestingly, *CTSS*$^{Y132D}$ mutations were found to exhibit a significant trend for mutual exclusivity with *TNFRSF14* and *RRAGC* mutations (MEMo p-value= 0.045), suggesting that lymphoma cells can alternatively mutate one of these genes to promote pro-tumorigenic communications with CD4+ Tfh cells. Overall, by using independent chimeric and transgenic animal models, it was observed that the activity of CTSS is essential to support the communication between B-cells and CD4+ Tfh cells and this interaction is crucial to promote follicular lymphoma development. Moreover, loss of Ctss activity increased the infiltration of CD8+ T-cells suggesting that this could contribute to control tumor growth.

***Cathepsin S KO restrains aggressive lymphoma growth by recruiting CD8+ T-cell***

In indolent follicular lymphoma, cancer cells require the support of CD4+ Tfh and others component of the tumor microenvironment to proliferate *(Scott and Gascoyne, 2014, supra*). However, these interactions are dispensable in more aggressive lymphoma, where activation of intracellular signals sustains cell proliferation. Thus, it was investigated whether aggressive lymphoma still rely on CTSS activity. *Ctss* was knocked out by CRISPR/Cas9 in A20 mouse B-cell lymphoma. As previously reported (*Riese et al*., *1996, supra*), loss of *Ctss* induced accumulation of a small fragment of CD74 (~10 kDa, p10) in multiple knock-out clones. Interestingly, while *Ctss* knock-out did not influence lymphoma cell proliferation *in vitro,* a significant reduction of tumor growth was observed independent clones of *Ctss* null cells (Ctss KO-4) were engrafted in syngeneic immunocompetent recipient animals **(Fig. 6b).** Autopsy and histological analyses confirmed that *Ctss* knock-out tumors tend to be smaller and less proliferative than wild-type tumors. In addition, A20 *Ctss* knock-out cells showed reduced ability to infiltrate and form liver metastases both at 19 and 21 days post-engraftment. Hence, *Ctss* knock-out reduces tumor growth *in vivo* but not *in vitro,* suggesting that its oncogenic effect still relies on the interactions between cancer cells and the tumor microenvironment. **Generation of syngeneic cell lines by CRISPR/Cas9.** CTSS, Pdl1 and β2m KO cell lines were obtained using the plasmids pSPCas9(BB)-2A-GFP (PX458) (Addgene, cat#48138) or pL-CRISPR.EFS.GFP (Addgene, cat #57818) expressing GFP, Cas9 and GFP and a gene-specific gRNA, which was cloned using the BbsI (NEB, cat #R0539) or BsmBI (NEB, cat #R0580) restriction sites. A20, A20-HA or DOHH2 cells were electroporated with the CRISPR plasmids using the NEPA21 electroporator (Nepagene) and single-cell cloning of GFP+ cells was performed by FACS 48 hours after electroporation. 10-20 days after sorting, clones negative for Pdl1 or B2m were screened by flow cytometry using an anti-Cd274 antibody PE (clone MIH5, BD bioscience, cat #558091) and anti-MHC-1 H-2Kd antibody PerCP eFluor 710 (clone SF1-1.1.1, Thermo Fisher scientific, cat #46-5957-82) respectively.

Clones negative for human or mouse Cathepsin S were screened by Western blot. The primers used for gRNA cloning were the following primers:

mCtss-gRNA-For: GGGAATGCATACCTACCAAG **(SEQ ID NO: 8);** mCtss-gRNA-Rev: CTTGGTAGGTATGCATTCCC **(SEQ ID NO: 9);** mPdl1-gRNA-For: CGAGGGTTATCCAGAAGCTG **(SEQ ID NO: 10);** mPdl1-gRNA-Rev: CAGCTTCTGGATAACCCTCG **(SEQ ID NO: 11);** mβ2m-gRNA-For: AGTATACTCACGCCACCCAC **(SEQ ID NO: 12);** mβ2m-gRNA-For: GTGGGTGGCGTGAGTATACT **(SEQ ID NO: 13);** hCTSS-gRNA-For: CACCGTTGCATAAAGATCCTACCC **(SEQ ID NO: 14);** and hCTSS- gRNA-Rev:AAACGGGTAGGATCTTTATGCAAC **(SEQ ID NO: 15).**

To identify differences in the microenvironment of *Ctss* wild-type and knock-out tumors, the abundance of

infiltrated T-cell subpopulations, macrophages, and neutrophils was quantified. Then, the percentage of each population was correlated with tumor weight. Notably, tumor weight was significantly anti-correlated with the number of CD8+PD1+ and CD4+CD25+ T-cells, and positively correlated with the number of neutrophils (Ly6C+ Ly6G+). In particular, accumulation of CD8+PD1+ was pronounced in small *Ctss* knock-out tumors. In addition, metastatic *Ctss* knock-out tumors in the liver showed increased CD8+ T-cell infiltration while the number of CD4+ T-cells remained constant. To directly demonstrate that CD8+ T cells but not CD4+ T-cells activity contributes to reduce growth in *Ctss* knock-out tumors of aggressive model of lymphoma, specific depletion of either CD4+ T-cells or CD8+ T-cells was carried out treating animals with an anti-CD4 and anti-CD8 antibodies, respectively. Depletion of CD4+ T-cell activity did not affect tumor growth in this model, indeed animal engrafted with *Ctss* KO cells develop smaller tumor than the wild-type in both treated and untreated group . Conversely, *Ctss* knock-out tumors (Ctss KO-4 and Ctss KO-27) grew faster in animals treated with anti-CD8 than in untreated animals and in particular Ctss KO-27 tumors proliferated similarly to the *Ctss* wild-type and the difference in tumor weight was abolished. All together, these results indicate that loss of Ctss and reduction of tumor growth in aggressive tumor can be mediated by activation of cytotoxic lymphocytes. Thus, cathepsin S may still play an important role in balancing interactions between malignant B and T-cells in the tumor microenvironment also in aggressive tumors that are independent from CD4+ Tfh cell interactions.

***Cathepsin S is essential to induce antigen specific CD4+ T-cell activation***

To functionally establish the role of *Ctss* in B and T cell interactions, co-culture assays were used: malignant B-cells were co-cultured with either CD4+ or CD8+ T-cells and T-cell proliferation and lymphoma B-cell survival were measured. First, lymphoma cells isolated from vavP-Bcl2 and vavP-Bcl2-Ctss-KO transgenic animals were compared for their ability to stimulate CD4+ T-cell proliferation. Briefly, isolated pan-B-cells were isolated from the spleen and co-incubated them with OVA and CD4+ T-cells isolated from OT-II transgenic animals for 96 hours. In agreement with observations *in vivo,* loss of *Ctss* in lymphoma cells impaired B-cell mediated CD4+ T-cell expansion and slightly reduced the number of B-cells. The same results were obtained using A20 cells wild-type and *Ctss* knock-out co-cultured with CD4+ T-cells isolated from D.11.10 transgenic animals although *in vivo* the proliferation and survival of these cells did not depend on the interaction with CD4+ T-cells. Overall, these results indicate that loss of *Ctss* alters the cross-talk between malignant B-cells and CD4+ T-cells, but since MHC-class-II expression on the cell surface of *Ctss* wild-type and knock-out cells is similar, this effect might be mediated by difference in antigen processing and loading.

***Loss of cathepsin S enhances CD8+ T-cell activation***

The analysis of the tumor microenvironment suggested that an increased infiltration of CD8+ T-cells contributes to reduce the growth of Ctss knock-out tumors. In particular, in the A20 model, reduction of tumor growth in animals engrafted with *Ctss* knock-out cells is associated with CD8+ T-cell but not with CD4+ T-cell activity. Thus, A20 model was used to assess whether loss of *Ctss* could directly influence CD8+ T-cell activation. To mimic trafficking and intracellular processing of endogenous peptides, A20 were engineered cells to express the HA model antigen (HA-IRES-GFP) which can be used in co-culture assays with CD8+ T-cells isolated from CL4-HA transgenic animals *(*Morgan et al., 1996, J. Immunol., 157, 978-983). *Ctss* knocked-out in A20-HA positive cells, and generated A20-HA beta-2 microglobulin *(B2m)* or *CD274* (*Pdl1*) knock-out cells that in the co-culture assays with CD8+ T-cells can be used as negative and positive controls, respectively. In multiple A20-HA *Ctss* knock-out clones, the accumulation of the CD74 fragment p10 was confirmed. As expected, lymphoma cell proliferation *in vitro* was not affected by loss of *Ctss* or *B2m* or *Pdl1* expression. Moreover, GFP expression was used as read-out of HA expression and verified that GFP-HA levels were similar in all clones, but one. Strikingly, co-incubation of A20-HA *Ctss* knock-out clones with CD8+ T-cells for 72h promoted rapid proliferation of CD8+ T cells, similar to *Pdl1* loss, and enhanced CD8+ T cell-mediated B-cell killing Hence, loss of *Ctss* in lymphoma on one side influences interactions with CD4+ T-cells, while on the other side promotes CD8+ T-cell activity, suggesting that cathepsin S may be a key regulator of antigen processing.

***Absence of cathepsin S activity changes the antigen repertoire of lymphoma cells***

Since alterations of both CD4+ and CD8+ T-cell activity were observed *in vivo* and *in vitro,* the impact of CTSS activity on both MHC-class-II and MHC-class-I antigen maturation and presentation processes was investigated. To directly analyze the repertoire of antigens bound to MHC-class-I and MHC-class-II molecules, immuno-peptidome analyses were performed in CTSS wild-type, knock-out and CTSS Y132D human transformed FL cells (DoHH2). As observed in murine cells, loss of CTSS activity resulted in accumulation of the CD74 peptide p10 also in human cells, indicating that CTSS has a similar proteolytic function on human and mouse proteins. Re-expression of the wild-type or mutated CTSS in the knock-out cells efficiently rescued CD74 processing. Unbiased analyses of MHC-class-II peptides revealed qualitative differences in the antigen repertoire of DoHH2 *CTSS* wild-type and knock-out cells. The peptides bound to

MHC-class-II molecules in *CTSS* wild-type and knock-out cells were systematically analyzed in 6 replicates for each condition, and for further analyses, only considered peptides that were detected in at least 5 out of 6 replicates in each condition were considered. 2’573 peptides were recovered in both *CTSS* wild-type and knock-out cells (shared peptides), while 1654 peptides were uniquely detected in *CTSS* wild-type (WT-unique peptides) and 2635 peptides were identified only in CTSS knock-out cells (KO-unique peptides). This difference was abrogated *CTSS* wild-type cells were compared versus the same knock-out clone that re-expressed the mutated form of *CTSS.* Indeed, between CTSS wild-type and mutated cells only a handful number of peptides were different between the two conditions (1’972 shared peptides, 182 unique peptides in CTSS-WT, and 226 unique peptides in CTSS-Y132D . The similarities in the antigen repertoire in CTSS WT versus CTSS KO and CTSS WT versus CTSS Y132D were quantified. It was confirmed that peptides bound to MHC class II by DOHH2 CTSS WT and KO cells have a low similarity score (median Jaccard similarity index = 0.35), while the similarity is higher between CTSS WT and Y132D cells (median Jaccard similarity index = 0.68). These results demonstrate that loss of CTSS activity altered the antigen repertoire of lymphoma cells, and re-expression of the mutated form of CTSS can rescue this phenotype.

***Cathepsin S regulates antigen processing in lymphoma cells***

To assess how loss of CTSS activity modified the antigenic repertoire of lymphoma cells, peptide sequences were scanned to identify potential CTSS cleavage sites ("STAR" Methods). Consistent with lack of CTSS proteolytic activity, peptides exclusively detected in *CTSS* knock-out cells (KO-unique) were significantly enriched for CTSS cleavage sites compared to peptides uniquely detected in *CTSS* wild-type cells (WT-unique) or in peptides of the same length that were randomly selected from the same set of proteins. Then, by sequence alignment, KO-unique and WT-unique peptides with highly overlapping sequences (i.e. that mapped to the same protein region) were identified. Interestingly, for 47.5% of KO-unique peptides containing a CTSS cleavage site, whereas the corresponding overlapping WT-unique peptides exhibited a worse conservation (or complete loss) of the CTSS cleavage site. These results indicate that KO-unique peptides could originate from uncleaved targets of CTSS. For example, peptides mapping to HSPA1/HSPA8 proteins were cleaved by CTSS WT and CTSS Y132D but not in the knock-out cells. Indeed, KO-unique peptides were significantly longer than shared ($p=1.8 \times 10^{-22}$) or WT-unique peptides ($p= 9x10^{-6}$). Interestingly, among KO-unique peptides, the ones containing a CTSS cleavage site were longer than peptides that did not contain a cleavage site (p=0.0003). Importantly, re-expression of CTSS Y132D in CTSS knock-out cells abrogated these differences, indeed the length of the unique and shared peptides in CTSS WT and CTSS Y132D cells is similar among conditions. Independent analyses of the MHC-class II antigenic repertoire in murine A20 cells confirmed diversification of the antigen repertoire between A20 Ctss WT and Ctss knock-out cells (median Jaccard similarity index = 0.54) and the presence of long peptides in *Ctss* knock-out cells. further supporting a role of CTSS in cleaving the antigens presented by MHC class II molecules. Overall, results in independent cell models indicate that CTSS knock-out impair cleavage of CTSS-target peptides, which results in the generation of long (uncleaved) peptides uniquely present in CTSS knock-out cells.

To directly test the ability of CTSS WT and Y132D to cleave the antigenic peptides identified by the immuno-peptidome analyses in the predicted cleavage site, peptides derived from CD74 **SEQ ID NO: 16** (QLENLRMKLPKPPKPVSKMR) and HSPA1/8 proteins **(SEQ ID NO: 17** (EEIERMVQEAEKYKAEDEVQ) were used. First, it was confirmed that both wild-type and mutated CTSS proteins are able to cleave these peptides. Indeed, the abundance of the CD74 and HSPA1/8 full-length peptides decreased after 15 minutes of incubation with purified CTSS-His-WT and CTSS-His-Y132D proteins. For the same time of reaction, CD74 peptides were completely cleaved while only 35-40% of HSPA1/8 peptides were cleaved, indicating preferential activity of CTSS for specific cleavage sites Importantly, proteolysis of the full-length CD74 and HSPA1/8 peptides was associated with the generation of fragments that originated from cleavage at the predicted proteolytic sites. Overall, these results suggested that CTSS activity is required for processing a group of antigens and loss of its activity diversifies the sets of antigens that are associated with MHC molecules.

Next, because of the existing cross-talk between MHC-class-II and MHC-class-I antigen presentation processes, loss of CTSS activity could also influence MHC-class I antigen repertoire. Immuno-peptidome analyses for MHC-class-I peptides revealed that the number of unique peptides is higher in *CTSS* knock-out cells (n=301 peptides detected in at least 5 out 6 replicates) than in *CTSS* wild-type cells (n=64 peptides detected in at least 5 out 6 replicates, p=0.05) and these peptides mapped to different proteins, which may also contribute to increase the heterogeneity of antigens detected in *CTSS* knock-out cells. On the cell surface, MHC-class-I and MHC-class II expression levels did not change in *CTSS* wild-type and knock-out cells and neither did the usage of MHC haplotypes, although it could not be discriminated whether MHC-class-II molecules on the cell surface were mainly associated with unprocessed CD74 or with different antigens.

Finally, to directly measure the effect of CTSS on antigens and T-cell activation, the same algorithmic approach was used that was applied to identify CD74 peptides targeted by CTSS to analyze the sequence of OVA and HA proteins since these are two proteins commonly used in antigen presentation assays. Thus, the top scoring peptides for OVA **(SEQ ID NO: 18** (IKVYLPRMKMEEKYNLTSVL)) and HA **(SEQ ID NO: 19** (NVKNLYEKVKSQLKNNAKEI)) were incubated with the purified CTSS-His-WT protein for 30 minutes and it was observed that CTSS can cleave both OVA and HA peptides (preferentially OVA then HA peptides) and generate fragments from cleaving the peptides at the predicted site. Based on these results, it was investigated whether in Ctss knock-out cells impaired cleavage of OVA protein led to lack of CD4+ activation that was observed. Hence, CD4+ T-cell activation in co-culture assay was compared with A20 CTSS wild-type and knock-out cells using either the full OVA protein or already cleaved OVA peptides. Striking, A20 *Ctss* knock-out could not stimulate the proliferation of CD4+ T-cells if incubated with full OVA protein whereas already cleaved peptides were presented on the cell surface and induced activation of CD4+ T-cells. Overall, these results support a role of CTSS in antigen processing of both endogenous and exogenous proteins. Hence, loss of CTSS activity in lymphoma cells alters antigen maturation that can contribute to reduce MHC-class-II mediated communication with CD4+ T-cells while enhancing tumor immunogenicity mediated by MHC-class-I and this could explain the anti-tumor immune response observed *in vivo.*

***Cathepsin S expression correlates with level of T-cell infiltration in FL patients.***

Lastly, it was investigated whether the increased infiltration and activity of CD8+ T-cells in *Ctss* null tumors could improve anti-tumor activity of immuno-checkpoint inhibitor such as anti-PD1. A20 wild-type or *Ctss* knock-out cells were engrafted in Balb/c mice and 7 days post-engraftment the animals were with 10 mg/kg of an anti-PDL-1 (clone 10F.9G2, BioXcell, cat # BP0101) twice a week. Upon following tumor growth for over 20 days, loss of *Ctss* expression or treatment with PD1 limited tumor growth was observed **(Fig. 6b and 6c).** Loss of Ctss did not influence the expression of PDL-1 on the cell surface, but treatment with anti-PD1 further reduced tumor growth using two independent *Ctss* knock-out clones. Next, to assess whether *CTSS* expression could be associated with response to anti-PD1 inhibitor, expression data of FL patients treated with an anti-PD1 in a phase II clinical trial (NTC00904722)*(*Westin et al., 2014, Lancet Oncol., 15, 69-77) were analyzed. In line with results in murine models *CTSS* expression significantly anti-correlated with *PD1* expression (gene name *PDCD1)* in this cohort (n=26 p=0.015). Indeed, the expression of *PD1* is strongly reduced in FL tumors with high expression of *CTSS* (p=0.016), indicative of reduced T-cell infiltration in these tumors. A significant anti-correlation between *CTSS* and *PD1* expression in an independent FL dataset was confirmed (n=137 p= 0.0079, GSE119214) (*Silva et al*., *2019, Haematologica, 104 (6), e252-e255 Jun 2019).* To specifically associate *CTSS* expression with response to anti-PD1, matching expression data before and after 14 days of treatment with anti-PD1, which were available for 8 patients was explored. Although independent data of complete or partial response were not available for this clinical trial (NTC00904722), the expression of PD1 was used as indicator of response to therapy, since treatment with anti-PD1 induces activation and recruitment in the tumor of CD8+ PD1+ cells (Tumeh et al., 2014, Nature, 515, 568-571). Treatment with anti-PD1 increased the levels of PD1 expression in tumors with low *CTSS* expression, while *PD1* expression was reduced in 2 out of 3 patients with high expression of *CTSS.* Despite the limited number of patients included in this study, these observations suggested that CTSS expression levels could correlate with anti-PD1 treatment response and blocking CTSS activity could improve response to anti-PD1 therapy.

**Cell lines**

Mouse Diffuse Large B Cell Lymphoma cell line A20 (ATCC, cat #ATCC-TIB208™, BALB/c background) and genetic variants were cultured in RPMI-1640 medium (Thermo-Fisher, cat #1870010) supplemented with 10% heat-inactivated fetal bovine serum (FBS) (Thermo-Fisher, cat #10270-106), 2 mM 1-glutamine (Thermo-Fisher, cat #M11-006), 1 mM sodium pyruvate (Sigma-Aldrich, cat #S8636), 10 mM HEPES (Sigma-Aldrich, cat #H3537), 5 mM d-glucose (Sigma-Aldrich, cat #G8769), 25ug/mL Gentamicin (Thermo-Fisher, cat #G1397) and 50 $\mu$M 2-mercaptoethanol (Thermo-Fisher, cat #31350-010,). A20-HA-GFP cell line expressing hemagglutinin (HA) gene from influenza virus strain A/Puerto Rico/8/1934 H1N1 (UniProtKB-P03452) was kindly provided by Novimmune SA. Human DOHH2 cells were cultured in RPMI 1640 GlutaMAX™ media (Thermo Fisher scientific, cat #1870010) supplemented with 10% FBS (Thermo-Fisher, cat#10270-106) and 1% penicillin/streptomycin (Thermo-Fisher, cat #15140122). All cells lines were maintained at 37 °C in a water-jacketed incubator containing 5% CO2, and authenticated within the past year by STR DNA profiling.

**Mouse models**

*Mice.* Six- to eight-week-old female BALB/c mice and C57BL/6J mice were purchased from Charles River Laboratories. OT-I, OT-II, were purchased from the Jackson laboratory and CL4 mice can be obtained from Jackson laboratory, vavP-Bcl2 (Egle et al., 2004, Blood, 103 (6): 2276-2283 and Ctss KO (Ctsstm1Hap, *Ctss* -/-) animals (Akkari et al. 2016, enes Dev, 30 (2), 220-32*).* All animal experiments were performed in accordance with the Swiss Federal Veterinary Office guidelines and as authorized by the Cantonal Veterinary Office (animal license VD2932 and VD2932.1).

*HSC model*

vavPBcl2 transgenic fetal liver cells were isolated from embryos at day 13.5, and kept in growth media DMEM (Gibco/Thermo Fisher scientific, cat #41966-029), IMDM (Thermo-Fisher, cat #31980-022), L-glutamine (Thermo Fisher Scientific, cat #35050061), pen/strep (Thermo-Fisher, cat 15140122), FBS (Thermo-Fisher, cat #10270-106), Wehi conditioned medium, IL3 (Seralab, cat #GEM-300-324P100), IL6 (Seralab, cat # EM-300-327P100), SCF (Seralab, cat# GEM-300-348P100), and polybrene (Merck Milipore, cat #TR-1003-G) for 4 days. During this time, HPCs were retrovirally transduced with MLS-GFP vectors empty or containing human CTSS WT or Y132D. The transduced HPCs were then inoculated in sublethally irradiated (mice irradiated with 3.6 Gy on two consecutive days) syngeneic wild-type C57BL/6 animals. Disease onset and progression were measured by blood smear and palpation, and data were pooled in Kaplan-Meier curves and analyzed using the log-rank (Mantel-Cox) test for statistical significance.

*Subcutaneous A20 model*

1 × 106 A20 or A20-Ctss-KO were injected subcutaneously into the left flank of 8-10-week-old BALB/c mice (Charles River Laboratories). Tumors were measured every 2-3 days using a caliper and volumes were calculated using the formula (width × length × height × Pi)/6. Tumor weight was measured at endpoint. Animals were euthanized when the tumor volume exceeded 1000 mm3 or at the experimental endpoint.

*Disseminated A20 model*

0.5 × 106 A20 or A20-Ctss-KO were injected intravenously in the tail vein of 8-10-week-old BALB/c mice. Animals were checked every 2-3 days and euthanized when mice developed sign of paralysis, or at the experimental endpoint.

*CD8+ T cell depletion*

To deplete CD8+ T cells, six- to eight-week-old female BALB/c mice were intravenously injected with 200ug anti-CD8β antibody (clone 53-5.8 , BioXCell, cat# BE0223) 5 days and 2 days before tumor cells engraftment and then injected intraperitoneally once a week after engraftment. Circulating CD8+ T cell depletion was confirmed one day before engraftment by flow cytometry. Briefly, blood was incubated in ACK buffer for 4 minutes at room temperature to lyse RBC. Then, cells were incubated with 50 μl Fc block for 15 minutes on ice and subsequently supplemented with 50 μl of an antibody mixture composed of APC-Cy7 rat anti-mouse CD45, BV786 hamster anti-mouse CD3 (Clone 145-2C11, BD Bioscience, cat #564379), PerCP efluor710 rat anti-mouse CD4 (clone RM4-5, bd bioscience, cat #46-0042-82) and FITC anti mouse-CD8a (clone 53-6.7, biolegend, cat# 100705) for 30 min at 4 °C in FACS buffer. Cells were washed twice to remove unbound antibody and analyzed using FORTESA flow-cytometer. Tumor volume were measured biweekly as described in the previous section.

*Organs processing*

For A20 subcutaneous tumors, tumor tissues were excised on day 16 post engraftment. Tumor were cut in 2-3 mm pieces and digested in gentle MACS C Tubes (miltenyi biotech, cat #130-096-334) using mouse tumor dissociation kit (Miltenyi biotech, cat #130-096-730) and gentleMACS Dissociator (miltenyi biotech, cat # 130-096-427) following manufacturer's instructions. For A20 disseminated model, mouse livers were excised on day 19 or 21 post engraftment and digested with mouse liver dissociation kit (miltenyi biotech, cat # 130-105-807). For HSC model, spleens were smashed and resuspended in PBS to generate a single cell suspension.

Digested organs were then washed with PBS and resuspended in Ammonium-Chloride-Potassium buffer (0.15M $NH_4Cl$, 1M $KHCO_3$, 0.1mM $Na_2$ EDTA, pH 7.2-7.4) for 4 minutes at room temperature to lyse RBC. Cells were then washed once with PBS and passed through a 70-μm cell strainer (Falcon, cat #352350) to remove large pieces of undigested tumor. Resulting single cell suspension was washed twice with PBS and cell concentration and viability was quantified using Guava.

**Flow cytometry**

For flow cytometry analysis of samples harvested from *in vivo* experiments, 2x106 total cells were incubated with 50 μL Fc Block (BD Biosciences, cat #553142) for 15 min on ice. Samples were subsequently supplemented with 50 μL of: (Antibody mixture 1) composed of APC-Cy7 rat anti-mouse CD45 (clone 30F11, BD bioscience, cat #557659), PE-CF594 rat-anti mouse B220 (Clone RA3-6B2, BD bioscience, cat #562313), BV786 hamster anti-mouse CD3 (Clone

145-2C11, BD Bioscience, cat #564379), PerCP efluor710 rat anti-mouse CD4 (clone RM4-5, bd bioscience, cat #46-0042-82), PE-Cy7 rat anti-mouse CD8a (clone 53-6.7, bd bioscience, cat #552877), APC rat anti-mouse PD-1 (clone 29F.1A12, ebioscience, cat #135210), for T-cell characterization, (Antibody mixture 2) composed of PerCP efluor710 rat anti-mouse CD4 (clone RM4-5, bd bioscience, cat #46-0042-82), APC rat anti-mouse PD-1 (clone 29F.1A12, ebioscience, cat #135210), BV421 rat anti-mouse CXCR5 (clone L138D7, biolegend, clone #145511) for follicular helper Tcells identification, (Antibody mixture 3) composed of APC-Cy7 rat anti-mouse CD45 (clone 30F11, BD bioscience, cat #557659), PE-CF594 rat-anti mouse B220 (Clone RA3-6B2, BD bioscience, cat #562313), BUV-737 rat anti-mouse CDllb (Clone M1/70, BD- bioscience, cat #564443), BV605 rat anti-mouse Ly-6C (Clone HK1.4, BD bioscience, cat #563011), BV421 rat anti-mouse Ly6G (clone 1A8, BD bioscience, cat #562737), BV710 rat anti-mouse F4/80 (Clone T45-2342, bd bioscience, cat #565612) for myeloid cells characterization, (Antibody Mixture 4) PE-CF594 rat-anti mouse B220 (Clone RA3-6B2, BD bioscience, cat #562313), APC rat anti-mouse GL7 antigen (clone GL7, Biolegend, cat #144617), PE mouse anti-mouse CD95 (Fas) (clone SA367H8, Biolegend, cat #152607) for Bcell characterization. Sample were then washed twice, resuspended in 200 μL of FACS buffer containing DAPI viability marker (Molecular probes, cat #D1306), and analyzed using LSR Foretesa flow cytometer (BD Biosciences). For flow cytometry analysis of MHCII and MHC-I expression, studied WT and CTSS-KO Bcell lines were washed with a FACS buffer (PBS, Gibco/Thermofisher scientific, cat #10010-015), 2% BSA (Sigma Aldrich, cat #A7906), incubated with APC rat anti-mouse IA/IE (clone M5/114.15.2, bd bioscience, cat #107613), mouse anti-human HLA-DR (clone G46-6, BD biosciences, cat #555812), or APC anti-human HLA-A,B,C (Biolegend, cat #BLG-311410) for 30 min at 4°C in FACS buffer. Cells were washed twice to remove unbound antibody and analyzed using Cytoflex flow-cytometer (Beckman Coulter, Indianapolis, IN, USA). For blood analysis, 20 μL of heparinized blood (Microvette, VWR, cat# 102971-718) was incubated for 4 minutes with red blood cell lysis solution (Miltenyi biotech, cat #130-094-183) for 4 minutes and subsequently stained with APC-Cy7 rat anti-mouse CD45 (clone 30F11, BD bioscience, cat #557659), BV786 hamster anti-mouse CD3 (Clone 145-2C11, BD Bioscience, cat #564379), PerCP efluor710 rat anti-mouse CD4 (clone RM4-5, bd bioscience, cat #46-0042-82), PE-Cy7 rat anti-mouse CD8a (clone 53-6.7, bd bioscience, cat #552877) for 30 min at 4 °C in FACS buffer. Cells were then washed twice to remove unbound antibody and analyzed using LSR Foretesa flow cytometer (BD Biosciences).

**Quantitative RT-PCR**

Total RNA was extracted from vav-P-Bcl2 tumors expressing vector or *CTSS*$^{HIGH}$ or CTSS$^{Y132D}$ with Trizol Reagent and purified using RNeasy Mini Kit(Qiagen, cat #74104). Reverse transcription was performed with the SuperScript™ III First-Strand Synthesis System (Invitrogen, cat #18080051), using oligo(dT) primers. cDNA quantification was carried out with SYBR Green (Invitrogen, cat # S7585), using human CTSS primers: (hCTSS-For: GGATCACCACTGGCATCTCT **(SEQ ID NO: 20),** hCTSS-Rev: ATTCCCATTGAATGCTCCAG) **(SEQ ID NO: 21)** and mouse HPRT1 primers (mHPRT1-For: CAAACTTTGCTTTCCCTGGT **(SEQ ID NO: 22),** mHPRT1-Rev: CAAGGGCATATCCAACAACA) **(SEQ ID NO: 23).** Data were analyzed using the ΔΔCt method.

**Western blot analyses**

Proteins were extracted using a buffer containing 20 mM Tris-HCl (pH 7.5) 150 mM NaCl, 1 mM Na2EDTA 1 mM EGTA 1% NP-40 1% sodium deoxycholate 2.5 mM sodium pyrophosphate 1 mM β-glycerophosphate 1 mM Na3VO4 1 μg/ml leupeptin) supplemented with protease (Sigma Aldrich, cat #11836153001) and phosphatases (Roche, cat #04906837001) inhibitors and quantified with the Bradford reagent (Bio-Rad, cat #5000006). 20-30 μg of proteins were resolved on SDS-PAGE and transferred at 20V for 20-30 minutes to Immobilon-P-membranes (Millipore). After blocking in 5% milk (Applichem, cat #A0830) in PBS-0.1%Tween (Fisher scientific, cat #BP337), membranes were incubated overnight in 5% milk in PBS-Tween with the following primary antibodies: goat anti-human Cathepsin S (RD, cat #AF1183, 1:2000), Cathepsin B (D1C7Y) XP® Rabbit (Cell signaling mAb #31718), rabbit anti-mouse Cathepsin S (Sino Biological, cat #50769-R054, 1:1000), mouse anti-human CD74 (PIN.1, StressMarq, cat #SMC-116, diluted 1:1000), rat-anti-mouse CD74 (clone In-1, BD biosciences, cat #555317, 1:1000), rabbit anti-his-Tag (Cell Signaling, cat #D3I10, dilute 1:1000), mouse anti-human alpha-Tubulin (clone B-5-1-2, Sigma Aldrich, cat#T6072, diluted 1:10000), anti-beta actin (clone 8H10D10, ThermoFisher Scientific, cat# MA5-15452, 1:1000).

Membranes were then washed for 30 minutes and incubated with the fluorescent secondary antibodies, diluted 1:10000: IRDye 800CW goat anti-Rabbit IgG (LICOR, cat #926-32211), IRDye 680RD goat anti-Mouse IgG (LICOR, cat #926-68070), or with HRP-conjugated antibodies, diluted 1:2500: goat anti-rabbit antibody (H+L) (Merck Millipore, cat #AP307P), goat anti-mouse antibody (H+L) (Merck Millipore, cat#AP308P), rabbit anti-goat IgG (H+L) (ThermoFisher scientific, cat #31402). Image acquisition was performed with LICOR for fluorescent antibodies or WesternBright Sirius (Advansta) for chemiluminescent antibodies.

**Immunohistochemistry**

Analysis of Ki67 (rabbit α-Ki67, clone SP6, Springbio, diluted 1:100, cat #ab16667) and Bcl6 (rabbit α-mouse Bcl6, clone EPR11410-43, Abcam, diluted 1:250, cat #ab172610) was performed using the fully automated Ventana Discovery ULTRA (Roche Diagnostics, Rotkreuz, Switzerland). All steps were performed on the automate with Ventana solutions. Briefly, dewaxed and rehydrated paraffin sections were pretreated with heat using standard condition (40 minutes). The primary antibody was incubated for 1 hour at 37°C. After incubation with a rabbit Immpress HRP (Ready to use, Vector laboratories Laboratories), chromogenic revelation was performed with ChromoMap DAB kit (Roche Diagnostics, cat #760-159). Sections were counterstained with Harris hematoxyline and permanently mounted. Immunohistochemical detection of mouse CTSS was performed manually. After dewaxing and rehydration, sections were incubated for 10 minutes in 3% H2O2 in PBS to inhibit endogenous peroxidase. They were pretreated with 10mM Tri Na citrate pH6 for 20min at 95°C using PT module (Thermo Fisher Scientific). Slides were then blocked in 1% BSA in PBS for 30 minutes. Rabbit α-mouse CTSS (clone #054, sino Biological, cat #50769-R054) diluted 1:100 in 1% BSA was incubated overnight at 4°C under agitation. After PBS washes, the secondary antibody Immpress HRP rabbit (Vector Laboratories, cat #MP-7401) was applied for 30 minutes at room temperature.

*Multiplexing*

The 4plex immonufluorescence was performed using the fully automated Ventana Discovery ULTRA (Roche Diagnostics, Rotkreuz, Switzerland). All steps were performed on the automate with Ventana solutions following their recommendations. Briefly, dewaxed and rehydrated paraffin sections were pretreated with heat using standard condition (40 minutes) CC1 solution. Primary antibodies: rat anti mouse F4/80 (clone C1:A3-1, Biorad, cat #MCA497GA), rat anti-mouse CD8 (clone 4SM15, ebioscience, cat #14-0808-82), rat anti mouse CD4 (clone 4SM95, ebioscience, cat #14-9766-82), rat anti mouse B220 (clone RA3-6B2, ebioscience, cat #13-0452-82) were applied sequentially and incubated 1 hour at 37°C. After incubation with a rat Immpress HRP (Ready to use, Vector laboratories Laboratories), they were respectively revealed with the Rhodamin6G (Ventana, cat #760-244), Red 610 (Ventana, cat #760-245), Cyanine 5 (Ventana, cat #760-238), Fluorescein amidite (Ventana, cat #760-243) fluorescent kits (Roche Diagnostics, Rotkreuz, Switzerland). Heat denaturation was applied between each antibody labeling. Slides were acquired with Olympus slide scanner and analyzed using QuPath.

**Tissue microarray analyses of primary human FL samples**

Immunohistochemistry detection of human CTSS was performed using the rabbit monoclonal Anti-Cathepsin S antibody (clone EPR5128, Abcam, cat #ab134157), diluted 1:50, on a fully automated Ventana Benchmark XT System. Revelation was performed with DAB (3,3'-Diaminobenzidine, Sigma-Aldrich) and sections were counterstained with Harris hematoxylin. To assess the combined levels of CD4, CD8, and PD1 in samples classified as CTSS High, Intermediate, and Low, the following was carried out:

1. Rescaled all protein levels to the [0,1] interval;
2. Classified each sample as "High" or "Low" for each protein (CD4, CD8, and PD1) based on the rescaled level being greater or smaller than 0.2, respectively;
3. For each combination of CD4, CD8, and PD1 statuses ($C_i$, i = 1,...,8) the number of samples classified as CTSS High, Intermediate, and Low (Observed counts) was computed;
4. For each combination of CD4, CD8, and PD1 statuses ($C_i$, i = 1,...,8) the expected number of samples classified as CTSS High, Intermediate, and Low was computed by multiplying the total number of samples in $C_i$ by the fraction of samples with a given CTSS status (Expected values);
5. Finally, the enrichment of each CTSS group in each $C_i$, was computed by the fold-changes of observed vs. expected counts [$\log_2$(Obs+1)-$\log_2$(Exp+1)].

Protein levels (original and rescaled values) and CTSS classes are reported in Table S4.

**Mutual exclusive analysis**

Mutual exclusivity between mutations in CTSS, RRAGC, and TNFRSF14 was tested using the MEMo algorithm (Ciriello et al., 2012). Briefly, the mutation status of 12 recurrently mutated genes in FL was derived from two distinct datasets (B2M, CREBBP, CTSS, KMT2D, TNFRSF14, ARID1A, FOXO1, MEF2B, CARD11, RRAGC, EP300, EZH2) including a total of 323 samples. Mutation calls were converted to a binary matrix of mutation occurrences per sample, M, and M was used as input to generate 1000 matrices of random alteration occurrences. Each random matrix is obtained

by permuting the values in M in a way that preserves the total number of mutations per gene and per sample. Next, the gene module [CTSS, RRAGC, TNFRSF14] was tested for significant mutual exclusivity by comparing the total observed number of samples exhibiting a mutation in at least one of the three genes, to the corresponding expected number estimated from the random matrices. An empirical p-value was derived as the fraction of random trials giving a total number of altered samples greater or equal to the one observed.

**T cells assay**

*Cell purification*

HA-specific TCR CD8+ T cells, OVA-specific CD8+ Tcells, and OVA-specific CD4+ Tcells were isolated from spleens of CL4, OT-I, OT-II or DO11.10 transgenic mice respectively. vavP-Bcl2WT and vavP-Bcl2-Ctss-KO were isolated from the spleen. Spleens were smashed, resuspended in PBS and filtered through a 40 μm filter (Falcon, #352350) to generate a single cell suspension. Single cell suspension was then processed through a negative mouse CD8 Tcell (Miltenyi Biotech, cat #130-104-075) CD4 Tcell (Miltenyi Biotech, cat#130-104-454) or pan-Bcell (Miltenyi Biotech, cat #130-104-443) isolation kit following the manufacturer's instructions.

*Co-culture*

Purified vavP-Bcl2 WT or Ctss KO B-cells, A20 WT or Ctss KO cells were stimulated for 2 hours with 100ng/mL LPS (Sigma Aldrich, cat # L4391), and subsequently loaded with 1mg/mL OVA (InvivoGen, cat #vac-stova), or 5ug/mL SIINFEKL (InvivoGen, cat #vac-sin) peptide for 4 hours at 37C. To study MHC-II mediated Tcell activation. OVA loaded WT and Ctss-KO Bcells were co-incubated with CellTrace Violet (Thermo Fisher Scientific, Reinach, Switzerland, cat # C34557) stained CD4+ OT-II Tcells while OVA loaded A20 WT and A20 Ctss KO cells were co-incubated with CellTrace Violet stained DO.11.10 CD4+ Tcells. Alternatively, OVA loaded WT and Ctss-KO Bcells were co-incubated with OT-I CD8+ Tcells to study MHC-I mediated Tcell activation.

At the end of the B-cell/T cell co-culture, supernatants were collected and analyzed for the proliferation of T cells stained with an Pe-Cy7 anti-mouse CD8a antibody (Clone 53-6.7, BD Biosciences, San Jose, CA, USA, cat #552877) or PercP efluor 710 rat anti-CD4 antibody (bd bioscience, cat#564379) by quantifying CellTrace violet proliferation peaks using flow cytometry. In parallel, B cell concentration was measured using cell-counting flow-cytometer (Guava easyCyte). A20-HA lymphoma cells wild-type, Ctss knock-out, B2m knock-out, and Pdl1 knock-out expressing HA as antigen were pre-treated with 30uM E64 (Apexbio Technology LLC (cat #A2576), 30uM Z-FL-COCHO (Anawa trading SA, cat #A13502-5), 30uM LY300328 (Lucerna Chem AG, cat # MCE-HY-15533) or-directly co-incubated for 72h with CellTrace violet stained CD8+ T-cells isolated from CL4-HA transgenic animals. At the end of the B-cell/T cell co-culture, cells were collected, stained with PE-Cy7 anti-mouse CD8a antibody (Clone 53-6.7, BD Biosciences, San Jose, CA, USA cat #552877) and analyzed for the proliferation of T cells stained by quantifying CellTrace violet proliferation peaks using flow cytometry. In parallel, B cell concentration was measured using cell-counting flow-cytometer (Guava easyCyte).

**Immunopeptidome**

*Data generation*

**Generation of antibody-crosslinked beads**

Anti-pan HLAI and anti-pan HLAII monoclonal antibodies were purified from the supernatant of HB95 (ATCC® HB-95™) and HB145 cells (ATCC® HB-145™). Anti-MHC-I and anti-MHC-II monoclonal antibodies were purified from the supernatant of HIB 34.1.2 (gift from Angel Miguel Garcia Laura, Junta de Andalucia Laboratory) and HB225 cells (ATCC® HB-225™). The hybridoma were grown in CELLLine CL-1000 and CL-350 flasks (Sigma-Aldrich, St. Louis, Missouri) and the antibodies were purified using Protein A-Sepharose 4B beads (Pro-A beads; Invitrogen, Carlsbad, California). Antibodies were cross-linked to Protein A-Sepharose 4B beads at a concentration of 5 mg of antibodies per 1 mL volume of beads.

**Purification of MHC-class-I and MHC-class-II peptides**

Human (DOHH2) and mouse (A20) cells were lysed in PBS containing 0.25% sodium deoxycholate (Sigma-Aldrich), 0.2 mM iodoacetamide (Sigma-Aldrich), 1 mM EDTA, 1:200 Protease Inhibitors Cocktail (Sigma-Aldrich), 1 mM Phenylmethylsulfonylfluoride (Roche, Basel, Switzerland), 1% octyl-beta-D glucopyranoside (Sigma-Alrich) at 4°C for 1 hour. The lysis buffer was added to the cells at a concentration of 1 x $10^8$ cells/mL. Lysates were cleared by centrifugation

with a table-top centrifuge (Eppendorf Centrifuge, Hamburg, Germany) at 4°C at 14200 rpm for 50 min. For mouse samples, peptides purification was performed in columns: MHC-II molecules were purified by incubating the cleared lysates with HB225 antibodies cross-linked to Protein A-Sepharose 4B beads in affinity columns for 2 hours at 4°C. Then the MHC-I molecules were purified by transferring and incubating the flow through lysates into similar affinity columns containing HIB antibodies cross-linked to Protein A-Sepharose 4B beads for 3 hours at 4°C. For human samples, peptides purification was performed using the Waters Positive Pressure-96 Processor (Waters) and 96-well single-use microplates with 3μm glass fiber and 10μm polypropylene membranes (Seahorse Bioscience, ref no: 360063): Anti-pan MHC-I and MHC-II antibodies cross-linked to beads were loaded on their respective plates and the lysates were passed sequentially through MHC-II and -I plates.

The beads in columns (mouse samples) and in plates (human samples) were then washed with varying concentrations of salts and finally with 20 mM Tris-HCl pH 8. MHC complexes and their bound peptides were eluted with 1% % trifluoroacetic acid (TFA; Sigma-Aldrich) and loaded on pre-conditioned Sep-Pak tC18 100 mg Sorbent 96-well plates (Waters, ref no: 186002321) for the purification and concentration of the peptides. After washing the C18 wells with 2 mL of 0.1 % TFA, class I peptides were eluted with 28% Acetonitrile (ACN; Sigma Aldrich) in 0.1% TFA while class II peptides were eluted with 32% ACN in 0.1% TFA. Recovered class I and -II peptides were dried using vacuum centrifugation (Thermo Fisher Scientific) and stored at -20°C.

**LC-MS/MS analyses**

The peptides bound to MHC-class-I and MHC-class-II were identified with LC-MS/MS system consisting of an Easy-nLC 1200 (Thermo Scientifc, Bremen, Germany) and the Q Exactive HF-X mass spectrometer (Thermo Scientific, Bremen, Germany). Peptides were separated on a 450 mm analytical column of 75 μm inner diameter for 120 min with a flow rate of 250 nL/min.

For MHC class I peptides, the following gradient were used: 0.1% formaldehyde in 80% acetonitrile buffer B): 0-5 min (5% B); 5-85 min (5-35% B); 85-100 min (35-60 % B); 100-105 min (60-95% B); 105-110 min (95% B); 110-115 min (95-2% B) and 115-125 min (2% B). For HLA or MHC class II peptides, the gradient consisted of: 0-5 min (2-5% B); 5-65 min (5-30% B); 65-70 min (30-60% B); 70-75 min (60-95 % B); 75-80 min (95% B), 80-85 min (95-2% B) and 85-90 min (2% B). MS spectra were acquired in the Orbitrap with m/z range from 300-1650, with a resolution of 60'000 (m/z = 200) and ion accumulation time of 80ms. The AGC was set to 3e6 ions. MS/MS spectra were acquired in a data-dependent manner. Ten most abundant precursor ions were selected for fragmentation, with a resolution of 15'000(m/z = 200) and ion accumulation time of 120 ms with isolation window of 1.2 m/z. For fragmentation, the AGC was set to 2e5 ions, dynamic exclusion to 20 s, and a normalized collision energy (NCE) of 27.

**Identification of HLA and MHC peptides**

The immunopeptidomics MS data was searched separately with MaxQuant version 1.5.5.1 against a human or mouse UniProt reference databases, (Mouse, 24,907 entries from June 2016 and Human 42,148 entries from March 2017), including a list of 247 frequently observed contaminants. The enzyme specificity was set as unspecific, and peptides with a length between 8 and 25 amino acids (AA) were allowed. The second peptide identification option in Andromeda was enabled. A false discovery rate (FDR) of 1% was required for MHC-II samples and 5% FDR for MHC-I samples. No protein FDR was set. The initial allowed mass deviation of the precursor ion was set to 6 ppm and the maximum fragment mass deviation was set to 20 ppm. Methionine oxidation and N-terminal acetylation were set as variable modifications.

*Analysis of number of peptides and length distribution*

To characterize MHC-presented peptides in CTSS knock-out (KO) and wild-type (WT) DOHH2 cells, three classes of peptides for both MHC-I and MHC-II were defined:

- KO-unique: the peptide was detected in at least 5 out 6 replicates of CTSS-KO cells and only in 1 or none of the CTSS-WT replicates;
- WT-unique: the peptide was detected in at least 5 out 6 replicates of CTSS-WT cells and only in 1 or none of the CTSS-KO replicates;
- Shared: the peptide was detected in at least 5 out 6 replicates of both CTSS-KO and CTSS-WT cells.

For the analysis of MHC-I peptides, peptides longer than 15 amino-acids were excluded, whereas for the analysis of MHC-II peptides, peptides shorter 13 amino-acids were excluded, as likely affected by cross-reactivity of MHC antibodies. The length distributions of the remaining peptides in each class were compared based on Kolmogorov-Smirnov statistics using the ks.test R function (one-tail p-values are reported). The same test was used in the comparison between MHC-II KO-unique peptides that included CTSS cleavages sites and those that did not.

*Peptides alignment and scoring*

Peptides mapping to the same protein can be highly overlapping, i.e. sharing a considerable fraction of their sequence and differ for only a few amino-acids. To identify sequence similarities among peptides, sequence alignment of pairs of peptides were performed. Protein sequence alignment was performed using the pairwiseAlignment function implemented in the Biostrings R package (with parameters: type = 'overlap', gapOpening = 100, gapExtension = 100). Based on the observed distribution of the alignment scores and visual inspection of the aligned sequences, we considered two sequences as *matched* if they obtained a score > 30. Using this peptide alignment procedure, the alignment of CTSS cleavage sites that were predicted in KO-unique peptides to both the corresponding KO-unique peptide and *matched* WT-unique peptides were tested. Alignment scores of CTSS cleavage sites to KO-unique and matched WT-unique peptides were computed and compared as follow:

1. First, each KO-unique peptide containing a CTSS cleavage site was aligned to all WT-unique peptides and retained only matched peptides (score > 30);
Next, for each KO-unique peptide *k*:
2. the corresponding CTSS cleavage site was aligned to *k* and determined its score, $S_k$;
3. the corresponding CTSS cleavage site was aligned to each matched WT-unique peptide, $w_i$, and determined their scores ($S_{w1}$, ... $S_{wn}$);
4. the scores were normalized with respect to $S_k$, such that $S_k$ = 1 and $S_{wi}$ < 1 (> 1) indicates a worse (better) alignment of the CTSS cleavage site to *k* than to $w_i$;
5. finally, KO-unique peptides were classified in 3 groups:

    a. *decreasing:* the majority of matched WT-unique peptides have scores < 1,
    b. *increasing:* the majority of matched WT-unique peptides have scores > 1,
    c. *stable:* all scores of matched peptides are within the [0.9,1.1] interval.

Note that if multiple CTSS cleavage sites were predicted for the same KO-unique peptide, then each was tested separately.

*Prediction of possible CTSS cleavage sites in KO-unique peptides*

Each peptide detected by MS/MS was mapped to one or several protein sequences as reported previously (*Bassani-Sternberg et al., 2017, PLOS Comput. Biol., 13, e1005725).* For each pair of MS-detected peptide and peptide-containing protein, the latter was decomposed into all possible octuplets of consecutive 8-mer peptides (i.e. sliding windows of 8 residues). These octuplets were ranked according to *the f*(*Pep*) score described above to predict potential CTSS cleavage sites. If the center of the top-scored octuplet is included in the MS-detected peptide, the latter was considered to be possibly cleavable by CTSS, defining a positive peptide at rank 1. The same procedure was repeated considering the top-5 octuplets, defining this way the positive peptides at rank 5. Dividing the number of positive peptides at rank 1 or rank 5 by the number of unique MS-detected peptides defined the fraction of positive peptides at rank 1 or rank 5, respectively. The calculation of the fractions of positive peptides at rank 1 and rank 5 was performed for the list of MS-detected peptides obtained from WT and CTSS-KO cells. As a control, the fraction of positive peptides at rank 1 and 5 was calculated, by replacing each MS-detected peptide by another one of the same size randomly chosen in the same mapped protein. These fractions are expected to provide an estimate of the frequency with which a predicted cleavage site could be found in a peptide by chance. This randomization procedure was repeated 1000 times for the WT and CTSS-KO list of peptides. The standard deviation of the fractions of positive peptides was calculated for both cases, and the p-value for the comparison to the real sets of peptides was estimated empirically.

**Example 6: Mass spectrometry**

For mass spectrometry experiments, CTSS-His WT and Y132D were activated by incubation at 10 μg/mL in Assay buffer (50 mM NaOAc, 5 mM DTT, 250 mM NaCl, pH 4.5) at 37°C for 90 minutes. 2.5 μg of activated CTSS-His WT and Y132D were then co-incubated with 5 ug of peptide in Assay buffer (final volume: 300 μl) at 37°C for 15 or 30 minutes. The reaction was stopped by addition of 50 μM E64 (Selleckchem, cat #S7379) following the co-incubation. Control reactions were prepared by incubating 5 μg of peptide in 300 μl of Assay buffer at 37°C for 15 or 30 minutes. Control and digested peptides were acidified, desalted on C18 StageTips (Rappsilber et al., 2007) and dried by *vacuum*

centrifugation. Samples were resuspended in LC-MS solvent A (2% Acetonitrile, 0.1% TFA) and equivalent sample amounts (3 to 10 ng) injections were performed. Nano-flow separations were achieved on a Dionex Ultimate 3000 RSLC nano UPLC system (Thermo Fischer Scientific) on-line connected with a Q-Exactive Mass-Spectrometer (Thermo Fischer Scientific). Samples were first trapped on a capillary C18 pre-column (Acclaim PepMap™ 100, 75 μm x 2cm) and then separated on a C18 home-made capillary column (ReproSil-Pur C18 AQ 1.9 μm; 50 cm x 75 μm ID) at 250 nl/min over a biphasic 60 min gradient ranging from 99% of solvent A (2% Acetonitrile and 0.1% Formic acid) to 90% of solvent B (90% Acetonitrile and 0.1% Formic acid). The mass spectrometer was operated in Data Dependent acquisition mode using a scan range of 200 to 1500 m/z. MS resolution was set to 70K and MS2 resolution to 17,5K. An isolation window of 2 m/z was used when selecting ions to fragment by HCD and the Normalized Collision Energy (NCE) was set to 27. Data were inspected and dedicated traces extracted with Xcalibur Qual Browser software (Thermo Fischer Scientific). The peptides used were the following: CD74 (QLENLRMKLPKPPKPVSKMR) **(SEQ ID NO: 16),** HSPA1/8 (EEIERMVQEAEKYKAEDEVQ) **(SEQ ID NO: 17),** OVA (IKVYLPRMKMEEKYNLTSVL) **(SEQ ID NO: 18),** HA (NVKNLYEKVKSQLKNNAKEI) **(SEQ ID NO: 19).**

## TCR sequencing

CD8+ T cells were isolated from the liver of A20 engrafted animals using the CD8a+ T Cell isolation kit, mouse (Miltenyi Biotec, cat #130-104-075). DNA was extracted using phenolchloroform, followed by isoamyl alcohol, and isopropanol and ethanol precipitations. The TCR repertoire was analyzed using the ImmunoSeq survey platform from Adaptive Biotechnologies. Briefly, the hyper-variable CDR3 region (complementarity-determining region 3) of the TCRβ was amplified with forward primers against the TCR-Vβ and reverse primers against the TCR-Jβ sequences, and PCR products were sequenced on Illumina platforms. The WT and KO conditions were analyzed in biological triplicates using cells engrafted in different mice. Raw data were analyzed with the ImmunoSeq pipeline, then harvested and processed using R. To assess TCR heterogeneity, the top 500 rearrangements detected in each condition were retrieved and only rearrangements with more than 10 templates were retained.

## *In vivo* treatments

To evaluate the anti-tumor activity of anti-PD1 treatment *in vivo,* animals were recruited when the tumors reached 6 × 6 mm. Mice were either treated intraperitoneally with 200 μg of rat anti-mouse PD1 (clone RMP1-14, BioXcell, cat# BE0146) or saline solution twice a week.

## Example 7: Antigen diversification in cathepsin S knock-out cells translates in CD8+ T-cell polyclonal expansion

To assess if the increased diversity in the endogenous antigenic repertoire in CTSS knock-out cells could influence the expansion of CD8+ T-cells infiltrating the tumor *in vivo,* the heterogeneity of the TCR repertoire of CD8+ T-cells was measured in *Ctss* knock-out and wild-type tumors. CD8+ T-cells were purified from three independent animals harboring *Ctss* knock-out and wild-type metastatic A20 lymphoma. Quantitative analysis of productive TCR rearrangements showed that CD8+ T-cells isolated from the *Ctss* knock-out tumors underwent polyclonal expansion. Indeed, in *Ctss* knock-out tumors, 14-15 TCR clones represented 50% of the TCR repertoire, compared to 5-to-11 clones in *Ctss* wild-type tumors. The frequency of the top 20 TCR in Ctss knock-out tumor accounted for 55-61% of the entire repertoire, while in tumor expressing Ctss wild-type it accounted for 66%-74% of the entire repertoire. This suggested that the TCR repertoire is more heterogeneous in animals engrafted with Ctss KO tumors than in animals engrafted with Ctss wild-type tumors. Thus, modifications of the antigenic repertoire can support multiclonal expansion of cytotoxic CD8+ T-cells in *Ctss* knock-out tumors and this contributes to trigger adaptive anti-tumor immune response.

Altogether, those data show that lymphoma development depends on cathepsin S activity and tumor cells acquire genomic alterations that enhance its proteolytic function. Cathepsin S activity is required to maintain pro-proliferative co-stimulatory signals between cancer cells and CD4+ T-cells in the germinal centers. Maintaining this communication is particularly important in the early phase of tumor development and in indolent tumors, where lymphoma cell proliferation depend on exogenous signals. Conversely, in more advanced stages of the disease and aggressive lymphoma, cancer cells acquire additional genomic alterations that will support tumor cell proliferation through intrinsic signals *(Okosun et al., 2014, supra; Oricchio et al., 2014, supra; Pasqualucci et al., 2011, supra).* Nevertheless, loss of CTSS in both indolent and aggressive lymphoma expose the tumor to unforeseen vulnerabilities.

Loss of cathepsin S activity in lymphoma cells alters the processing of both CD74 and endogenous and exogenous antigenic peptides. CD74 is the chaperon protein bound to MHC-class-II molecules and CD74 processing is important to regulate antigen loading. The alteration of CD74 and antigenic peptide processing weakened the communication between lymphoma and CD4+ Tfh cells in the germinal centers. In transgenic animal models, this loss of interaction abrogates the proliferation of lymphoma cells. Importantly, cathepsin S knock-out results in diversification of

the antigen repertoire in lymphoma cells, and this is associated with recruitment and activation of CD8+ T-cells in both aggressive and indolent tumors. Thus, altering the processing of endogenous antigenic peptides can directly increase CD8+ T-cells anti-tumor cytotoxic activity. Cathepsin S activity seems to be at the cross-road between MHC-class-I and MHC-class-II cross-antigen presentation accumulation or delay in MHC-class-II antigen processing and loading could contribute to cross-presentation of these antigens by MHC-class-I. Therefore, by targeting cathepsin S, adaptive immuno-response and response to immunotherapies could be improved. Hence, cathepsin S represents an interesting therapeutic target.

**Example 8: Activity of Cathepsin S inhibitors of the invention**

A known generic E64 protease inhibitor (CAS :66701-25-5) (Selleckchem, cat #S7379 was tested in the co-culture assay with two inhibitors Z-FL-COCHO (Anawa trading SA, cat #A13502-5, CAS: 1373215-15-6), and LY300328 (Lucerna Chem AG, cat # MCE-HY-15533) described as having an increased specificity for cathepsin S *(*Seo et al., 2016, Antioxid. Redox Signal., 27, 215-233 *and* 2018, Biochem. Biophys. Res. Commun., 498, 849-854*;* Jadhav et al., 2014, ACS Med. Chem. Lett., 5, 1138-1142*;* Payne et al., 2014, Br. J. Clin. Pharmacol., 78, 1334-134). However, while accumulation of the small fragment of CD74, p10 was observed that indicates alterations of MHC-class-II-antigen processing no over-activation of CD8+ T-cells was observed. Thus, the lack of specificity of currently available protease inhibitors limits their application.

Therefore, the design of pharmacological inhibitors which selectively bind to cathepsin S specific amino acids such as tyrosine 132 would help improving specificity and overcoming limitations of current small molecule inhibitors.

For this purpose, candidate Cathepsin S inhibitors were identified in the FRET assay described above.

The FRET assay was miniaturized to performed the screen in 396-well plate and each molecule was tested in duplicates. 10 μg/ml of CTSS was incubated in the activation buffer for 105 minutes to allow a complete activation of the enzyme. Then, the enzyme was diluted at 0.5 μg/ml and dispensed in 396-well plate pre-loaded with the small molecule candidates using an automatic dispenser (Biotek Dispenser multiflo). The plates were incubated for 10 minutes at room temperature and then 20 μM of substrate was added in each well and acquired the fluorescence signal (excitation: 330 nm, emission: 390 nm) after 90 minutes. For each plate, were included untreated wells as negative control and E64 was used (150 μM) as positive control. To assess the quality of the screen, a Z'-score for each plate was calculated and all plates had Z' score > 0.6. To identify possible hits, the average value and standard deviation of the positive and negative control were calculated. The upper and lower limit values for each plate were calculated considering plus/minus three times the standard deviation to the average and only compounds that in both independent duplicate scored below the positive control threshold were retained.

Following these criteria, 20 compounds were selected and a dose response curve and interference test to exclude auto fluorescent compounds was performed. After evaluation of $IC_{50}$, interference assay and *in silico* analyses of chemical properties of the compound, 6 compounds were selected to perform cell viability and specificity assays.

**Specificity assay**

In order to confirm whether the small molecules Cathepsin S inhibitors identified in the FRET assay could also inhibit the activity of other cathepsin proteins as cathepsin B (CTSB) or cathepsin H (CTSH), a FRET assay to test CTSB and CTSH activity was developed. First, specific conditions to activate the enzymes were used as follows:
CTSB was diluted in active buffer (MES 25 mM, DTT 5 mM, pH 5) at 10 μg/ml, incubated at 37°C for 2h and then diluted to 1 μg/ml in assay buffer (MES 25 mM, pH 5) for the FRET assay. CTSH was diluted in a specific active buffer (MES 50 mM, $CaCl_2$ 10 mM, NaCl 150 mM, pH 6.5) 200 μg/ml. In addition, as co-stimulatory activator thermolysin diluted at 100 μg/ml in active buffer was used mixed with equal volume of the diluted CTSH. The solution was incubated for 1h room temperature and then we added 2 mM Phosporamidon diluted in assay buffer (MES 50 mM, pH 6,5) and incubate the mix for 10 minutes at room temperature. Lastly, an equal volume of assay buffer containing 20 mM DTT was added and the mixture incubated 5 minutes at room temperature. For the FRET assay, diluted both CTSH, and CTSB at 1 μg/ml were used in their specific assay buffer and incubated with a specific substrate. As substrate, the following peptides were used: peptide of **SEQ ID NO: 5** (KPPKPVSD) in CTSB FRET assays and **SEQ ID NO: 1** (KLRMKLPKP) in CTSH FRET assays and tested increasing doses of candidate inhibitors (20 μM, 10 μM, 1 μM, 0.1 μM). Fluorescence emission was measured with SpectraMax iD3 (Molecular Devices) in kinetic mode over three hours (excitation: 330 nm, emission: 390 nm).

**Cell viability assay**

Two human lymphoma cell lines DoHH2 and Karpas-422 and one murine lymphoma cell line A20 were treated with increasing concentration of compounds **(1), (5)** and **(9).** 24 hours before treatment cells were diluted at $2*10^5$ cells/ml.

Cell number was measured at 48h and 72h after treatment using automatic cell counting of Millipore Guava. The values were normalized to cells treated with the same dose of vehicle (DMSO).

To assess the ability of Cathepsin S inhibitors of the invention to inhibit the activity of cathepsin proteins, each compound was tested using a specific FRET assay for cathepsin S (CTSS), cathepsin B (CTSB) and cathepsin H (CTSH). E64 a generic protease inhibitor commercially available was used as positive control. The inhibition of CTSS activity is dose dependent with all three compounds **(Fig. 3).** However, these three compounds have less potent activity on CTSB **(Fig. 4)** and CTSH **(Fig. 5).**

Next, the toxicity of these inhibitors on two human lymphoma cancer cell lines (Karpas-422 and DoHH2). The cells were treated with the small molecules for 48h or 72h at increasing doses (50 μM, 10 μM, μM, 0.1 μM). In these conditions, compounds **(1)** and **(5)** are well tolerated in both lymphoma cell lines and cell survival was not affected indicating that the effect of cathepsin S inhibition is associated with activation of immune-response. Conversely, compound **(9)** progressively decreases cell proliferation of up to 50% after 72 hours of exposure to the 0. 1 μM of the compound or less than 30% in cells treated with 10 or 50 μM. Overall, these results suggest that the above Cathepsin S inhibitors have a selective activity to inhibit cathepsin S.

**SEQUENCE LISTING**

**SEQ ID NO: 1 -** Synthetic peptide as Cathepsin S and H substrate
KLRMKLPKP

**SEQ ID NO: 2** - Synthetic peptide as Cathepsin S and H substrate
KLRMKLPKD

**SEQ ID NO: 3 -** Synthetic peptide as Cathepsin S and H substrate
KLRMKLPKK

**SEQ ID NO: 4** - Synthetic peptide as Cathepsin S and B substrate
KPPKPVSK

**SEQ ID NO: 5** - Synthetic peptide as Cathepsin S and B substrate
KPPKPVSD

**SEQ ID NO: 6** - CTSS_pHLsec_fw plasmid sequence (artificial)
GCTGAAACCGGTCAGTTGCATAAAGATCCTAC

**SEQ ID NO: 7** - CTSS_pHLsec_rev plasmid sequence (artificial)
GTGCTTGGTACCGATTTCTGGGTAAGAGGGAAAGC

**SEQ ID NO: 8 -** mCtss-gRNA-For oligonucleotide to generate cathepsin S KO cells (artificial)
GGGAATGCATACCTACCAAG

**SEQ ID NO: 9** - mCtss-gRNA-Rev oligonucleotide to generate cathepsin S KO cells (artificial)
CTTGGTAGGTATGCATTCCC

**SEQ ID NO: 10-** mPdl1-gRNA- For oligonucleotide to generate Pdl1 KO cells (artificial)
CGAGGGTTATCCAGAAGCTG

**SEQ ID NO: 11 -** mPdl1-gRNA-Rev oligonucleotide to generate Pdl1 KO cells (artificial)
CAGCTTCTGGATAACCCTCG

**SEQ ID NO: 12 -** mβ2m -gRNA-For oligonucleotide to generate β2m KO cells (artificial)
AGTATACTCACGCCACCCAC

**SEQ ID NO: 13 -** mβ2m -gRNA-For oligonucleotide to generate β2m KO cells (artificial)
GTGGGTGGCGTGAGTATACT

**SEQ ID NO: 14 -** hCTSS-gRNA-For oligonucleotide to generate cathepsin S KO cells (artificial)
CACCGTTGCATAAAGATCCTACCC

**SEQ ID NO: 15 -** hCTSS- gRNA-Rev oligonucleotide to generate cathepsin S KO cells
AAACGGGTAGGATCTTTATGCAAC

**SEQ ID NO: 16 -** Synthetic CD74 peptide
QLENLRMKLPKPPKPVSKMR

**SEQ ID NO: 17 -** Synthetic HSPA1/8 peptide
EEIERMVQEAEKYKAEDEVQ

**SEQ ID NO: 18** - Synthetic OVA peptide
IKVYLPRMKMEEKYNL TSVL

**SEQ ID NO: 19 -** Synthetic HA peptide
NVKNLYEKVKSQLKNNAKEI

**SEQ ID NO: 20** - hCTSS-For oligonucleotide to detect expression of cathepsin S in human cells (synthetic)
GGATCACCACTGGCATCTCT

**SEQ ID NO: 21 -** hCTSS-Rev oligonucleotide to detect expression of cathepsin S in human cells (synthetic)
ATTCCCATTGAATGCTCCAG

**SEQ ID NO: 22 -** mHPRT1-For oligonucleotide to detect expression of HPRT in human cells (synthetic)
CAAACTTTGCTTTCCCTGGT

**SEQ ID NO: 23 -** mHPRT1-Rev oligonucleotide to detect expression of HPRT in human cells (synthetic)
CAAGGGCATATCCAACAACA

**SEQ ID NO: 24 -** Consensus sequence for peptides substrates of Cathepsin S and H
KLRMKLPKXaa wherein Xaa is any amino acid.

## Claims

1. A cathepsin inhibitor, in particular cathepsin S inhibitor for use in the prevention and/or treatment of a cancer wherein said cathepsin inhibitor is to be administered in combination with immunotherapy or an immunological disorder.

2. A cathepsin inhibitor for use according to claim 1, for use in anti-cancer immunotherapy.

3. A cathepsin inhibitor for use according to claim 1 or 2 wherein cancer is a solid tumor cancer, such as lung cancer, breast cancer, ovarian cancer, cervical cancer, uterus cancer, head and neck cancer, glioblastoma, hepatocellular carcinoma, colon cancer, rectal cancer, colorectal carcinoma, kidney cancer, prostate cancer, gastric cancer, bronchus cancer, pancreatic cancer, urinary bladder cancer, hepatic cancer and brain cancer or skin cancer, in particular melanoma or an hematological cancer.

4. A cathepsin inhibitor for use according to any one of claims 1 to 3, wherein said cathepsin inhibitor is of Formula (I):

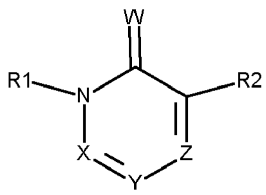

**(I)**

Wherein $R^1$ is optionally substituted $C_1$-$C_6$ alkyl, such as optionally substituted amino $C_1$-$C_6$ alkyl, optionally substituted aminocarbonyl $C_1$-$C_6$ alkyl, optionally substituted acylamino $C_1$-$C_6$ alkyl and optionally substituted aminosulfonyl $C_1$-$C_6$ alkyl; $R^2$ is selected from H, optionally substituted $C_1$-$C_6$ alkyl optionally substituted aryl and optionally substituted heteroaryl; W is selected from O and S; X is selected from N and $CR^{11}$; Y is selected from N and $CR^{12}$ and Z is selected from N and $CR^{13}$, wherein $R^{11}$ is selected from H and optionally substituted $C_1$-$C_6$ alkyl, $R^{12}$ is selected from H, optionally substituted $C_1$-$C_6$ alkyl, optionally substituted aryl such as optionally substituted phenyl (e.g. methoxy phenyl like 4-methoxy phenyl, chlorophenyl like 2-chlorophenyl, 4-fluorophenyl and optionally substituted heteroaryl such as optionally substituted furanyl (e.g. optionally substituted 2-furanyl) or optionally substituted

thiophene; or $R^2$ and $R^{13}$ can form an optionally substituted fused ring such as an optionally substituted heteroaryl like an optionally substituted thiazole (e.g. methyl thiazole fused to the diazinone ring) and an optionally substituted oxazole (e.g. methyl oxazole fused to the diazinone ring) as well as pharmaceutically acceptable salts, hydrates, solvates, tautomers, polymorphs, racemic mixtures, optically active forms, prodrugs and pharmaceutically active derivative thereof.

**5.** A cathepsin inhibitor for use according to claim 4, wherein $R^1$ is selected from optionally substituted $C_1$-$C_6$ alkyl, optionally substituted by one of the following groups: -NHC(O)-$R^{14}$, -O(CO)-$R^{14}$, -C(O)N$R^{14}R^{19}$ and -NHS$(O)_2$-$R^{14}$, wherein $R^{14}$ is selected from optionally substituted $C_4$-$C_{10}$ cycloalkyl, optionally substituted phenyl or optionally substituted heteroaryl $C_1$-$C_6$ alkyl, $R^{19}$ is selected from H and $C_1$-$C_6$ alkyl or $R^{14}$ and $R^{19}$ can form an optionally substituted $C_3$-$C_{10}$ cycloalkyl or an optionally substituted $C_3$-$C_8$ heterocycloalkyl.

**6.** A cathepsin inhibitor for use according to claim 4 or 5, wherein X is selected from N and CH.

**7.** A cathepsin inhibitor for use according to any one of claims 4 to 6, wherein compounds of Formula (I) are of the following Formula (Ia):

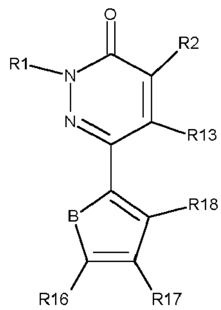

**(Ia)**

Wherein $R^2$ is selected from H and and optionally substituted $C_1$-$C_6$ alkyl, $R^{16}$ is selected from H and optionally substituted $C_1$-$C_6$ alkyl; $R^{17}$ is selected from H and optionally substituted $C_1$-$C_6$ alkyl, $R^{18}$ is selected from H and optionally substituted $C_1$-$C_6$ alkyl, B is selected from O and S and $R^1$ and R3 are as defined in any one of the preceding claims.

**8.** A cathepsin inhibitor for use according to any one of claims 4 to 7 selected from the following group:

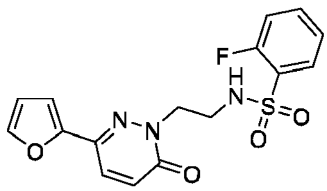

**(1)**

(2-fluoro-N-[2-[3-(2-furanyl)-6-oxo-1(6H)-pyridazinyl] ethyl]-benzenesulfonamide, CAS registry n° 946263-96-3);

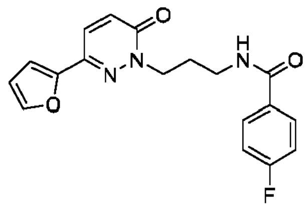

**(2)**

(4-fluoro-N-[3-[3-(2-furanyl)-6-oxo-1(6H)-pyridazinyl] propyl]-benzamide, CAS registry n° 1021225-32-0);

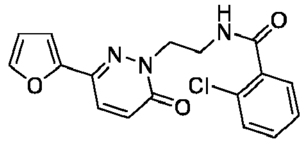

**(3)**

(2-chloro-N-[2-[3-(2-furanyl)-6-oxo-1(6H)-pyridazinyl] ethyl]-benzamide, CAS registry n° 946365-20-4);

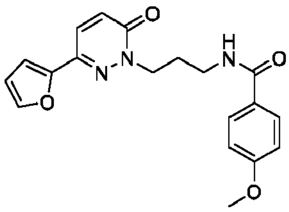

**(4)**

(N-[3-[3-(2-furanyl)-6-oxo-1(6H)-pyridazinyl]propyl]-4-methoxy-benzamide, CAS registry n° 1021225-23-9);

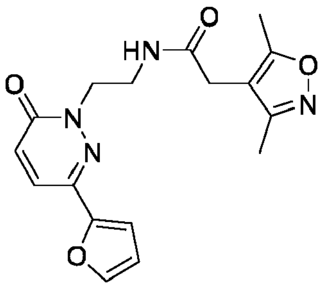

**(5)**

(N-[2-[3-(2-furanyl)-6-oxo-1(6H)-pyridazinyl]ethyl]-3, 5-dimethyl-4-isoxazoleacetamide, CAS registry n° 1219845-08-5);

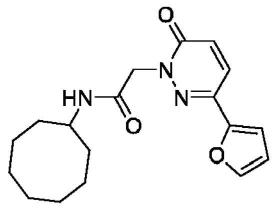

**(6)**

(N-cyclooctyl-3-(2-furanyl)-6-oxo-1(6H)-pyridazineacetamide, CAS registry n° 1282103-56-3);

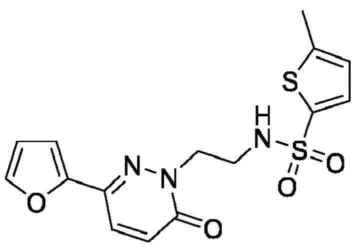

**(7)**

(N-[2-[3-(2-furanyl)-6-oxo-1(6H)-pyridazinyl]ethyl] -5-methyl-2-thiophenesulfonamide, CAS registry n° 946212-65-3); and

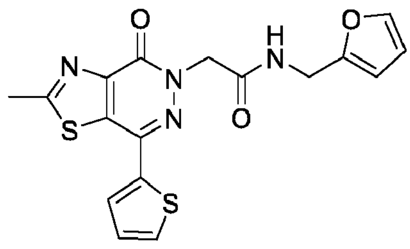

**(8)**

(N-(2-furanylmethyl)-2-methyl-4-oxo-7-(2-thienyl)-thiazolo[4,5-d]pyridazine-5(4H)-acetamide, CAS registry n° 942004-60-6), as well as pharmaceutically acceptable salts, hydrates, solvates, tautomers, polymorphs, racemic mixtures, optically active forms prodrugs and pharmaceutically active derivative thereof.

**9.** A cathepsin inhibitor for use according to any one of claims 1 to 3, wherein said cathepsin inhibitor is of Formula (II):

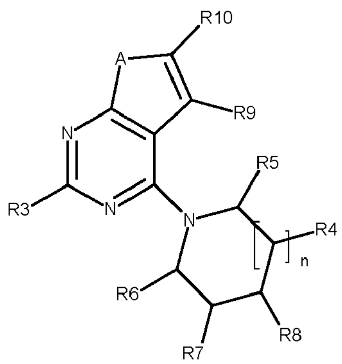

**(II)**

Wherein A is selected from O, S, N and $CR^{20}$; $R^3$ is selected from H, optionally substituted $C_1$-$C_6$ alkyl; $R^4$ is selected from H and optionally substituted $C_1$-$C_6$ alkyl; $R^5$ and $R^6$ are independently selected from H, optionally substituted $C_1$-$C_6$ alkyl, $R^7$ is selected from $-C(O)NR^{21}R^{22}$, $-C(O)OR^{21}$ and $-S(O_2)R^{21}$; $R^8$ is selected from H and optionally substituted $C_1$-$C_6$ alkyl; $R^9$ and $R^{10}$ are independently selected from optionally substituted $C_1$-$C_6$ alkyl, $R^{21}$ is selected from H and optionally substituted $C_1$-$C_6$ alkyl; $R^{22}$ is selected from optionally substituted optionally substituted heteroaryl $C_1$-$C_6$ alkyl; n is an integer selected from 0, 1 and 2, as well as pharmaceutically acceptable salts, hydrates, solvates, tautomers, polymorphs, racemic mixtures, optically active forms prodrugs and pharmaceutically active derivative thereof.

**10.** A cathepsin inhibitor for use according to claim 9 selected from the following group:

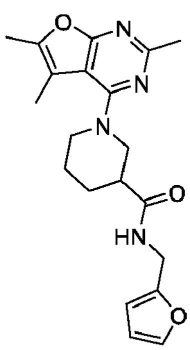

**(9)**

(N-(2-furanylmethyl)-1-(2,5,6-trimethylfuro[2,3-d]pyrimidin-4-yl)-3-piperidinecarboxamide, CAS registry n°1114614-87-7);

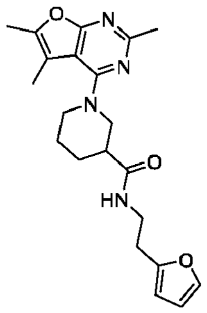

(10)

(N-[2-(2-furanyl)ethyl]-1-(2,5,6-trimethylfuro[2,3-d]pyrimidin-4-y-3-ppernecaroxame, CAS registry n°1111056-12-2); and

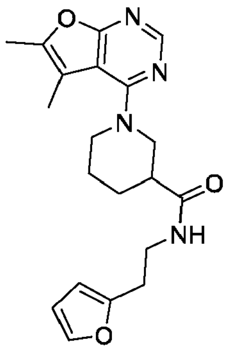

(11)

(1-(5,6-dimethylfuro[2,3-d]pyrimidin-4-yl)-N-[2-(2-furanyl) ethyl]-3-piperidinecarboxamide, CAS registry n°1114632-87-9), as well as pharmaceutically acceptable salts, hydrates, solvates, tautomers, polymorphs, racemic mixtures, optically active forms prodrugs and pharmaceutically active derivative thereof.

**11.** A pharmaceutical composition comprising at least one cathepsin inhibitor and a pharmaceutically acceptable carrier, diluent or excipient thereof and at least one agent useful in anti-cancer therapy or immunotherapy.

**12.** A method of identifying agents useful in the potentiation of immunotherapy, said method comprising the following steps:

- providing an activated Cathepsin enzyme selected from Cathepsin S, Cathepsin B and Cathepsin H;
- providing a Cathepsin substrate for said Cathepsin enzyme;
- contacting said activated Cathepsin enzyme with said substrate in presence or absence of at least one candidate agent;
- identifying agents that are able to inhibit Cathepsin, wherein said Cathepsin substrate is at least one peptide comprising or consisting in a sequence selected from **SEQ ID NO: 1, SEQ ID NO: 2, SEQ ID NO: 3, SEQ ID NO: 4,** and **SEQ ID NO: 5** or variant thereof when said Cathepsin enzyme is Cathepsin S, wherein said Cathepsin substrate is at least one peptide comprising or consisting in a sequence selected from **SEQ ID NO: 1, SEQ ID NO: 2** and **SEQ ID NO: 3,** or variant thereof wherein said Cathepsin enzyme is Cathepsin H and wherein said Cathepsin substrate is a peptide comprising or consisting in a sequence selected from **SEQ ID NO: 4 and SEQ ID NO: 5** or variant thereof, wherein said Cathepsin enzyme is Cathepsin B, said peptide further comprise a fluorescent label if Cathepsin enzyme activity is measured by fluorescence emission.

**13.** A use of a peptide comprising or consisting in a sequence selected from SEQ **ID NO: 1, SEQ ID NO: 2, SEQ ID NO: 3, SEQ ID NO: 4** and **SEQ ID NO: 5** or a variant thereof as a Cathepsin enzyme substrate.

**14.** A peptide comprising or consisting in a sequence selected from **SEQ ID NO: 1, SEQ ID NO: 2, SEQ ID NO: 3, SEQ ID NO: 4** and **SEQ ID NO: 5** or a variant thereof as a Cathepsin enzyme substrate, wherein said sequence comprises a fluorescent or fluorescence quencher label.

**15.** A kit for assaying Cathepsin enzyme activity, said kit comprising at least one peptide described in claim 13 or 14.

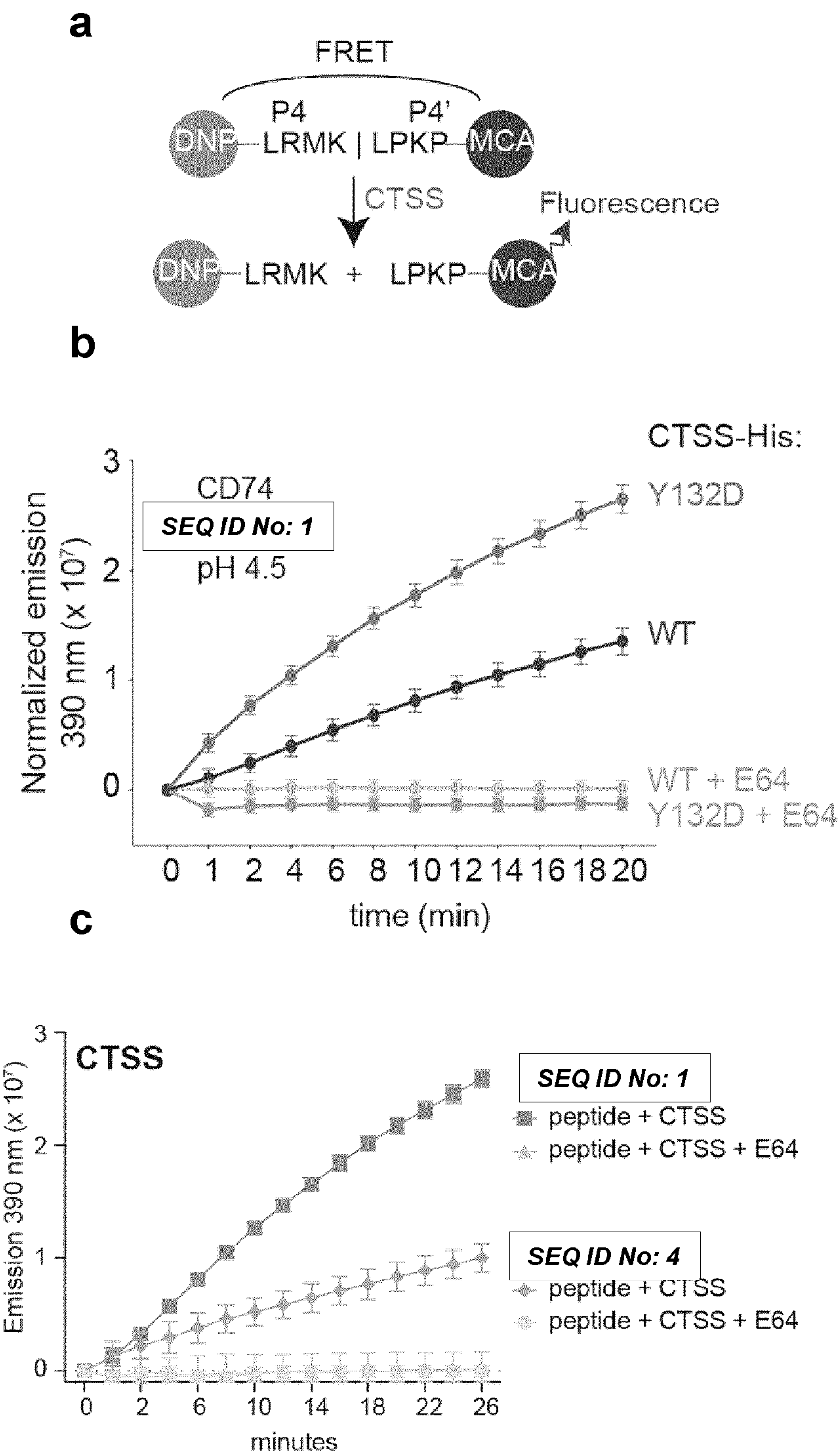
a
FRET
P4
P4'
DNP
LRMK | LPKP
MCA
CTSS
Fluorescence
DNP
LRMK + LPKP
MCA
b
CTSS-His:
CD74
SEQ ID No: 1
pH 4.5
Y132D
WT
WT + E64
Y132D + E64
Normalized emission 390 nm (x 10^7)
0 1 2 4 6 8 10 12 14 16 18 20
time (min)
c
CTSS
SEQ ID No: 1
peptide + CTSS
peptide + CTSS + E64
SEQ ID No: 4
peptide + CTSS
peptide + CTSS + E64
Emission 390 nm (x 10^7)
0 2 6 10 14 18 22 26
minutes

Figure 1

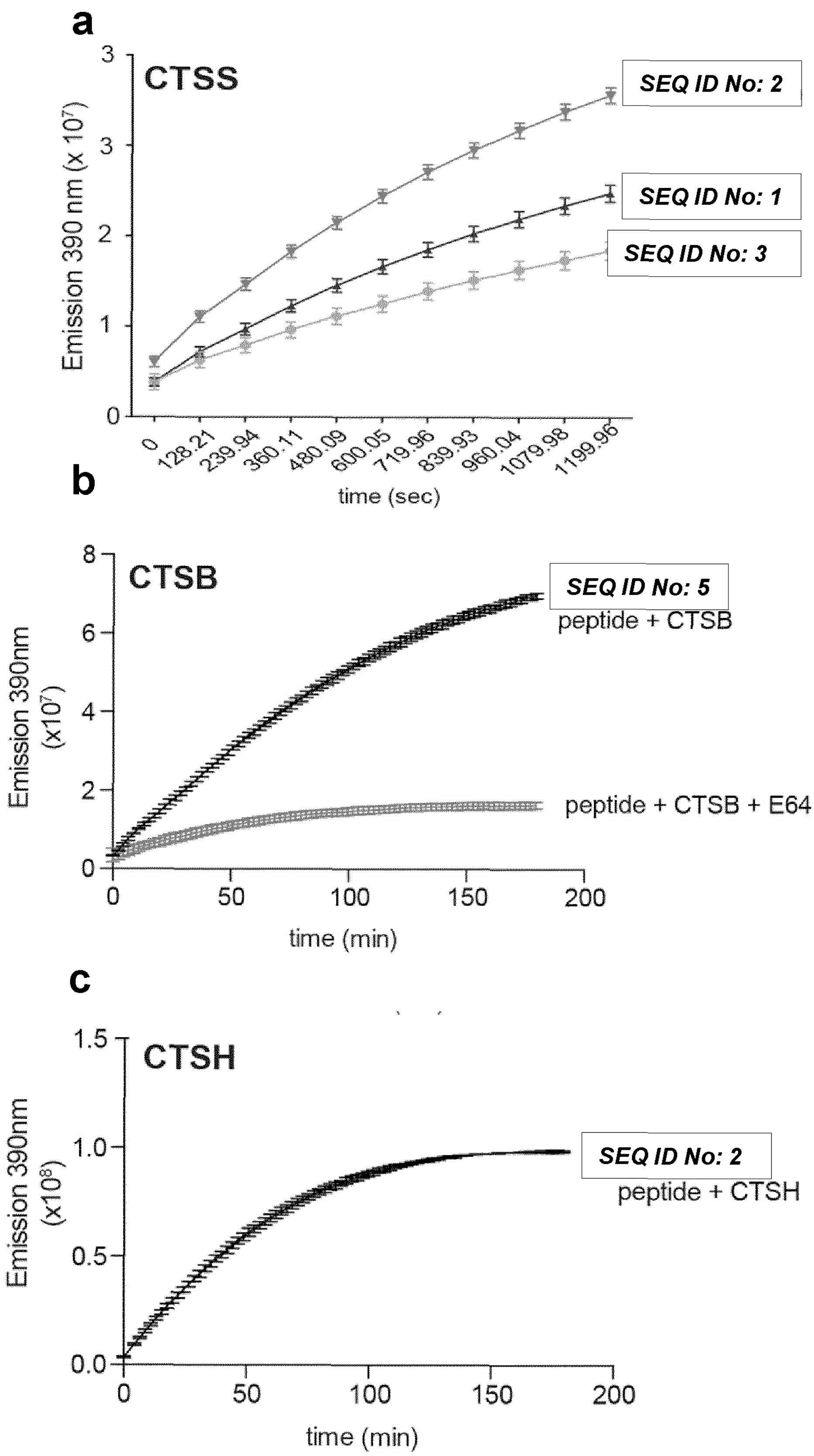
a
CTSS
SEQ ID No: 2
SEQ ID No: 1
SEQ ID No: 3
Emission 390 nm (x 10^7)
time (sec)
0
128.21
239.94
360.11
480.09
600.05
719.96
839.93
960.04
1079.98
1199.96
b
CTSB
SEQ ID No: 5
peptide + CTSB
peptide + CTSB + E64
Emission 390nm (x10^7)
time (min)
c
CTSH
SEQ ID No: 2
peptide + CTSH
Emission 390nm (x10^8)
time (min)

Figure 2

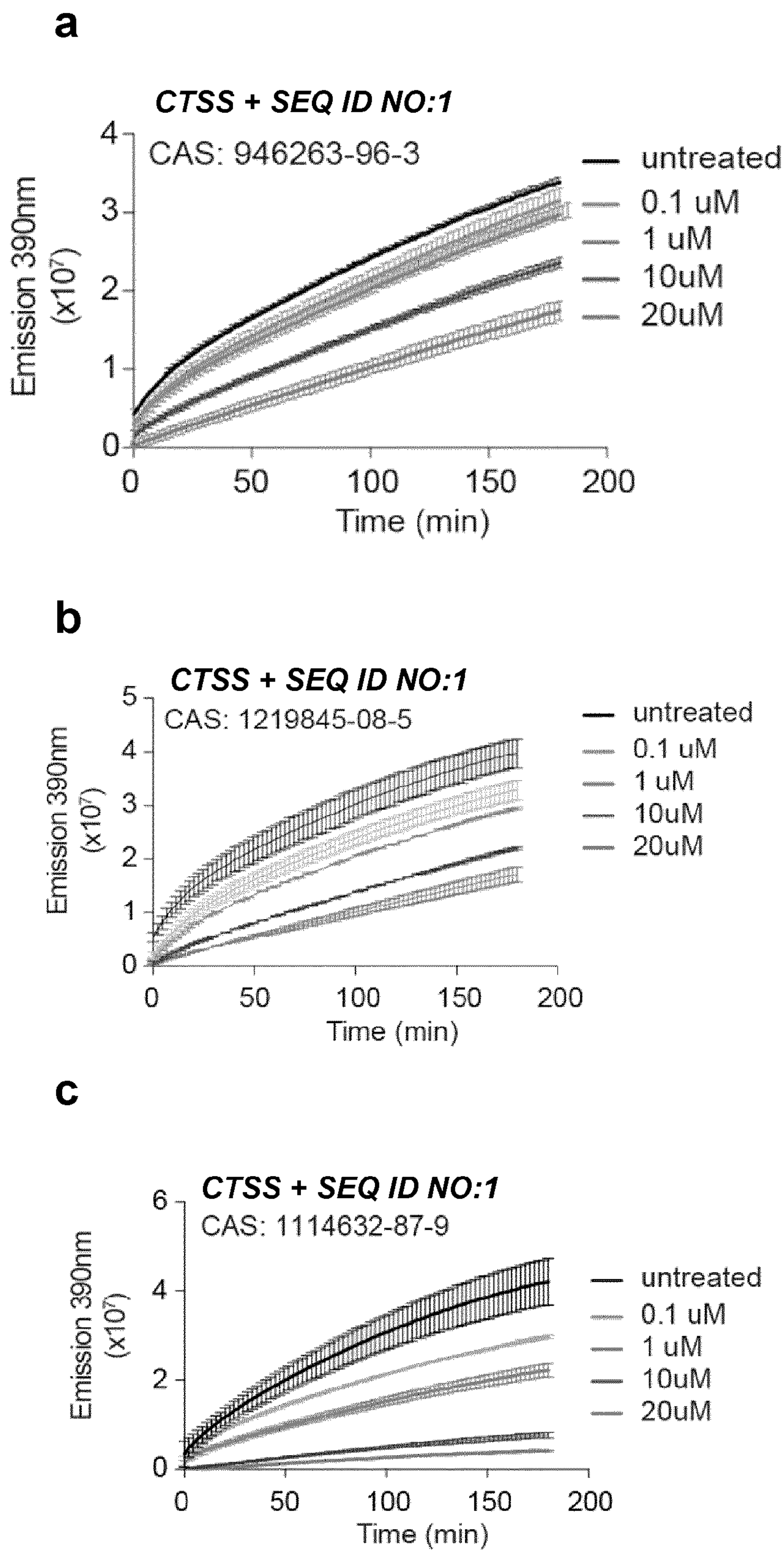
a
CTSS + SEQ ID NO:1
CAS: 946263-96-3
Emission 390nm ($x10^7$)
Time (min)
untreated
0.1 uM
1 uM
10uM
20uM
b
CTSS + SEQ ID NO:1
CAS: 1219845-08-5
Emission 390nm ($x10^7$)
Time (min)
untreated
0.1 uM
1 uM
10uM
20uM
c
CTSS + SEQ ID NO:1
CAS: 1114632-87-9
Emission 390nm ($x10^7$)
Time (min)
untreated
0.1 uM
1 uM
10uM
20uM

Figure 3

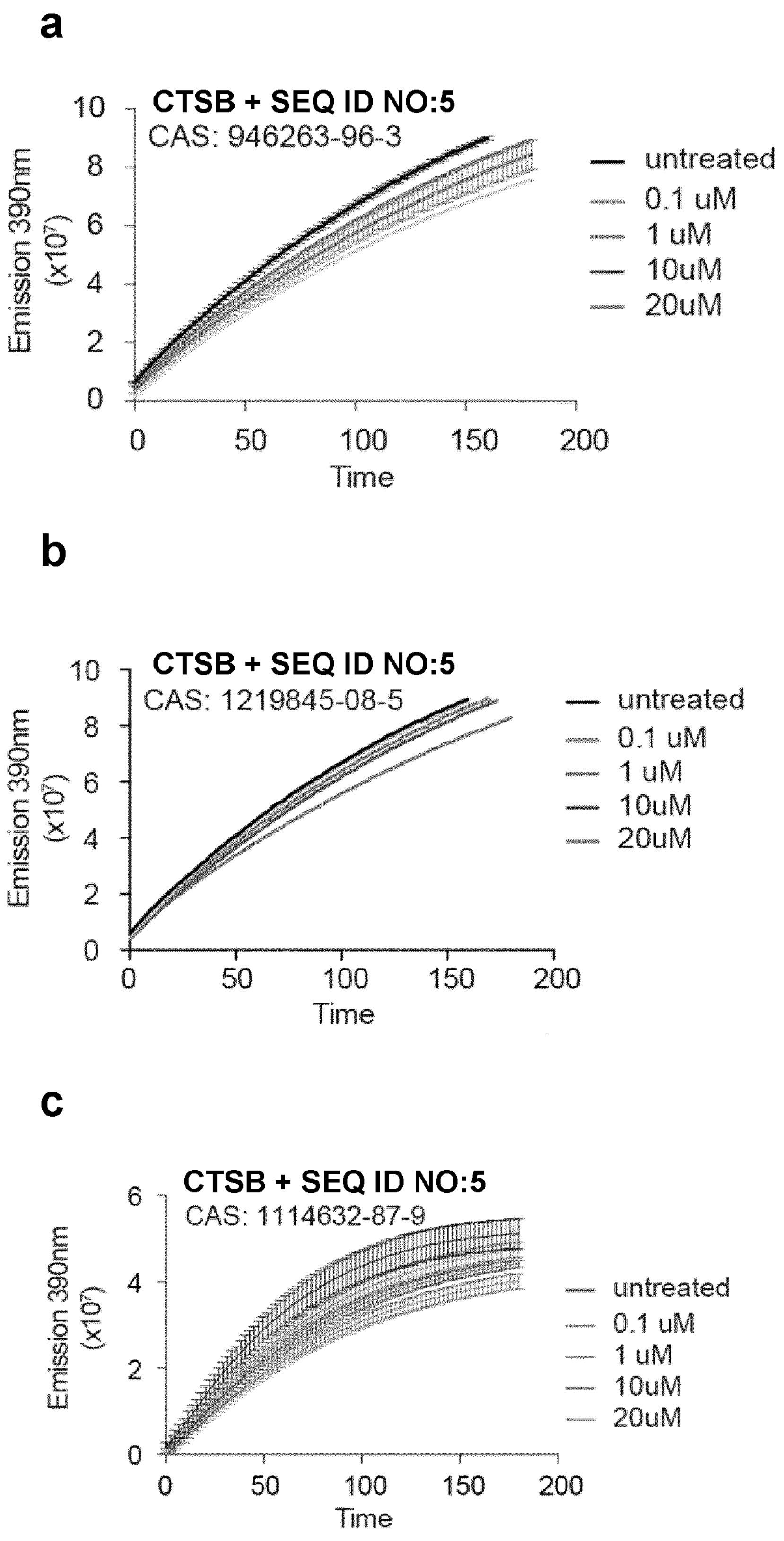
a
CTSB + SEQ ID NO:5
CAS: 946263-96-3
Emission 390nm (x10^7)
Time
untreated
0.1 uM
1 uM
10uM
20uM
b
CTSB + SEQ ID NO:5
CAS: 1219845-08-5
Emission 390nm (x10^7)
Time
untreated
0.1 uM
1 uM
10uM
20uM
c
CTSB + SEQ ID NO:5
CAS: 1114632-87-9
Emission 390nm (x10^7)
Time
untreated
0.1 uM
1 uM
10uM
20uM

Figure 4

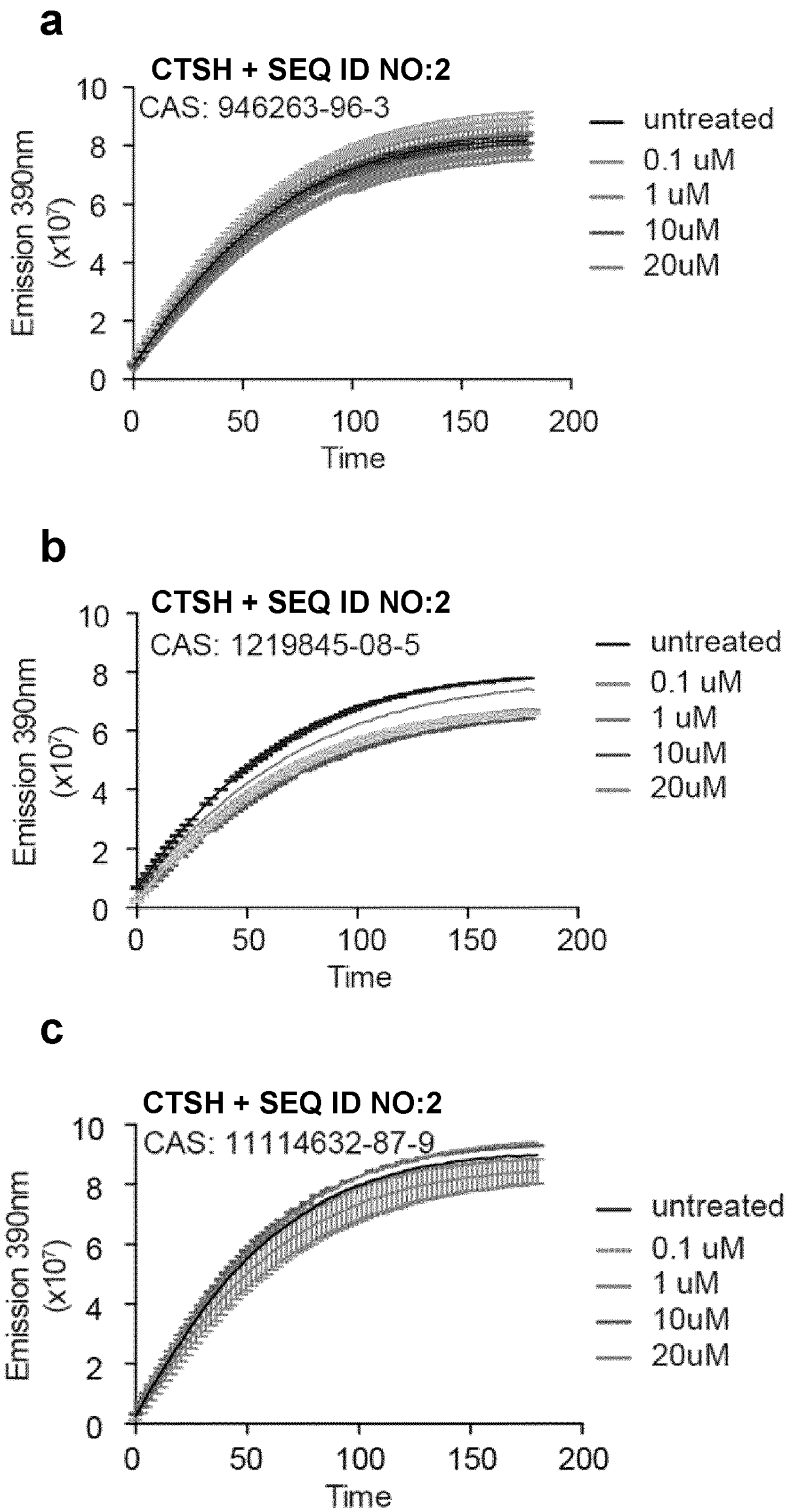
a
CTSH + SEQ ID NO:2
CAS: 946263-96-3
Emission 390nm ($x10^7$)
Time
0
2
4
6
8
10
0
50
100
150
200
untreated
0.1 uM
1 uM
10uM
20uM
b
CTSH + SEQ ID NO:2
CAS: 1219845-08-5
Emission 390nm ($x10^7$)
Time
0
2
4
6
8
10
0
50
100
150
200
untreated
0.1 uM
1 uM
10uM
20uM
c
CTSH + SEQ ID NO:2
CAS: 11114632-87-9
Emission 390nm ($x10^7$)
Time
0
2
4
6
8
10
0
50
100
150
200
untreated
0.1 uM
1 uM
10uM
20uM

Figure 5

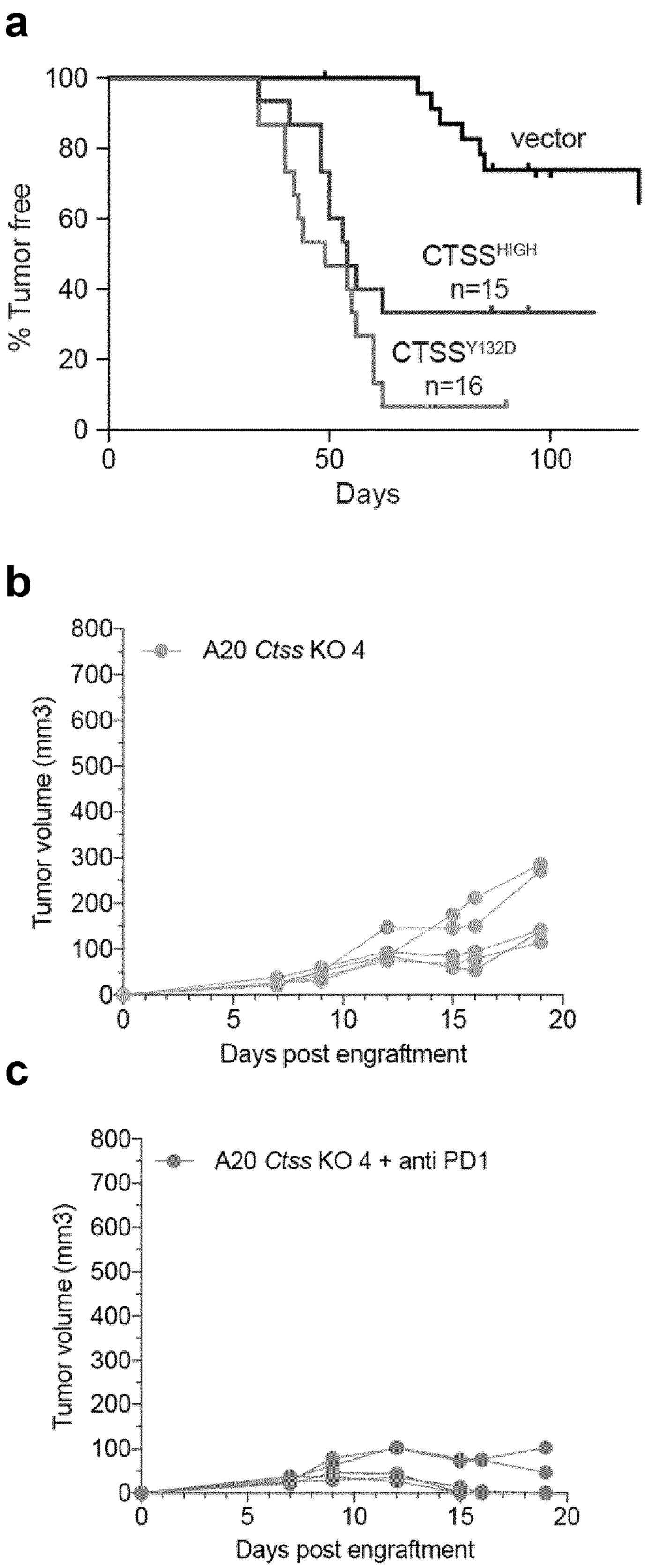
a
100
80
60
40
20
0
% Tumor free
vector
CTSS HIGH
n=15
CTSS Y132D
n=16
0
50
100
Days
b
800
700
600
500
400
300
200
100
0
Tumor volume (mm3)
A20 Ctss KO 4
0
5
10
15
20
Days post engraftment
c
800
700
600
500
400
300
200
100
0
Tumor volume (mm3)
A20 Ctss KO 4 + anti PD1
0
5
10
15
20
Days post engraftment

Figure 6

Europäisches Patentamt
European Patent Office
Office européen des brevets

# EUROPEAN SEARCH REPORT

Application Number
EP 20 16 5999

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | Elena Battistello: "Novel therapeutic strategies targeting the B-cell receptor signaling and antigen presentation pathways in non-Hodgkin lymphoma", 2019, EPFL, Lausanne, XP055729726, DOI: 10.5075/epfl-thesis-7205, * abstract * | 1-7,11 | INV. A61K31/497 A61P35/00 |
| Y | & Elena Battistello: "Novel therapeutic strategies targeting the B-cell receptor signaling and antigen presentation pathways in non-Hodgkin lymphoma", , 8 February 2020 (2020-02-08), XP055730449, Retrieved from the Internet: URL:https://web.archive.org/web/20200208154607/https://infoscience.epfl.ch/record/268268 [retrieved on 2020-09-14] ----- | 1-7,11 | |
| Y | ANDERS BONDE JENSEN ET AL: "The Cathepsin K Inhibitor Odanacatib Suppresses Bone Resorption in Women With Breast Cancer and Established Bone Metastases: Results of a 4-Week, Double-Blind, Randomized, Controlled Trial", CLINICAL BREAST CANCER, vol. 10, no. 6, 1 December 2010 (2010-12-01), pages 452-458, XP055730285, US ISSN: 1526-8209, DOI: 10.3816/CBC.2010.n.059 * the whole document * ----- | 1-7,11 | TECHNICAL FIELDS SEARCHED (IPC): A61K A61P |

-/--

~~The present search report has been drawn up for all claims~~

1

| Place of search | Date of completion of the search | Examiner |
|---|---|---|
| Munich | 17 September 2020 | Orlando, Michele |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 20 16 5999

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 6 605 589 B1 (UCKUN FATIH M [US] ET AL) 12 August 2003 (2003-08-12)<br>* claims; examples; compounds Z-Phe-Gly-NHO-Bz *<br>* the whole document *<br>----- | 1-7,11 | |
| A | WO 02/14314 A2 (ORTHO MCNEIL PHARM INC [US]) 21 February 2002 (2002-02-21)<br>* claims *<br>* the whole document *<br>----- | 1-8,11 | |
| A | WO 2017/074998 A1 (THE BRIGHAM AND WOMEN'S HOSPITAL INC [US]) 4 May 2017 (2017-05-04)<br>* the whole document *<br>* claims; examples *<br>----- | 1-8,11 | |
| A | WO 2008/019491 A1 (CA NAT RESEARCH COUNCIL [CA]; MORENO MARIA J [CA] ET AL.) 21 February 2008 (2008-02-21)<br>* abstract *<br>----- | 1-8,11 | TECHNICAL FIELDS SEARCHED (IPC) |
| A | KIM S H ET AL: "Effect of novel N-cyano-tetrahydro-pyridazine compounds, a class of cathepsin K inhibitors, on the bone resorptive activity of mature osteoclasts", BIOORGANIC & MEDICINAL CHEMISTRY LETTERS, PERGAMON, AMSTERDAM, NL, vol. 18, no. 14, 15 July 2008 (2008-07-15), pages 3988-3991, XP022852880, ISSN: 0960-894X, DOI: 10.1016/J.BMCL.2008.06.017 [retrieved on 2008-06-10]<br>* abstract; compounds *<br>-----<br>-/-- | 1-8,11 | |

1 ~~The present search report has been drawn up for all claims~~

| Place of search | Date of completion of the search | Examiner |
|---|---|---|
| Munich | 17 September 2020 | Orlando, Michele |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 3

Europäisches Patentamt
European Patent Office
Office européen des brevets

# EUROPEAN SEARCH REPORT

Application Number
EP 20 16 5999

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | ELIE DHEILLY ET AL: "Cathepsin S Regulates Antigen Processing and T Cell Activity in Non-Hodgkin Lymphoma", CANCER CELL, vol. 37, no. 5, 23 April 2020 (2020-04-23), pages 674-689.e12, XP055730259, US ISSN: 1535-6108, DOI: 10.1016/j.ccell.2020.03.016 * the whole document * ----- | 1-8,11 | |
| T | DEEPAK BARARIA ET AL: "Cathepsin S Alterations Induce a Tumor-Promoting Immune Microenvironment in Follicular Lymphoma", CELL REPORTS, vol. 31, no. 5, 1 May 2020 (2020-05-01), page 107522, XP055730444, US ISSN: 2211-1247, DOI: 10.1016/j.celrep.2020.107522 * the whole document * ----- | 1-8,11 | TECHNICAL FIELDS SEARCHED (IPC) |

~~The present search report has been drawn up for all claims~~

1

| Place of search | Date of completion of the search | Examiner |
|---|---|---|
| Munich | 17 September 2020 | Orlando, Michele |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

EPO FORM 1503 03.82 (P04C01)

Europäisches Patentamt
European Patent Office
Office européen des brevets

Application Number
EP 20 16 5999

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-8, 11(all partially)

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 20 16 5999

The Search Division considers that the present European patent application does not comply with the requirements of unity of invention and relates to several inventions or groups of inventions, namely:

1. claims: 1-8, 11(all partially)

   Cathepsin inhibitors of formula I for use in the treatment of cancer.

   ---

2. claims: 1, 4-8, 11(all partially)

   Cathepsin inhibitors of formula I for use in the treatment of immunological disorders.

   ---

3. claims: 1-6, 9-11(all partially)

   Cathepsin inhibitors of formula II for use in the treatment of cancer.

   ---

4. claims: 1, 4-6, 9-11(all partially)

   Cathepsin inhibitors of formula II for use in the treatment of immunological disorders.

   ---

5. claims: 12-15

   Method for identifying agents useful in the potentiation of immunotherapy.

   ---

## ANNEX TO THE EUROPEAN SEARCH REPORT ON EUROPEAN PATENT APPLICATION NO. EP 20 16 5999

This annex lists the patent family members relating to the patent documents cited in the above-mentioned European search report. The members are as contained in the European Patent Office EDP file on
The European Patent Office is in no way liable for these particulars which are merely given for the purpose of information.

17-09-2020

| Patent document cited in search report | Publication date | Patent family member(s) | Publication date |
|---|---|---|---|
| US 6605589 B1 | 12-08-2003 | AU 4962601 A | 15-10-2001 |
| | | US 6605589 B1 | 12-08-2003 |
| | | US 2004005329 A1 | 08-01-2004 |
| | | WO 0174376 A2 | 11-10-2001 |
| WO 0214314 A2 | 21-02-2002 | AR 035714 A1 | 07-07-2004 |
| | | AT 352552 T | 15-02-2007 |
| | | AU 8125501 A | 25-02-2002 |
| | | AU 2001281255 B2 | 20-07-2006 |
| | | CA 2419540 A1 | 21-02-2002 |
| | | CN 1468239 A | 14-01-2004 |
| | | DE 60126286 T2 | 15-11-2007 |
| | | DK 1309591 T3 | 26-03-2007 |
| | | EP 1309591 A2 | 14-05-2003 |
| | | ES 2281434 T3 | 01-10-2007 |
| | | HK 1052509 A1 | 19-09-2003 |
| | | JP 5140225 B2 | 06-02-2013 |
| | | JP 2004512272 A | 22-04-2004 |
| | | MX PA03001421 A | 26-01-2004 |
| | | NZ 524193 A | 24-12-2004 |
| | | PT 1309591 E | 30-04-2007 |
| | | US 2002040020 A1 | 04-04-2002 |
| | | US 2007004738 A1 | 04-01-2007 |
| | | US 2007004747 A1 | 04-01-2007 |
| | | US 2007004754 A1 | 04-01-2007 |
| | | US 2007004755 A1 | 04-01-2007 |
| | | US 2007010530 A1 | 11-01-2007 |
| | | US 2007021437 A1 | 25-01-2007 |
| | | US 2008293732 A1 | 27-11-2008 |
| | | US 2008300255 A1 | 04-12-2008 |
| | | WO 0214314 A2 | 21-02-2002 |
| WO 2017074998 A1 | 04-05-2017 | US 2019060361 A1 | 28-02-2019 |
| | | WO 2017074998 A1 | 04-05-2017 |
| WO 2008019491 A1 | 21-02-2008 | CA 2659585 A1 | 21-02-2008 |
| | | EP 2056868 A1 | 13-05-2009 |
| | | EP 2462945 A2 | 13-06-2012 |
| | | EP 2462946 A1 | 13-06-2012 |
| | | ES 2418434 T3 | 13-08-2013 |
| | | US 2010279937 A1 | 04-11-2010 |
| | | US 2016082083 A1 | 24-03-2016 |
| | | WO 2008019491 A1 | 21-02-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **PASQUALUCCI et al.** *Cell Rep.,* 2014, vol. 6, 130-140
- **BOICE et al.** *Cell,* 2016, vol. 167, 405-418
- **KRIDEL et al.** *PLOS Med.,* 2016, vol. 13, e1002197
- **OKOSUN et al.** *Nat. Genet.,* 2014, vol. 46, 176-181
- **PASQUALUCCI et al.** *Nat. Genet.,* 2011, vol. 43, 830-837
- **CHEUNG et al.** *Cancer Res.,* 2010, vol. 70, 9166-9174
- **KOTSIOU et al.** *Blood,* 2014, vol. 124, 2426-2426
- *Blood,* 2016, vol. 128, 72-81
- **CHALLA-MALLADI et al.** *Cancer Cell,* 2011, vol. 20, 728-740
- **CROTTY.** *Immunity,* 2014, vol. 41, 529-542
- **SCOTT ; GASCOYNE.** *Nat. Rev. Cancer,* 2014, vol. 14, 517-534
- **ISE et al.** *Immunity,* 2018, vol. 48, 702-715
- **RIESE et al.** *J. Clin. Invest.,* 1996, vol. 101, 2351-2363
- **SHI et al.** *Immunity,* 1999, vol. 10, 197-206
- **ROCHE ; FURUTA.** *Nat. Rev. Immunol.,* 2015, vol. 15, 203-216
- **SCHRÖDER.** *Mol. Cell Res.,* 2016, vol. 1863, 1269-1281
- **VILLADANGOS et al.** *J. Exp. Med.,* 1997, vol. 186, 549-560
- **RIESE et al.** *J. Clin. Invest.,* 1998, vol. 101, 2351-2363
- **GOCHEVA et al.** *Genes Dev.,* 2006, vol. 20, 543-556
- **OLSON ; JOYCE.** *Nat. Rev. Cancer,* 2015, vol. 15, 712-729
- **SEVENICH et al.** *Nat. Cell Biol.,* 2014, vol. 16, 876-888
- **GAROFALO et al.** *Cancer - Oncolytic Viruses II,* 2015, vol. 23 (I), 247
- **GUEDAN et al.** *Molecular Therapy, Methods & Clinical Development,* 2019, vol. 12, 145-156
- **CHAU et al.** *Therapeutics,* 2019, vol. 394 (10200), 793-804
- **THERON et al.** *Front Immunol.,* 2017, vol. 8, 806
- *CHEMICAL ABSTRACTS,* 946263-96-3
- *CHEMICAL ABSTRACTS,* 1021225-32-0
- *CHEMICAL ABSTRACTS,* 946365-20-4
- *CHEMICAL ABSTRACTS,* 1021225-23-9
- *CHEMICAL ABSTRACTS,* 1219845-08-5
- *CHEMICAL ABSTRACTS,* 1282103-56-3
- *CHEMICAL ABSTRACTS,* 946212-65-3
- *CHEMICAL ABSTRACTS,* 942004-60-6
- *CHEMICAL ABSTRACTS,* 1114614-87-7
- *CHEMICAL ABSTRACTS,* 1111056-12-2
- *CHEMICAL ABSTRACTS,* 1114632-87-9
- **GEURINK et al.** *J. Med. Chem.,* 2013, vol. 56, 1262
- **RAND et al.** *Med. Chem. Commun,* 2012, vol. 3, 1282
- **AMERIKS et al.** *Bioorganic & Medicinal Chemistry Letters,* 2010, vol. 20, 4060-4064
- **COATES et al.** *Synthesis,* 1993, vol. 03, 334-342
- Remington's "The Science and Practice of Pharmacy". University of the Sciences in Philadelphia, 2012
- **REISER et al.** *J. Clin. Invest.,* 2010, vol. 120, 3421-3431
- **ARAF et al.** *Leukemia,* 2018, vol. 32, 1261-1265
- **GREEN et al.** *Blood,* 2013, vol. 121, 1604-1611
- **KRIDEL et al.** *PLOSMed.,* 2016, vol. 13, e1002197
- **MORIN et al.** *Nature,* 2011, vol. 476, 298-30
- **ORTEGA-MOLINA et al.** *Nat. Med.,* 2015, vol. 21, 1199-1208
- **REDDY et al.** *Cell,* 2017, vol. 171, 481-494
- **SCHMITZ et al.** *N. Engl. J. Med.,* 2018, vol. 378, 1396-1407
- **TURK et al.** *Biochim. Biophys. Acta BBA - Proteins Proteomics,* 2012, vol. 1824, 68-88
- **VASILJEVA et al.** *FEBS Lett.,* 2005, vol. 579, 1285-1290
- **VIZOVIŠEK et al.** *PROTEOMICS,* 2015, vol. 15, 2479-2490
- *CHEMICAL ABSTRACTS,* 66701-25-5
- **CHUN et al.** *Cancer Cell,* 2016, vol. 29, 394-406
- **BAKER et al.** *roc. Natl. Acad. Sci.,* 2001, vol. 98, 10037-10041
- **PETTERSEN et al.** *J. Comput. Chem.,* 2004, vol. 25, 1605-1612
- **DOLINSKY et al.** *Nucleic Acids Res.,* vol. 35, W522-W525
- *Nucleic Acids Res.,* 2004, vol. 32, W665-667

- **SITKOFF et al.** *Biophys. J.,* 1994, vol. 67, 2251-2260
- **LI et al.** *Proteins,* 2005, vol. 61, 704-721
- **SONDERGAARD et al.** *J. Chem. Theory Comput.,* 2011, vol. 7, 2284-2295
- **PAULY et al.** *Biochemistry,* 2003, vol. 42, 3203-3213
- **SHAPOVALOV ; DUNBRACK.** *Struct. Lond. Engl.,* 1993, vol. 19, 844-858
- **ZOETE et al.** *J. Comput. Chem.,* 2016, vol. 37, 437-447
- **DAVE et al.** *N. Engl. J. Med.,* 2004, vol. 351, 2159-2169
- **MILPIED et al.** *Nat. Immunol.,* 2018, vol. 19, 1013
- **KRIDEL et al.** *Clin. Cancer Research,* 2015, vol. 21 (15), 3428-35
- **DONALDSON-COLLIER et al.** *Nature Genetics,* vol. 219
- **BUTLER et al.** *Nat. Biotechnol.,* 2018, vol. 36, 411-420
- **SATIJA et al.** *Nat. Biotechnol.,* 2015, vol. 33, 495-502
- **HÄNZELMANN et al.** *BMC Bioinformatics,* 2013, vol. 14, 7
- **EGLE et al.** *Blood,* 2004, vol. 103, 2276-2283
- **ORICCHIO et al.** *J. Exp. Med.,* 2011, vol. 211, 1379-1391
- **CIRIELLO et al.** *Genome Res.,* 2012, vol. 22, 398-406
- **MORGAN et al.** *J. Immunol.,* 1996, vol. 157, 978-983
- **WESTIN et al.** *Lancet Oncol.,* 2014, vol. 15, 69-77
- **TUMEH et al.** *Nature,* 2014, vol. 515, 568-571
- **EGLE et al.** *Blood,* 2004, vol. 103 (6), 2276-2283
- **AKKARI et al.** *enes Dev,* 2016, vol. 30 (2), 220-32
- *CHEMICAL ABSTRACTS,* 1373215-15-6
- **SEO et al.** *Antioxid. Redox Signal.,* 2016, vol. 27, 215-233
- *Biochem. Biophys. Res. Commun.,* 2018, vol. 498, 849-854
- **JADHAV et al.** *ACS Med. Chem. Lett.,* 2014, vol. 5, 1138-1142
- **PAYNE et al.** *Br. J. Clin. Pharmacol.,* 2014, vol. 78, 1334-134